# EUROPEAN PATENT SPECIFICATION

(11) **EP 1 395 261 B1**
(45) Date of publication and mention of the grant of the patent: **28.06.2006**
(21) Application number: 02769490.0
(22) Date of filing: 16.05.2002
(51) Int. Cl.: A61K 31/506

(54) **PYRIDYLPYRIMIDINE DERIVATIVES AS EFFECTIVE COMPOUNDS AGAINST PRION DISEASES**
PYRIDYLPYRIMIDIN-DERIVATE ALS WIRKSAME VERBINDUNGEN GEGEN PRIONEN-KRANKHEITEN
DERIVES DE PYRIDYLPYRIMIDINE UTILISES COMME COMPOSES ACTIFS CONTRE DES INFECTIONS ET DES MALADIES A PRIONS

(30) Priority: 16.05.2001 EP 01111858; 29.05.2001 US 293528 P; 13.07.2001 EP 01117113; 18.07.2001 US 305898 P
(43) Date of publication of application: 10.03.2004
(73) Proprietor: GPC Biotech AG, 82152 Planegg-Martinsried (DE)
(72) Inventor: STEIN-GERLACH, Matthias, 81475 München (DE); SALASSIDIS, Konstadinos, 85386 Eching (DE); BACHER, Gerald, 82110 Germering (DE); MÜLLER, Stefan, 81371 München (DE); KLEBL, Bert, 82294 Günzelhofen (DE); MAROSFALVI, Jenö, 1118 Budapest (HU); KERI, György, 1021 Budapest (HU); ÖRFI, Laszlo, 1161 Budapest (HU); VARGA, Zoltan, 7052 Kölesd (HU)
(74) Representative: Dörries, Hans Ulrich
(86) International application number: PCT/EP2002/005420
(87) International publication number: WO 2002/093164

(56) References cited:
- EP-A- 0 564 409
- WO-A-00/64894
- WO-A-95/09847
- US-A- 6 040 321
- US-A- 6 093 713
- US-A- 6 107 301
- US-A- 6 107 305
- US-A- 6 140 330
- ZIMMERMANN J ET AL: "Phenylamino-pyrimidine (PAP) - derivatives: a new class of potent and highly selective PDGF-receptor autophosphorylation inhibitors" BIOORGANIC & MEDICINAL CHEMISTRY LETTERS, OXFORD, GB, vol. 6, no. 11, 4 June 1996 (1996-06-04), pages 1221-1226, XP004134858 ISSN: 0960-894X
- ZIMMERMANN J ET AL: "Potent and selective inhibitors of the Abl-kinase: phenylamino-pyrimidine (PAP) derivatives" BIOORGANIC & MEDICINAL CHEMISTRY LETTERS, OXFORD, GB, vol. 7, no. 2, 21 January 1997 (1997-01-21), pages 187-192, XP004135990 ISSN: 0960-894X
- ZIMMERMANN J ET AL: "PHENYLAMINO-PYRIMIDINE (PAP) DERIVATIVES: A NEW CLASS OF POTENT ANDSELECTIVE INHIBITORS OF PROTEIN KINASE C (PKC)" ARCHIV DER PHARMAZIE, VCH VERLAGSGESELLSCHAFT MBH, WEINHEIM, DE, vol. 329, no. 7, July 1996 (1996-07), pages 371-376, XP000885618 ISSN: 0365-6233
- JIMI T ET AL: "HIGH LEVELS OF NERVOUS SYSTEM-SPECIFIC PROTEINS IN CEREBROSPINAL FLUID IN PATIENTS WITH EARLY STAGE CRUTZFELDT-JAKOB DISEASE" CLINICA CHIMICA ACTA, AMSTERDAM, NL, vol. 211, no. 1/2, 15 October 1992 (1992-10-15), pages 37-46, XP002071132 ISSN: 0009-8981
- JAE-KWANG ET AL.: "Increased expression of CaM kinase II alpha in the brains of scrapie-infected mice" NEUROSCIENCE LETTERS, vol. 273, 1999, pages 37-40, XP002229677
- MAGURA ET AL: 'MECHANISMS OF HIV-1 MEDIATED NEURODEGENERATION PROMOTED BY MACROPHAGE AND ASTROGLIAL FACTORS' BIOPOLIMERY I KLETKA vol. 13, 1997, pages 328 - 334

### SEQUENCE LISTING

<110> Axxima Pharmaceuticals AG
<120> Human cellular protein kinases and phosphatases as targets for dignosis and treatment of prion diseases
<130> AXX-P11001-WO
<140> 0111858.5
   <141> 2001-05-16
<150> US 60/293,528
   <151> 2001-05-29
<160>
<170> PatentIn Ver. 2.1
<210> 1
   <211> 2662
   <212> DNA
   <213> Homo sapiens
<220> FGF-R1
   <223> Description of Sequence: N/A
<400> 1
   tcagtttgaa aaggaggatc gagctcactc gtggagtatc catggagatg tggagccttg 60
   tcaccaacct ctaactgcag aactgggatg tggagctgga agtgcctcct cttctgggct 120
   gtgctggtca cagccacact ctgcaccgct aggccgtccc cgaccttgcc tgaacaagcc 180
   cagccctggg gagcccctgt ggaagtggag tccttcctgg tccaccccgg tgacctgctg 240
   cagcttcgct gtcggctgcg ggacgatgtg cagagcatca actggctgcg ggacggggtg 300
   cagctggcgg aaagcaaccg cacccgcatc acaggggagg aggtggaggt gcaggactcc 360
   gtgcccgcag actccggcct ctatgcttgc gtaaccagca gcccctcggg cagtgacacc 420
   acctacttct ccgtcaatgt ttcagatgct ctcccctcct cggaggatga tgatgatgat 480
   gatgactcct cttcagagga gaaagaaaca gataacacca aaccaaaccg tatgcccgta 540
   gctccatatt ggacatcccc agaaaagatg gaaaagaaat tgcatgcagt gccggctgcc 600
   aagacagtga agttcaaatg cccttccagt gggaccccaa accccacact gcgctggttg 660
   aaaaatggca aagaattcaa acctgaccac agaattggag gctacaaggt ccgttatgcc 720
   acctggagca tcataatgga ctctgtggtg ccctctgaca agggcaacta cacctgcatt 780
   gtggagaatg agtacggcag catcaaccac acataccagc tggatgtcgt ggagcggtcc 840
   cctcaccgcc ccatcctgca agcagggttg cccgccaaca aaacagtggc cctgggtagc 900
   aacgtggagt tcatgtgtaa ggtgtacagt gacccgcagc cgcacatcca gtggctaaag 960
   cacatcgagg tgaatgggag caagattggc ccagacaacc tgccttatgt ccagatcttg 1020
   aagactgctg gagttaatac caccgacaaa gagatggagg tgcttcactt aagaaatgtc 1080
   tcctttgagg acgcagggga gtatacgtgc ttggcgggta actctatcgg actctcccat 1140
   cactctgcat ggttgaccgt tctggaagcc ctggaagaga ggccggcagt gatgacctcg 1200
   cccctgtacc tggagatcat catctattgc acaggggcct tcctcatctc ctgcatggtg 1260
   gggtcggtca tcgtctacaa gatgaagagt ggtaccaaga agagtgactt ccacagccag 1320
   atggctgtgc acaagctggc caagagcatc cctctgcgca gacaggtaac agtgtctgct 1380
   gactccagtg catccatgaa ctctggggtt cttctggttc ggccatcacg gctctcctcc 1440
   agtgggactc ccatgctagc aggggtctct gagtatgagc ttcccgaaga ccctcgctgg 1500
   gagctgcctc gggacagact ggtcttaggc aaacccctgg gagagggctg ctttgggcag 1560
   gtggtgttgg cagaggctat cggcctggac aaggacaaac ccaaccgtgt gaccaaagtg 1620
   gctgtgaaga tgttgaagtc ggacgcaaca gagaaagact tgtcagacct gatctcagaa 1680
   atggagatga tgaagatgat cgggaagcat aagaatatca tcaacctgct gggggcctgc 1740
   acgcaggatg gtcccttgta tgtcatcgtg gagtatgcct ccaagggcaa cctgcgggag 1800
   tacctgcagg cccggaggcc cccagggctg gaatactgct acaaccccag ccacaaccca 1860
   gaggagcagc tctcctccaa ggacctggtg tcctgcgcct accaggtggc ccgaggcatg 1920
   gagtatctgg cctccaagaa gtgcatacac cgagacctgg cagccaggaa tgtcctggtg 1980
   acagaggaca atgtgatgaa gatagcagac tttggcctcg cacgggacat tcaccacatc 2040
   gactactata aaaagacaac caacggccga ctgcctgtga agtggatggc acccgaggca 2100
   ttatttgacc ggatctacac ccaccagagt gatgtgtggt ctttcggggt gctcctgtgg 2160
   gagatcttca ctctgggcgg ctccccatac cccggtgtgc ctgtggagga acttttcaag 2220
   ctgctgaagg agggtcaccg catggacaag cccagtaact gcaccaacga gctgtacatg 2280
   atgatgcggg actgctggca tgcagtgccc tcacagagac ccaccttcaa gcagctggtg 2340
   gaagacctgg accgcatcgt ggccttgacc tccaaccagg agtacctgga cctgtccatg 2400
   cccctggacc agtactcccc cagctttccc gacacccgga gctctacgtg ctcctcaggg 2460
   gaggattccg tcttctctca tgagccgctg cccgaggagc cctgcctgcc ccgacaccca 2520
   gcccagcttg ccaatggcgg actcaaacgc cgctgactgc cacccacacg ccctccccag 2580
   actccaccgt cagctgtaac cctcacccac agcccctgcc tgggcccacc acctgtccgt 2640
   ccctgtcccc tttcctgctg gg                                          2662
<210> 2
   <211> 822
   <212> PRT
   <213> Homo sapiens
<220>
   <223> Description of Sequence: N/A
<400> 2
<210> 3
   <211> 3840
   <212> DNA
   <213> Homo sapiens
<220> Abl
   <223> Description of Sequence: N/A
<400> 3
   ggccttcccc ctgcgaggat cgccgttggc ccgggttggc tttggaaagc ggcggtggct 60
   ttgggccggg ctcggcctcg ggaacgccag gggcccctgg gtgcggacgg gcgcggccag 120
   gagggggtta aggcgcaggc ggcggcgggg cgggggcggg cctggcgggc gccctctccg 180
   ggccctttgt taacaggcgc gtcccggcca gcggagacgc ggccgccctg ggcgggcgcg 240
   ggcggcgggc ggcggtgagg gcggcctgcg gggcggcgcc cgggggccgg gccgagccgg 300
   gcctgagccg ggcccggacc gagctgggag aggggctccg gcccgatcgt tcgcttggcg 360
   caaaatgttg gagatctgcc tgaagctggt gggctgcaaa tccaagaagg ggctgtcctc 420
   gtcctccagc tgttatctgg aagaagccct tcagcggcca gtagcatctg actttgagcc 480
   tcagggtctg agtgaagccg ctcgttggaa ctccaaggaa aaccttctcg ctggacccag 540
   tgaaaatgac cccaaccttt tcgttgcact gtatgatttt gtggccagtg gagataacac 600
   tctaagcata actaaaggtg aaaagctccg ggtcttaggc tataatcaca atggggaatg 660
   gtgtgaagcc caaaccaaaa atggccaagg ctgggtccca agcaactaca tcacgccagt 720
   caacagtctg gagaaacact cctggtacca tgggcctgtg tcccgcaatg ccgctgagta 780
   tccgctgagc agcgggatca atggcagctt cttggtgcgt gagagtgaga gcagtcctag 840
   ccagaggtcc atctcgctga gatacgaagg gagggtgtac cattacagga tcaacactgc 900
   ttctgatggc aagctctacg tctcctccga gagccgcttc aacaccctgg ccgagttggt 960
   tcatcatcat tcaacggtgg ccgacgggct catcaccacg ctccattatc cagccccaaa 1020
   gcgcaacaag cccactgtct atggtgtgtc ccccaactac gacaagtggg agatggaacg 1080
   cacggacatc accatgaagc acaagctggg cgggggccag tacggggagg tgtacgaggg 1140
   cgtgtggaag aaatacagcc tgacggtggc cgtgaagacc ttgaaggagg acaccatgga 1200
   ggtggaagag ttcttgaaag aagctgcagt catgaaagag atcaaacacc ctaacctagt 1260
   gcagctcctt ggggtctgca cccgggagcc cccgttctat atcatcactg agttcatgac 1320
   ctacgggaac ctcctggact acctgaggga gtgcaaccgg caggaggtga acgccgtggt 1380
   gctgctgtac atggccactc agatctcgtc agccatggag tacctagaga agaaaaactt 1440
   catccacaga gatcttgctg cccgaaactg cctggtaggg gagaaccact tggtgaaggt 1500
   agctgatttt ggcctgagca ggttgatgac aggggacacc tacacagccc atgctggagc 1560
   caagttcccc atcaaatgga ctgcacccga gagcctggcc tacaacaagt tctccatcaa 1620
   gtccgacgtc tgggcatttg gagtattgct ttgggaaatt gctacctatg gcatgtcccc 1680
   ttacccggga attgaccgtt cccaggtgta tgagctgcta gagaaggact accgcatgaa 1740
   gcgcccagaa ggctgcccag agaaggtcta tgaactcatg cgagcatgtt ggcagtggaa 1800
   tccctctgac cggccctcct ttgctgaaat ccaccaagcc tttgaaacaa tgttccagga 1860
   atccagtatc tcagacgaag tggaaaagga gctggggaaa caaggcgtcc gtggggctgt 1920
   gactaccttg ctgcaggccc cagagctgcc caccaagacg aggacctcca ggagagctgc 1980
   agagcacaga gacaccactg acgtgcctga gatgcctcac tccaagggcc agggagagag 2040
   cgatcctctg gaccatgagc ctgccgtgtc tccattgctc cctcgaaaag agcgaggtcc 2100
   cccggagggc ggcctgaatg aagatgagcg ccttctcccc aaagacaaaa agaccaactt 2160
   gttcagcgcc ttgatcaaga agaagaagaa gacagcccca acccctccca aacgcagcag 2220
   ctccttccgg gagatggacg gccagccgga gcgcagaggg gccggcgagg aagagggccg 2280
   agacatcagc aacggggcac tggctttcac ccccttggac acagctgacc cagccaagtc 2340
   cccaaagccc agcaatgggg ctggggtccc caatggagcc ctccgggagt ccgggggctc 2400
   aggcttccgg tctccccacc tgtggaagaa gtccagcacg ctgaccagca gccgcctagc 2460
   caccggcgag gaggagggcg gtggcagctc cagcaagcgc ttcctgcgct cttgctccgt 2520
   ctcctgcgtt ccccatgggg ccaaggacac ggagtggagg tcagtcacgc tgcctcggga 2580
   cttgcagtcc acgggaagac agtttgactc gtccacattt ggagggcaca aaagtgagaa 2640
   gccggctctg cctcggaaga gggcagggga gaacaggtct gaccaggtga cccgaggcac 2700
   agtaacgcct ccccccaggc tggtgaaaaa gaatgaggaa gctgctgatg aggtcttcaa 2760
   agacatcatg gagtccagcc cgggctccag cccgcccaac ctgactccaa aacccctccg 2820
   gcggcaggtc accgtggccc ctgcctcggg cctcccccac aaggaagaag cctggaaagg 2880
   cagtgcctta gggacccctg ctgcagctga gccagtgacc cccaccagca aagcaggctc 2940
   aggtgcacca aggggcacca gcaagggccc cgccgaggag tccagagtga ggaggcacaa 3000
   gcactcctct gagtcgccag ggagggacaa ggggaaattg tccaagctca aacctgcccc 3060
   gccgccccca ccagcagcct ctgcagggaa ggctggagga aagccctcgc agaggcccgg 3120
   ccaggaggct gccggggagg cagtcttggg cgcaaagaca aaagccacga gtctggttga 3180
   tgctgtgaac agtgacgctg ccaagcccag ccagccggca gagggcctca aaaagcccgt 3240
   gctcccggcc actccaaagc cacaccccgc caagccgtcg gggaccccca tcagcccagc 3300
   ccccgttccc ctttccacgt tgccatcagc atcctcggcc ttggcagggg accagccgtc 3360
   ttccactgcc ttcatccctc tcatatcaac ccgagtgtct cttcggaaaa cccgccagcc 3420
   tccagagcgg gccagcggcg ccatcaccaa gggcgtggtc ttggacagca ccgaggcgct 3480
   gtgcctcgcc atctctggga actccgagca gatggccagc cacagcgcag tgctggaggc 3540
   cggcaaaaac ctctacacgt tctgcgtgag ctatgtggat tccatccagc aaatgaggaa 3600
   caagtttgcc ttccgagagg ccatcaacaa actggagaat aatctccggg agcttcagat 3660
   ctgcccggcg tcagcaggca gtggtccggc ggccactcag gacttcagca agctcctcag 3720
   ttcggtgaag gaaatcagtg acatagtgca gaggtagcag cagtcagggg tcaggtgtca 3780
   ggcccgtcgg agctgcctgc agcacatgcg ggctcgccca tacccatgac agtggctgag 3840
<210> 4
   <211> 1130
   <212> PRT
   <213> Homo sapiens
<220>
   <223> Description of Sequence: N/A
<400> 4
   Met Leu Glu Ile Cys Leu Lys Leu Val Gly Cys Lys Ser Lys Lys Gly
   1               5                   10                  15
   Leu Ser Ser Ser Ser Ser Cys Tyr Leu Glu Glu Ala Leu Gln Arg Pro
               20                  25                  30
   Val Ala Ser Asp Phe Glu Pro Gln Gly Leu Ser Glu Ala Ala Arg Trp
           35                  40                  45
   Asn Ser Lys Glu Asn Leu Leu Ala Gly Pro Ser Glu Asn Asp Pro Asn
       50                  55                  60
   Leu Phe Val Ala Leu Tyr Asp Phe Val Ala Ser Gly Asp Asn Thr Leu
   65                  70                  75                  80
   Ser Ile Thr Lys Gly Glu Lys Leu Arg Val Leu Gly Tyr Asn His Asn
                   85                  90                  95
   Gly Glu Trp Cys Glu Ala Gln Thr Lys Asn Gly Gln Gly Trp Val Pro
               100                 105                 110
   Ser Asn Tyr Ile Thr Pro Val Asn Ser Leu Glu Lys His Ser Trp Tyr
           115                 120                 125
   His Gly Pro Val Ser Arg Asn Ala Ala Glu Tyr Pro Leu Ser Ser Gly
       130                 135                 140
   Ile Asn Gly Ser Phe Leu Val Arg Glu Ser Glu Ser Ser Pro Ser Gln
   145                 150                 155                 160
   Arg Ser Ile Ser Leu Arg Tyr Glu Gly Arg Val Tyr His Tyr Arg Ile
                   165                 170                 175
   Asn Thr Ala Ser Asp Gly Lys Leu Tyr Val Ser Ser Glu Ser Arg Phe
               180                 185                 190
   Asn Thr Leu Ala Glu Leu Val His His His Ser Thr Val Ala Asp Gly
           195                 200                 205
   Leu Ile Thr Thr Leu His Tyr Pro Ala Pro Lys Arg Asn Lys Pro Thr
       210                 215                 220
   Val Tyr Gly Val Ser Pro Asn Tyr Asp Lys Trp Glu Met Glu Arg Thr
   225 230 235 240
   Asp Ile Thr Met Lys His Lys Leu Gly Gly Gly Gln Tyr Gly Glu Val
   245 250 255
   Tyr Glu Gly Val Trp Lys Lys Tyr Ser Leu Thr Val Ala Val Lys Thr
   260 265 270
   Leu Lys Glu Asp Thr Met Glu Val Glu Glu Phe Leu Lys Glu Ala Ala
   275 280 285
   Val Met Lys Glu Ile Lys His Pro Asn Leu Val Gln Leu Leu Gly Val
   290 295 300
   Cys Thr Arg Glu Pro Pro Phe Tyr Ile Ile Thr Glu Phe Met Thr Tyr
   305 310 315 320
   Gly Asn Leu Leu Asp Tyr Leu Arg Glu Cys Asn Arg Gln Glu Val Asn
   325 330 335
   Ala Val Val Leu Leu Tyr Met Ala Thr Gln Ile Ser Ser Ala Met Glu
   340 345 350
   Tyr Leu Glu Lys Lys Asn Phe Ile His Arg Asp Leu Ala Ala Arg Asn
   355 360 365
   Cys Leu Val Gly Glu Asn His Leu Val Lys Val Ala Asp Phe Gly Leu
   370 375 380
   Ser Arg Leu Met Thr Gly Asp Thr Tyr Thr Ala His Ala Gly Ala Lys
   385 390 395 400
   Phe Pro Ile Lys Trp Thr Ala Pro Glu Ser Leu Ala Tyr Asn Lys Phe
   405 410 415
   Ser Ile Lys Ser Asp Val Trp Ala Phe Gly Val Leu Leu Trp Glu Ile
   420 425 430
   Ala Thr Tyr Gly Met Ser Pro Tyr Pro Gly Ile Asp Arg Ser Gln Val
   435 440 445
   Tyr Glu Leu Leu Glu Lys Asp Tyr Arg Met Lys Arg Pro Glu Gly Cys
   450 455 460
   Pro Glu Lys Val Tyr Glu Leu Met Arg Ala Cys Trp Gln Trp Asn Pro
   465 470 475 480
   Ser Asp Arg Pro Ser Phe Ala Glu Ile His Gln Ala Phe Glu Thr Met
   485 490 495
   Phe Gln Glu Ser Ser Ile Ser Asp Glu Val Glu Lys Glu Leu Gly Lys
   500 505 510
   Gln Gly Val Arg Gly Ala Val Thr Thr Leu Leu Gln Ala Pro Glu Leu
   515 520 525
   Pro Thr Lys Thr Arg Thr Ser Arg Arg Ala Ala Glu His Arg Asp Thr
   530 535 540
   Thr Asp Val Pro Glu Met Pro His Ser Lys Gly Gln Gly Glu Ser Asp
   545 550 555 560
   Pro Leu Asp His Glu Pro Ala Val Ser Pro Leu Leu Pro Arg Lys Glu
   565 570 575
   Arg Gly Pro Pro Glu Gly Gly Leu Asn Glu Asp Glu Arg Leu Leu Pro
   580 585 590
   Lys Asp Lys Lys Thr Asn Leu Phe Ser Ala Leu Ile Lys Lys Lys Lys
   595 600 605
   Lys Thr Ala Pro Thr Pro Pro Lys Arg Ser Ser Ser Phe Arg Glu Met
   610 615 620
   Asp Gly Gln Pro Glu Arg Arg Gly Ala Gly Glu Glu Glu Gly Arg Asp
   625 630 635 640
   Ile Ser Asn Gly Ala Leu Ala Phe Thr Pro Leu Asp Thr Ala Asp Pro
   645 650 655
   Ala Lys Ser Pro Lys Pro Ser Asn Gly Ala Gly Val Pro Asn Gly Ala
   660 665 670
   Leu Arg Glu Ser Gly Gly Ser Gly Phe Arg Ser Pro His Leu Trp Lys
   675 680 685
   Lys Ser Ser Thr Leu Thr Ser Ser Arg Leu Ala Thr Gly Glu Glu Glu
   690 695 700
   Gly Gly Gly Ser Ser Ser Lys Arg Phe Leu Arg Ser Cys Ser Val Ser
   705 710 715 720
   Cys Val Pro His Gly Ala Lys Asp Thr Glu Trp Arg Ser Val Thr Leu
   725 730 735
   Pro Arg Asp Leu Gln Ser Thr Gly Arg Gln Phe Asp Ser Ser Thr Phe
   740 745 750
   Gly Gly His Lys Ser Glu Lys Pro Ala Leu Pro Arg Lys Arg Ala Gly
   755 760 765
   Glu Asn Arg Ser Asp Gln Val Thr Arg Gly Thr Val Thr Pro Pro Pro
   770 775 780
   Arg Leu Val Lys Lys Asn Glu Glu Ala Ala Asp Glu Val Phe Lys Asp
   785 790 795 800
   Ile Met Glu Ser Ser Pro Gly Ser Ser Pro Pro Asn Leu Thr Pro Lys
   805 810 815
   Pro Leu Arg Arg Gln Val Thr Val Ala Pro Ala Ser Gly Leu Pro His
   820 825 830
   Lys Glu Glu Ala Trp Lys Gly Ser Ala Leu Gly Thr Pro Ala Ala Ala
   835 840 845
   Glu Pro Val Thr Pro Thr Ser Lys Ala Gly Ser Gly Ala Pro Arg Gly
   850 855 860
   Thr Ser Lys Gly Pro Ala Glu Glu Ser Arg Val Arg Arg His Lys His
   865 870 875 880
   Ser Ser Glu Ser Pro Gly Arg Asp Lys Gly Lys Leu Ser Lys Leu Lys
   885 890 895
   Pro Ala Pro Pro Pro Pro Pro Ala Ala Ser Ala Gly Lys Ala Gly Gly
   900 905 910
   Lys Pro Ser Gln Arg Pro Gly Gln Glu Ala Ala Gly Glu Ala Val Leu
   915 920 925
   Gly Ala Lys Thr Lys Ala Thr Ser Leu Val Asp Ala Val Asn Ser Asp
   930 935 940
   Ala Ala Lys Pro Ser Gln Pro Ala Glu Gly Leu Lys Lys Pro Val Leu
   945 950 955 960
   Pro Ala Thr Pro Lys Pro His Pro Ala Lys Pro Ser Gly Thr Pro Ile
   965 970 975
   Ser Pro Ala Pro Val Pro Leu Ser Thr Leu Pro Ser Ala Ser Ser Ala
   980 985 990
   Leu Ala Gly Asp Gln Pro Ser Ser Thr Ala Phe Ile Pro Leu Ile Ser
   995 1000 1005
   Thr Arg Val Ser Leu Arg Lys Thr Arg Gln Pro Pro Glu Arg Ala Ser
   1010 1015 1020
   Gly Ala Ile Thr Lys Gly Val Val Leu Asp Ser Thr Glu Ala Leu Cys
   1025 1030 1035 1040
   Leu Ala Ile Ser Gly Asn Ser Glu Gln Met Ala Ser His Ser Ala Val
   1045 1050 1055
   Leu Glu Ala Gly Lys Asn Leu Tyr Thr Phe Cys Val Ser Tyr Val Asp
   1060 1065 1070
   Ser Ile Gln Gln Met Arg Asn Lys Phe Ala Phe Arg Glu Ala Ile Asn
   1075 1080 1085
   Lys Leu Glu Asn Asn Leu Arg Glu Leu Gln Ile Cys Pro Ala Ser Ala
   1090 1095 1100
   Gly Ser Gly Pro Ala Ala Thr Gln Asp Phe Ser Lys Leu Leu Ser Ser
   1105 1110 1115 1120
   Val Lys Glu Ile Ser Asp Ile Val Gln Arg
   1125 1130
<210> 5
   <211> 1461
   <212> DNA
   <213> Homo sapiens
<220> MKK7
   <223> Description of Sequence: N/A
<400> 5
   aggcggtgtt tgtctgccgg actgacgggc ggccgggcgg tgcgcggcgg cggtggcggc 60
   ggggaaaatg gcggcgtcct ccctggaaca gaagctgtcc cgcctggaag caaagctgaa 120
   gcaggagaac cgggaggccc ggcggaggat cgacctcaac ctggatatca gcccccagcg 180
   gcccaggccc accctgcagc tcccgctggc caacgatggg ggcagccgct cgccatcctc 240
   agagagctcc ccgcagcacc ccacgccccc cgcccggccc cgccacatgc tggggctccc 300
   gtcaaccctg ttcacacccc gcagcatgga gagcattgag attgaccaga agctgcagga 360
   gatcatgaag cagacgggct acctgaccat cgggggccag cgctaccagg cagaaatcaa 420
   cgacctggag aacttgggcg agatgggcag cggcacctgc ggccaggtgt ggaagatgcg 480
   cttccggaag accggccacg tcattgccgt taagcaaatg cggcgctccg ggaacaagga 540
   ggagaacaag cgcatcctca tggacctgga tgtggtgctg aagagccacg actgccccta 600
   catcgtgcag tgctttggga cgttcatcac caacacggac gtcttcatcg ccatggagct 660
   catgggcacc tgcgctgaga agctcaagaa gcggatgcag ggccccatcc ccgagcgcat 720
   tctgggcaag atgacagtgg cgattgtgaa ggcgctgtac tacctgaagg agaagcacgg 780
   tgtcatccac cgcgacgtca agccctccaa catcctgctg gacgagcggg gccagatcaa 840
   gttctgcgac ttcggcatca gcggccgcct ggtggactcc aaagccaaga cgcggagcgc 900
   cggctgtgcc gcctacatgg cacccgagcg cattgacccc ccagacccca ccaagccgga 960
   ctatgacatc cgggccgacg tatggagcct gggcatctcg ctggtggagc tggcaacagg 1020
   acagtttccc tacaagaact gcaagacgga ctttgaggtc ctcaccaaag tcctacagga 1080
   agagcccccg cttctgcccg gacacatggg cttctcgggg gacttccagt ccttcgtcaa 1140
   agactgcctt actaaagatc acaggaagag accaaagtat aataagctac ttgaacacag 1200
   cttcatcaag cgctacgaga cgctggaggt ggacgtggcg tcctggttca aggatgtcat 1260
   ggcgaagact gagtcaccgc ggactagcgg cgtcctgagc cagccccacc tgcccttctt 1320
   caggtagctg cttggcggcg gccagcccca caggggccca ggggcatggc cacaggcccc 1380
   cctccccact tggccaccca gctgcctgcc aggggagacc tgggacctgg acggccacct 1440
   aggactgagg acagagagtg g                                      1461
<210> 6
   <211> 419
   <212> PRT
   <213> Homo sapiens
<220>
   <223> Description of Sequence: N/A
<400> 6
<210> 7
   <211> 1050
   <212> DNA
   <213> Homo sapiens
<220> CDC2
   <223> Description of Sequence: N/A
<400> 7
   gggggggggg ggcacttggc ttcaaagctg gctcttggaa attgagcgga gacgagcggc 60
   ttgttgtagc tgccgtgcgg ccgccgcgga ataataagcc gggatctacc ataccattga 120
   ctaactatgg aagattatac caaaatagag aaaattggag aaggtaccta tggagttgtg 180
   tataagggta gacacaaaac tacaggtcaa gtggtagcca tgaaaaaaat cagactagaa 240
   agtgaagagg aaggggttcc tagtactgca attcgggaaa tttctctatt aaaggaactt 300
   cgtcatccaa atatagtcag tcttcaggat gtgcttatgc aggattccag gttatatctc 360
   atctttgagt ttctttccat ggatctgaag aaatacttgg attctatccc tcctggtcag 420
   tacatggatt cttcacttgt taagagttat ttataccaaa tcctacaggg gattgtgttt 480
   tgtcactcta gaagagttct tcacagagac ttaaaacctc aaaatctctt gattgatgac 540
   aaaggaacaa ttaaactggc tgattttggc cttgccagag cttttggaat acctatcaga 600
   gtatatacac atgaggtagt aacactctgg tacagatctc cagaagtatt gctggggtca 660
   gctcgttact caactccagt tgacatttgg agtataggca ccatatttgc tgaactagca 720
   actaagaaac cacttttcca tggggattca gaaattgatc aactcttcag gattttcaga 780
   gctttgggca ctcccaataa tgaagtgtgg ccagaagtgg aatctttaca ggactataag 840
   aatacatttc ccaaatggaa accaggaagc ctagcatccc atgtcaaaaa cttggatgaa 900
   aatggcttgg atttgctctc gaaaatgtta atctatgatc cagccaaacg aatttctggc 960
   aaaatggcac tgaatcatcc atattttaat gatttggaca atcagattaa gaagatgtag 1020
   ctttctgaca aaaagtttcc atatgttatg                                  1050
<210> 8
   <211> 297
   <212> PRT
   <213> Homo sapiens
<220>
   <223> Description of Sequence: N/A
<400> 8
   Met Glu Asp Tyr Thr Lys Ile Glu Lys Ile Gly Glu Gly Thr Tyr Gly
     1               5                  10                  15
   Val Val Tyr Lys Gly Arg His Lys Thr Thr Gly Gln Val Val Ala Met
               20                  25                  30
   Lys Lys Ile Arg Leu Glu Ser Glu Glu Glu Gly Val Pro Ser Thr Ala
           35                  40                  45
   Ile Arg Glu Ile Ser Leu Leu Lys Glu Leu Arg His Pro Asn Ile Val
       50                  55                  60
   Ser Leu Gln Asp Val Leu Met Gln Asp Ser Arg Leu Tyr Leu Ile Phe
    65                  70                  75                  80
   Glu Phe Leu Ser Met Asp Leu Lys Lys Tyr Leu Asp Ser Ile Pro Pro
                   85                  90                  95
   Gly Gln Tyr Met Asp Ser Ser Leu Val Lys Ser Tyr Leu Tyr Gln Ile
               100                 105                 110
   Leu Gln Gly Ile Val Phe Cys His Ser Arg Arg Val Leu His Arg Asp
           115                 120                 125
   Leu Lys Pro Gln Asn Leu Leu Ile Asp Asp Lys Gly Thr Ile Lys Leu
       130                 135                 140
   Ala Asp Phe Gly Leu Ala Arg Ala Phe Gly Ile Pro Ile Arg Val Tyr
   145                 150                 155                 160
   Thr His Glu Val Val Thr Leu Trp Tyr Arg Ser Pro Glu Val Leu Leu
                   165                 170                 175
   Gly Ser Ala Arg Tyr Ser Thr Pro Val Asp Ile Trp Ser Ile Gly Thr
               180                 185                 190
   Ile Phe Ala Glu Leu Ala Thr Lys Lys Pro Leu Phe His Gly Asp Ser
           195                 200                 205
   Glu Ile Asp Gln Leu Phe Arg Ile Phe Arg Ala Leu Gly Thr Pro Asn
       210                 215                 220
   Asn Glu Val Trp Pro Glu Val Glu Ser Leu Gln Asp Tyr Lys Asn Thr
   225                 230                 235                 240
   Phe Pro Lys Trp Lys Pro Gly Ser Leu Ala Ser His Val Lys Asn Leu
                   245                 250                 255
   Asp Glu Asn Gly Leu Asp Leu Leu Ser Lys Met Leu Ile Tyr Asp Pro
               260                 265                 270
   Ala Lys Arg Ile Ser Gly Lys Met Ala Leu Asn His Pro Tyr Phe Asn
           275                 280                 285
   Asp Leu Asp Asn Gln Ile Lys Lys Met
       290                 295
<210> 9
   <211> 1480
   <212> DNA
   <213> Homo sapiens
<220> CamKI
   <223> Description of Sequence: N/A
<400> 9
   gaattccgag caagagcgcg ggcgggtggc ccaggcacgc agcgggtgag gaccgcgccc 60
   acagctcggc gccaaccacc gcgggcctcc cagccagccc cgcnnngagc cgcaggancc 120
   ctggctgtgg tcggggggca gtgggccatg ctgggggcag tggaaggccc caggtggaag 180
   caggcggagg acattagaga catctacgac ttccgagatg ttctgggcac gggggccttc 240
   tcggaggtga tcctggcaga agataagagg acgcagaagc tggtggccat caaatgcatt 300
   gccaaggagg ccctggaggg caaggaaggc agcatggaga atgagattgc tgtcctgcac 360
   aagatcaagc accccaacat tgtagccctg gatgacatct atgagagtgg gggccacctc 420
   tacctcatca tgcagctggt gtcgggtggg gagctctttg accgtattgt ggaaaaaggc 480
   ttctacacgg agcgggacgc cagccgcctc atcttccagg tgctggatgc tgtgaaatac 540
   ctgcatgacc tgggcattgt acaccgggat ctcaagccag agaatctgct gtactacagc 600
   ctggatgaag actccaaaat catgatctcc gactttggcc tctccaagat ggaggacccg 660
   ggcagtgtgc tctccaccgc ctgtggaact ccgggatacg tggcccctga agtcctggcc 720
   cagaagccct acagcaaggc tgtggattgc tggtccatag gtgtcatcgc ctacatcttg 780
   ctctgcggtt accctccctt ctatgacgag aatgatgcca aactctttga acagattttg 840
   aaggccgagt acgagtttga ctctccttac tgggacgaca tctctgactc tgccaaagat 900
   ttcatccggc acttgatgga gaaggaccca gagaaaagat tcacctgtga gcaggccttg 960
   cagcacccat ggattgcagg agatacagct ctagataaga atatccacca gtcggtgagt 1020
   gagcagatca agaagaactt tgccaagagc aagtggaagc aagccttcaa tgccacggct 1080
   gtggtgcggc acatgaggaa actgcagctg ggcaccagcc aggaggggca ggggcagacg 1140
   gcgagccatg gggagctgct gacaccagtg gctggggggc cggcagctgg ctgttgctgt 1200
   cgagactgct gcgtggagcc gggcacagaa ctgtccccca cactgcccca ccagctctag 1260
   ggccctggac ctcgggtcat gatcctctgc gtgggagggc ttgggggcca gcctgctccc 1320
   cttccctccc tgaaccggga gtttctctgc cctgtcccct cctcacctgc ttccctacca 1380
   ctcctcactg cattttccat acaaatgttt ctattttatt gttccttctt gtaataaagg 1440
   gaagataaaa ccaaaaaaaa aaaaaaaaaa acggaattcc                       1480
<210> 10
   <211> 370
   <212> PRT
   <213> Homo sapiens
<220>
   <223> Description of Sequence: N/A
<400> 10
   Met Leu Gly Ala Val Glu Gly Pro Arg Trp Lys Gln Ala Glu Asp Ile
   1 5 10 15
   Arg Asp Ile Tyr Asp Phe Arg Asp Val Leu Gly Thr Gly Ala Phe Ser
   20 25 30
   Glu Val Ile Leu Ala Glu Asp Lys Arg Thr Gln Lys Leu Val Ala Ile
   35 40 45
   Lys Cys Ile Ala Lys Glu Ala Leu Glu Gly Lys Glu Gly Ser Met Glu
   50 55 60
   Asn Glu Ile Ala Val Leu His Lys Ile Lys His Pro Asn Ile Val Ala
   65 70 75 80
   Leu Asp Asp Ile Tyr Glu Ser Gly Gly His Leu Tyr Leu Ile Met Gln
   85 90 95
   Leu Val Ser Gly Gly Glu Leu Phe Asp Arg Ile Val Glu Lys Gly Phe
   100 105 110
   Tyr Thr Glu Arg Asp Ala Ser Arg Leu Ile Phe Gln Val Leu Asp Ala
   115 120 125
   Val Lys Tyr Leu His Asp Leu Gly Ile Val His Arg Asp Leu Lys Pro
   130 135 140
   Glu Asn Leu Leu Tyr Tyr Ser Leu Asp Glu Asp Ser Lys Ile Met Ile
   145 150 155 160
   Ser Asp Phe Gly Leu Ser Lys Met Glu Asp Pro Gly Ser Val Leu Ser
   165 170 175
   Thr Ala Cys Gly Thr Pro Gly Tyr Val Ala Pro Glu Val Leu Ala Gln
   180 185 190
   Lys Pro Tyr Ser Lys Ala Val Asp Cys Trp Ser Ile Gly Val Ile Ala
   195 200 205
   Tyr Ile Leu Leu Cys Gly Tyr Pro Pro Phe Tyr Asp Glu Asn Asp Ala
   210 215 220
   Lys Leu Phe Glu Gln Ile Leu Lys Ala Glu Tyr Glu Phe Asp Ser Pro
   225 230 235 240
   Tyr Trp Asp Asp Ile Ser Asp Ser Ala Lys Asp Phe Ile Arg His Leu
   245 250 255
   Met Glu Lys Asp Pro Glu Lys Arg Phe Thr Cys Glu Gln Ala Leu Gln
   260 265 270
   His Pro Trp Ile Ala Gly Asp Thr Ala Leu Asp Lys Asn Ile His Gln
   275 280 285
   Ser Val Ser Glu Gln Ile Lys Lys Asn Phe Ala Lys Ser Lys Trp Lys
   290 295 300
   Gln Ala Phe Asn Ala Thr Ala Val Val Arg His Met Arg Lys Leu Gln
   305 310 315 320
   Leu Gly Thr Ser Gln Glu Gly Gln Gly Gln Thr Ala Ser His Gly Glu
   325 330 335
   Leu Leu Thr Pro Val Ala Gly Gly Pro Ala Ala Gly Cys Cys Cys Arg
   340 345 350
   Asp Cys Cys Val Glu Pro Gly Thr Glu Leu Ser Pro Thr Leu Pro His
   355 360 365
   Gln Leu
   370
<210> 11
   <211> 1782
   <212> DNA
   <213> Homo sapiens
<220> JNK2
   <223> Description of Sequence: N/A
<400> 11
   gggcgggcga gggatctgaa acttgcccac ccttcgggat attgcaggac gctgcatcat 60
   gagcgacagt aaatgtgaca gtcagtttta tagtgtgcaa gtggcagact caaccttcac 120
   tgtcctaaaa cgttaccagc agctgaaacc aattggctct ggggcccaag ggattgtttg 180
   tgctgcattt gatacagttc ttgggataag tgttgcagtc aagaaactaa gccgtccttt 240
   tcagaaccaa actcatgcaa agagagctta tcgtgaactt gtcctcttaa aatgtgtcaa 300
   tcataaaaat ataattagtt tgttaaatgt gtttacacca caaaaaactc tagaagaatt 360
   tcaagatgtg tatttggtta tggaattaat ggatgctaac ttatgtcagg ttattcacat 420
   ggagctggat catgaaagaa tgtcctacct tctttaccag atgctttgtg gtattaaaca 480
   tctgcattca gctggtataa ttcatagaga tttgaagcct agcaacattg ttgtgaaatc 540
   agactgcacc ctgaagatcc ttgactttgg cctggcccgg acagcgtgca ctaacttcat 600
   gatgacccct tacgtggtga cacggtacta ccgggcgccc gaagtcatcc tgggtatggg 660
   ctacaaagag aacgttgata tctggtcagt gggttgcatc atgggagagc tggtgaaagg 720
   ttgtgtgata ttccaaggca ctgaccatat tgatcagtgg aataaagtta ttgagcagct 780
   gggaacacca tcagcagagt tcatgaagaa acttcagcca actgtgagga attatgtcga 840
   aaacagacca aagtatcctg gaatcaaatt tgaagaactc tttccagatt ggatattccc 900
   atcagaatct gagcgagaca aaataaaaac aagtcaagcc agagatctgt tatcaaaaat 960
   gttagtgatt gatcctgaca agcggatctc tgtagacgaa gctctgcgtc acccatacat 1020
   cactgtttgg tatgaccccg ccgaagcaga agccccacca cctcaaattt atgatgccca 1080
   gttggaagaa agagaacatg caattgaaga atggaaagag ctaatttaca aagaagtcat 1140
   ggattgggaa gaaagaagca agaatggtgt tgtaaaagat cagccttcag atgcagcagt 1200
   aagtagcaac gccactcctt ctcagtcttc atcgatcaat gacatttcat ccatgtccac 1260
   tgagcagacg ctggcctcag acacagacag cagtcttgat gcctcgacgg gaccccttga 1320
   aggctgtcga tgataggtta gaaatagcaa acctgtcagc attgaaggaa ctctcacctc 1380
   cgtgggcctg aaatgcttgg gagttgatgg aaccaaatag aaaaactcca tgttctgcat 1440
   gtaagaaaca caatgccttg ccctattcag acctgatagg attgcctgct tagatgataa 1500
   aatgaggcag aatatgtctg aagaaaaaaa ttgcaagcca cacttctaga gattttgttc 1560
   aagatcattt caggtgagca gttagagtag gtgaatttgt ttcaaattgt actagtgaca 1620
   gtttctcatc atctgtaact gttgagatgt atgtgcatgt gaccacaaat gcttgcttgg 1680
   acttgcccat ctagcacttt ggaaatcagt atttaaatgc caaataatct tccaggtagt 1740
   gctgcttctg aagttatctc ttaatcctct taagtaattt gg                   1782
<210> 12
   <211> 424
   <212> PRT
   <213> Homo sapiens
<220>
   <223> Description of Sequence: N/A
<400> 12
<210> 13
   <211> 3668
   <212> DNA
   <213> Homo sapiens
<220> LIMK-2
   <223> Description of Sequence: N/A
<400> 13
   gtggtcttcc cgcgcctgag gcggcggcgg caggagctga ggggagttgt agggaactga 60
   ggggagctgc tgtgtccccc gcctcctcct ccccatttcc gggctcccgg gaccatgtcc 120
   gcgctggcgg gtgaagatgt ctggaggtgt ccaggctgtg gggaccacat tgctccaagc 180
   cagatatggt acaggactgt caacgaaacc tggcacggct cttgcttccg gtgttcagaa 240
   tgccaggatt ccctcaccaa ctggtactat gagaaggatg ggaagctcta ctgccccaag 300
   gactactggg ggaagtttgg ggagttctgt catgggtgct ccctgctgat gacagggcct 360
   tttatggtgg ctggggagtt caagtaccac ccagagtgct ttgcctgtat gagctgcaag 420
   gtgatcattg aggatgggga tgcatatgca ctggtgcagc atgccaccct ctactgtggg 480
   aagtgccaca atgaggtggt gctggcaccc atgtttgaga gactctccac agagtctgtt 540
   caggagcagc tgccctactc tgtcacgctc atctccatgc cggccaccac tgaaggcagg 600
   cggggcttct ccgtgtccgt ggagagtgcc tgctccaact acgccaccac tgtgcaagtg 660
   aaagaggtca accggatgca catcagtccc aacaatcgaa acgccatcca ccctggggac 720
   cgcatcctgg agatcaatgg gacccccgtc cgcacacttc gagtggagga ggtggaggat 780
   gcaattagcc agacgagcca gacacttcag ctgttgattg aacatgaccc cgtctcccaa 840
   cgcctggacc agctgcggct ggaggcccgg ctcgctcctc acatgcagaa tgccggacac 900
   ccccacgccc tcagcaccct ggacaccaag gagaatctgg aggggacact gaggagacgt 960
   tccctaaggc gcagtaacag tatctccaag tcccctggcc ccagctcccc aaaggagccc 1020
   ctgctgttca gccgtgacat cagccgctca gaatcccttc gttgttccag cagctattca 1080
   cagcagatct tccggccctg tgacctaatc catggggagg tcctggggaa gggcttcttt 1140
   gggcaggcta tcaaggtgac acacaaagcc acgggcaaag tgatggtcat gaaagagtta 1200
   attcgatgtg atgaggagac ccagaaaact tttctgactg aggtgaaagt gatgcgcagc 1260
   ctggaccacc ccaatgtgct caagttcatt ggtgtgctgt acaaggataa gaagctgaac 1320
   ctgctgacag agtacattga ggggggcaca ctgaaggact ttctgcgcag tatggatccg 1380
   ttcccctggc agcagaaggt caggtttgcc aaaggaatcg cctccggaat ggcctatttg 1440
   cactctatgt gcatcatcca ccgggatctg aactcgcaca actgcctcat caagttggac 1500
   aagactgtgg tggtggcaga ctttgggctg tcacggctca tagtggaaga gaggaaaagg 1560
   gcccccatgg agaaggccac caccaagaaa cgcaccttgc gcaagaacga ccgcaagaag 1620
   cgctacacgg tggtgggaaa cccctactgg atggcccctg agatgctgaa cggaaagagc 1680
   tatgatgaga cggtggatat cttctccttt gggatcgttc tctgtgagat cattgggcag 1740
   gtgtatgcag atcctgactg ccttccccga acactggact ttggcctcaa cgtgaagctt 1800
   ttctgggaga agtttgttcc cacagattgt cccccggcct tcttcccgct ggccgccatc 1860
   tgctgcagac tggagcctga gagcagacca gcattctcga aattggagga ctcctttgag 1920
   gccctctccc tgtacctggg ggagctgggc atcccgctgc ctgcagagct ggaggagttg 1980
   gaccacactg tgagcatgca gtacggcctg acccgggact cacctcccta gccctggccc 2040
   agccccctgc aggggggtgt tctacagcca gcattgcccc tctgtgcccc attcctgctg 2100
   tgagcagggc cgtccgggct tcctgtggat tggcggaatg tttagaagca gaacaagcca 2160
   ttcctattac ctccccagga ggcaagtggg cgcagcacca gggaaatgta tctccacagg 2220
   ttctggggcc tagttactgt ctgtaaatcc aatacttgcc tgaaagctgt gaagaagaaa 2280
   aaaacccctg gcctttgggc caggaggaat ctgttactcg aatccaccca ggaactccct 2340
   ggcagtggat tgtgggaggc tcttgcttac actaatcagc gtgacctgga cctgctgggc 2400
   aggatcccag ggtgaacctg cctgtgaact ctgaagtcac tagtccagct gggtgcagga 2460
   ggacttcaag tgtgtggacg aaagaaagac tgatggctca aagggtgtga aaaagtcagt 2520
   gatgctcccc ctttctactc cagatcctgt ccttcctgga gcaaggttga gggagtaggt 2580
   tttgaagagt cccttaatat gtggtggaac aggccaggag ttagagaaag ggctggcttc 2640
   tgtttacctg ctcactggct ctagccagcc cagggaccac atcaatgtga gaggaagcct 2700
   ccacctcatg ttttcaaact taatactgga gactggctga gaacttacgg acaacatcct 2760
   ttctgtctga aacaaacagt cacaagcaca ggaagaggct gggggactag aaagaggccc 2820
   tgccctctag aaagctcaga tcttggcttc tgttactcat actcgggtgg gctccttagt 2880
   cagatgccta aaacattttg cctaaagctc gatgggttct ggaggacagt gtggcttgtc 2940
   acaggcctag agtctgaggg aggggagtgg gagtctcagc aatctcttgg tcttggcttc 3000
   atggcaacca ctgctcaccc ttcaacatgc ctggtttagg cagcagcttg ggctgggaag 3060
   aggtggtggc agagtctcaa agctgagatg ctgagagaga tagctccctg agctgggcca 3120
   tctgacttct acctcccatg tttgctctcc caactcatta gctcctgggc agcatcctcc 3180
   tgagccacat gtgcaggtac tggaaaacct ccatcttggc tcccagagct ctaggaactc 3240
   ttcatcacaa ctagatttgc ctcttctaag tgtctatgag cttgcaccat atttaataaa 3300
   ttgggaatgg gtttggggta ttaatgcaat gtgtggtggt tgtattggag cagggggaat 3360
   tgataaagga gagtggttgc tgttaatatt atcttatcta ttgggtggta tgtgaaatat 3420
   tgtacataga cctgatgagt tgtgggacca gatgtcatct ctggtcagag tttacttgct 3480
   atatagactg tacttatgtg tgaagtttgc aagcttgctt tagggctgag ccctggactc 3540
   ccagcagcag cacagttcag cattgtgtgg ctggttgttt cctggctgtc cccagcaagt 3600
   gtaggagtgg tgggcctgaa ctgggccatt gatcagacta aataaattaa gcagttaaca 3660
   taactggc 3668
<210> 14
   <211> 638
   <212> PRT
   <213> Homo sapiens
<220>
   <223> Description of Sequence: N/A
<400> 14
   Met Ser Ala Leu Ala Gly Glu Asp Val Trp Arg Cys Pro Gly Cys Gly
   1 5 10 15
   Asp His Ile Ala Pro Ser Gln Ile Trp Tyr Arg Thr Val Asn Glu Thr
   20 25 30
   Trp His Gly Ser Cys Phe Arg Cys Ser Glu Cys Gln Asp Ser Leu Thr
   35 40 45
   Asn Trp Tyr Tyr Glu Lys Asp Gly Lys Leu Tyr Cys Pro Lys Asp Tyr
   50 55 60
   Trp Gly Lys Phe Gly Glu Phe Cys His Gly Cys Ser Leu Leu Met Thr
   65 70 75 80
   Gly Pro Phe Met Val Ala Gly Glu Phe Lys Tyr His Pro Glu Cys Phe
   85 90 95
   Ala Cys Met Ser Cys Lys Val Ile Ile Glu Asp Gly Asp Ala Tyr Ala
   100 105 110
   Leu Val Gln His Ala Thr Leu Tyr Cys Gly Lys Cys His Asn Glu Val
   115 120 125
   Val Leu Ala Pro Met Phe Glu Arg Leu Ser Thr Glu Ser Val Gln Glu
   130 135 140
   Gln Leu Pro Tyr Ser Val Thr Leu Ile Ser Met Pro Ala Thr Thr Glu
   145 150 155 160
   Gly Arg Arg Gly Phe Ser Val Ser Val Glu Ser Ala Cys Ser Asn Tyr
   165 170 175
   Ala Thr Thr Val Gln Val Lys Glu Val Asn Arg Met His Ile Ser Pro
   180 185 190
   Asn Asn Arg Asn Ala Ile His Pro Gly Asp Arg Ile Leu Glu Ile Asn
   195 200 205
   Gly Thr Pro Val Arg Thr Leu Arg Val Glu Glu Val Glu Asp Ala Ile
   210 215 220
   Ser Gln Thr Ser Gln Thr Leu Gln Leu Leu Ile Glu His Asp Pro Val
   225 230 235 240
   Ser Gln Arg Leu Asp Gln Leu Arg Leu Glu Ala Arg Leu Ala Pro His
   245 250 255
   Met Gln Asn Ala Gly His Pro His Ala Leu Ser Thr Leu Asp Thr Lys
   260 265 270
   Glu Asn Leu Glu Gly Thr Leu Arg Arg Arg Ser Leu Arg Arg Ser Asn
   275 280 285
   Ser Ile Ser Lys Ser Pro Gly Pro Ser Ser Pro Lys Glu Pro Leu Leu
   290 295 300
   Phe Ser Arg Asp Ile Ser Arg Ser Glu Ser Leu Arg Cys Ser Ser Ser
   305 310 315 320
   Tyr Ser Gln Gln Ile Phe Arg Pro Cys Asp Leu Ile His Gly Glu Val
   325 330 335
   Leu Gly Lys Gly Phe Phe Gly Gln Ala Ile Lys Val Thr His Lys Ala
   340 345 350
   Thr Gly Lys Val Met Val Met Lys Glu Leu Ile Arg Cys Asp Glu Glu
   355 360 365
   Thr Gln Lys Thr Phe Leu Thr Glu Val Lys Val Met Arg Ser Leu Asp
   370 375 380
   His Pro Asn Val Leu Lys Phe Ile Gly Val Leu Tyr Lys Asp Lys Lys
   385 390 395 400
   Leu Asn Leu Leu Thr Glu Tyr Ile Glu Gly Gly Thr Leu Lys Asp Phe
   405 410 415
   Leu Arg Ser Met Asp Pro Phe Pro Trp Gln Gln Lys Val Arg Phe Ala
   420 425 430
   Lys Gly Ile Ala Ser Gly Met Ala Tyr Leu His Ser Met Cys Ile Ile
   435 440 445
   His Arg Asp Leu Asn Ser His Asn Cys Leu Ile Lys Leu Asp Lys Thr
   450 455 460
   Val Val Val Ala Asp Phe Gly Leu Ser Arg Leu Ile Val Glu Glu Arg
   465 470 475 480
   Lys Arg Ala Pro Met Glu Lys Ala Thr Thr Lys Lys Arg Thr Leu Arg
   485 490 495
   Lys Asn Asp Arg Lys Lys Arg Tyr Thr Val Val Gly Asn Pro Tyr Trp
   500 505 510
   Met Ala Pro Glu Met Leu Asn Gly Lys Ser Tyr Asp Glu Thr Val Asp
   515 520 525
   Ile Phe Ser Phe Gly Ile Val Leu Cys Glu Ile Ile Gly Gln Val Tyr
   530 535 540
   Ala Asp Pro Asp Cys Leu Pro Arg Thr Leu Asp Phe Gly Leu Asn Val
   545 550 555 560
   Lys Leu Phe Trp Glu Lys Phe Val Pro Thr Asp Cys Pro Pro Ala Phe
   565 570 575
   Phe Pro Leu Ala Ala Ile Cys Cys Arg Leu Glu Pro Glu Ser Arg Pro
   580 585 590
   Ala Phe Ser Lys Leu Glu Asp Ser Phe Glu Ala Leu Ser Leu Tyr Leu
   595 600 605
   Gly Glu Leu Gly Ile Pro Leu Pro Ala Glu Leu Glu Glu Leu Asp His
   610 615 620
   Thr Val Ser Met Gln Tyr Gly Leu Thr Arg Asp Ser Pro Pro
   625 630 635
<210> 15
   <211> 2169
   <212> DNA
   <213> Homo sapiens
<220> PRK
   <223> Description of Sequence: N/A
<400> 15
   ccgcctccga gtgccttgcg cggacctgag ctggagatgc tggccgggct accgacgtca 60
   gaccccgggc gcctcatcac ggacccgcgc agcggccgca cctacctcaa aggccgcttg 120
   ttgggcaagg ggggcttcgc ccgctgctac gaggccactg acacagagac tggcagcgcc 180
   tacgctgtca aagtcatccc gcagagccgc gtcgccaagc cgcatcagcg cgagaagatc 240
   ctaaatgaga ttgagctgca ccgagacctg cagcaccgcc acatcgtgcg tttttcgcac 300
   cactttgagg acgctgacaa catctacatt ttcttggagc tctgcagccg aaagtccctg 360
   gcccacatct ggaaggcccg gcacaccctg ttggagccag aagtgcgcta ctacctgcgg 420
   cagatccttt ctggcctcaa gtacttgcac cagcgcggca tcttgcaccg ggacctcaag 480
   ttgggaaatt ttttcatcac tgagaacatg gaactgaagg tgggggattt tgggctggca 540
   gcccggttgg agcctccgga gcagaggaag aagaccatct gtggcacccc caactatgtg 600
   gctccagaag tgctgctgag acagggccac ggccctgaag cggatgtatg gtcactgggc 660
   tgtgtcatgt acacgctgct ctgcgggagc cctccctttg agacggctga cctgaaggag 720
   acgtaccgct gcatcaagca ggttcactac acgctgcctg ccagcctctc actgcctgcc 780
   cggcagctcc tggccgccat ccttcgggcc tcaccccgag accgcccctc tattgaccag 840
   atcctgcgcc atgacttctt taccaagggc tacacccccg atcgactccc tatcagcagc 900
   tgcgtgacag tcccagacct gacacccccc aacccagcta ggagtctgtt tgccaaagtt 960
   accaagagcc tctttggcag aaagaagaag agtaagaatc atgcccagga gagggatgag 1020
   gtctccggtt tggtgagcgg cctcatgcgc acatccgttg gccatcagga tgccaggcca 1080
   gaggctccag cagcttctgg cccagcccct gtcagcctgg tagagacagc acctgaagac 1140
   agctcacccc gtgggacact ggcaagcagt ggagatggat ttgaagaagg tctgactgtg 1200
   gccacagtag tggagtcagc cctttgtgct ctgagaaatt gtatagcttt catgccccca 1260
   gcggaacaga acccggcccc cctggcccag ccagagcctc tggtgtgggt cagcaagtgg 1320
   gttgactact ccaataagtt cggctttggg tatcaactgt ccagccgccg tgtggctgtg 1380
   ctcttcaacg atggcacaca tatggccctg tcggccaaca gaaagactgt gcactacaat 1440
   cccaccagca caaagcactt ctccttctcc gtgggtgctg tgccccgggc cctgcagcct 1500
   cagctgggta tcctgcggta cttcgcctcc tacatggagc agcacctcat gaagggtgga 1560
   gatctgccca gtgtggaaga ggtagaggta cctgctccgc ccttgctgct gcagtgggtc 1620
   aagacggatc aggctctcct catgctgttt agtgatggca ctgtccaggt gaacttctac 1680
   ggggaccaca ccaagctgat tctcagtggc tgggagcccc tccttgtgac ttttgtggcc 1740
   cgaaatcgta gtgcttgtac ttacctcgct tcccaccttc ggcagctggg ctgctctcca 1800
   gacctgcggc agcgactccg ctatgctctg cgcctgctcc gggaccgcag cccagcttag 1860
   gacccaagcc ctgaaggcct gaggcctgtg cctgtcaggc tctggccctt gcctttgtgg 1920
   ccttccccct tcctttggtg cctcactggg ggctttgggc cgaatccccc agggaatcag 1980
   ggaccagctt tactggagtt gggggcggct tgtcttcgct ggctcctacc ccatctccaa 2040
   gataagcctg agccttagct cccagctagg gggcgttatt tatggaccac ttttatttat 2100
   tgtcagacac ttatttattg ggatgtgagc cccagggggc ctcctcctag gataataaac 2160
   aattttgca 2169
<210> 16
   <211> 607
   <212> PRT
   <213> Homo sapiens
<220>
   <223> Description of Sequence: N/A
<400> 16
   Met Leu Ala Gly Leu Pro Thr Ser Asp Pro Gly Arg Leu Ile Thr Asp
   1               5                   10                  15
   Pro Arg Ser Gly Arg Thr Tyr Leu Lys Gly Arg Leu Leu Gly Lys Gly
               20                  25                  30
   Gly Phe Ala Arg Cys Tyr Glu Ala Thr Asp Thr Glu Thr Gly Ser Ala
           35                  40                  45
   Tyr Ala Val Lys Val Ile Pro Gln Ser Arg Val Ala Lys Pro His Gln
       50                  55                  60
   Arg Glu Lys Ile Leu Asn Glu Ile Glu Leu His Arg Asp Leu Gln His
   65                  70                  75                  80
   Arg His Ile Val Arg Phe Ser His His Phe Glu Asp Ala Asp Asn Ile
                   85                  90                  95
   Tyr Ile Phe Leu Glu Leu Cys Ser Arg Lys Ser Leu Ala His Ile Trp
               100                 105                 110
   Lys Ala Arg His Thr Leu Leu Glu Pro Glu Val Arg Tyr Tyr Leu Arg
           115                 120                 125
   Gln Ile Leu Ser Gly Leu Lys Tyr Leu His Gln Arg Gly Ile Leu His
       130                 135                 140
   Arg Asp Leu Lys Leu Gly Asn Phe Phe Ile Thr Glu Asn Met Glu Leu
   145                 150                 155                 160
   Lys Val Gly Asp Phe Gly Leu Ala Ala Arg Leu Glu Pro Pro Glu Gln
                   165                 170                 175
   Arg Lys Lys Thr Ile Cys Gly Thr Pro Asn Tyr Val Ala Pro Glu Val
               180                 185                 190
   Leu Leu Arg Gln Gly His Gly Pro Glu Ala Asp Val Trp Ser Leu Gly
           195                 200                 205
   Cys Val Met Tyr Thr Leu Leu Cys Gly Ser Pro Pro Phe Glu Thr Ala
       210                 215                 220
   Asp Leu Lys Glu Thr Tyr Arg Cys Ile Lys Gln Val His Tyr Thr Leu
   225                 230                 235                 240
   Pro Ala Ser Leu Ser Leu Pro Ala Arg Gln Leu Leu Ala Ala Ile Leu
                   245                 250                 255
   Arg Ala Ser Pro Arg Asp Arg Pro Ser Ile Asp Gln Ile Leu Arg His
               260                 265                 270
   Asp Phe Phe Thr Lys Gly Tyr Thr Pro Asp Arg Leu Pro Ile Ser Ser
           275                 280                 285
   Cys Val Thr Val Pro Asp Leu Thr Pro Pro Asn Pro Ala Arg Ser Leu
       290                 295                 300
   Phe Ala Lys Val Thr Lys Ser Leu Phe Gly Arg Lys Lys Lys Ser Lys
   305 310 315 320
   Asn His Ala Gln Glu Arg Asp Glu Val Ser Gly Leu Val Ser Gly Leu
   325 330 335
   Met Arg Thr Ser Val Gly His Gln Asp Ala Arg Pro Glu Ala Pro Ala
   340 345 350
   Ala Ser Gly Pro Ala Pro Val Ser Leu Val Glu Thr Ala Pro Glu Asp
   355 360 365
   Ser Ser Pro Arg Gly Thr Leu Ala Ser Ser Gly Asp Gly Phe Glu Glu
   370 375 380
   Gly Leu Thr Val Ala Thr Val Val Glu Ser Ala Leu Cys Ala Leu Arg
   385 390 395 400
   Asn Cys Ile Ala Phe Met Pro Pro Ala Glu Gln Asn Pro Ala Pro Leu
   405 410 415
   Ala Gln Pro Glu Pro Leu Val Trp Val Ser Lys Trp Val Asp Tyr Ser
   420 425 430
   Asn Lys Phe Gly Phe Gly Tyr Gln Leu Ser Ser Arg Arg Val Ala Val
   435 440 445
   Leu Phe Asn Asp Gly Thr His Met Ala Leu Ser Ala Asn Arg Lys Thr
   450 455 460
   Val His Tyr Asn Pro Thr Ser Thr Lys His Phe Ser Phe Ser Val Gly
   465 470 475 480
   Ala Val Pro Arg Ala Leu Gln Pro Gln Leu Gly Ile Leu Arg Tyr Phe
   485 490 495
   Ala Ser Tyr Met Glu Gln His Leu Met Lys Gly Gly Asp Leu Pro Ser
   500 505 510
   Val Glu Glu Val Glu Val Pro Ala Pro Pro Leu Leu Leu Gln Trp Val
   515 520 525
   Lys Thr Asp Gln Ala Leu Leu Met Leu Phe Ser Asp Gly Thr Val Gln
   530 535 540
   Val Asn Phe Tyr Gly Asp His Thr Lys Leu Ile Leu Ser Gly Trp Glu
   545 550 555 560
   Pro Leu Leu Val Thr Phe Val Ala Arg Asn Arg Ser Ala Cys Thr Tyr
   565 570 575
   Leu Ala Ser His Leu Arg Gln Leu Gly Cys Ser Pro Asp Leu Arg Gln
   580 585 590
   Arg Leu Arg Tyr Ala Leu Arg Leu Leu Arg Asp Arg Ser Pro Ala
   595 600 605
<210> 17
   <211> 3492
   <212> DNA
   <213> Homo sapiens
<220> PTP-SL
   <223> Description of Sequence: N/A
<400> 17
   cagctaagac ccggagaggt ggaatttcac tttgaaattc ccttgcctcg tgagggccgg 60
   cgctgggcat gctcagtagc cgcggcgctg ctgctgggct gctgggctgg cgcggagtcc 120
   accctgccgt ctccgccttg gcttctgggc gtccagaagg ccaggcattt gccgcctctg 180
   agcgcttctg ttccccttac ccgcaacctc ctactgctct tcctctctcc ctctcttagg 240
   gaggttgaag ctggtgctgg tttctgtcgg cgccacagac tgactgctct gcaaacccca 300
   gccgaggacc tgaatcccgg agactagaag acccttggcg gtggctcttt ctaatagcac 360
   tttacctgaa gtggggtcgt ggtggagttt ctcctccacc tctcaatgca aacactatgc 420
   ggagagcagt ctgcttccct gcgctgtgcc tgctccttaa tcttcacgct gcagggtgct 480
   tttcaggaaa caatgatcat tttttggcaa ttaatcagaa gaagagtggg aagccggtat 540
   tcatttataa gcattcacaa gacattgaga agagcctgga tatagcccca caaaaaatct 600
   acagacatag ctaccattcc tcttccgaag ctcaagtaag caaacgccac cagattgtca 660
   attcagcatt tcctagaccc gcatatgacc cgtctctcaa tctgctggcc atggatggtc 720
   aagatcttga agtggaaaat ctcccaatcc cagcagcaaa tgtaattgtg gtgacactgc 780
   aaatggatgt aaacaagctg aacataacct tgcttcggat cttccgccaa ggagtggctg 840
   cagctttagg actcttaccc cagcaagtgc acatcaatcg cctcattgga aagaagaaca 900
   gtattgaact gtttgtgtct cccataaacc gaaaaacagg aatttctgat gctctgccct 960
   ctgaggaagt tcttcgttca cttaatatca atgttttgca tcaaagttta tcccagtttg 1020
   gaattacaga agtctctcct gagaaaaatg ttttacaagg gcagcatgaa gcggacaaaa 1080
   tctggagcaa agaaggattt tatgctgttg tcatttttct cagcatcttt gttattatag 1140
   taacgtgttt gatgattctt tacagattaa aagaaagatt tcagctttcc ttaagacaag 1200
   acaaagagaa aaaccaggag atccacctat cgcccatcac attacagcca gcactgtccg 1260
   aggcaaagac agtccacagc atggtccaac ctgagcaggc cccaaaggta ctgaatgttg 1320
   tcgtggaccc tcaaggccga ggtgctcctg agatcagagc taccaccgct acctctgttt 1380
   gcccttctcc tttcaaaatg aagcccatag gacttcaaga gagaagaggg tccaacgtat 1440
   ctcttacatt ggacatgagt agcttgggga acattgaacc ctttgtgtct ataccaacac 1500
   cacgggagaa ggtagcaatg gagtatctgc agtcagccag ccgaattctc acaaggtctc 1560
   agctgaggga cgtcgtggca agttcacatt tactccaaag tgaattcatg gaaataccga 1620
   tgaactttgt ggatcccaaa gaaattgata ttccgcgtca tggaactaaa aatcgctata 1680
   agaccatttt accaaatccc ctcagcagag tgtgtttaag accaaaaaat gtaaccgatt 1740
   cattgagcac ctacattaat gctaattata ttaggggcta cagtggcaag gagaaagcct 1800
   tcattgccac gcagggcccc atgatcaaca ccgtggatga tttctggcag atggtttggc 1860
   aggaagacag ccctgtgatt gttatgatca caaaactcaa agaaaaaaat gagaaatgtg 1920
   tgctatactg gccggaaaag agagggatat atggaaaagt tgaggttctg gttatcagtg 1980
   taaatgaatg tgataactac accattcgaa accttgtctt aaagcaagga agccacaccc 2040
   aacatgtgaa gcattactgg tacacctcat ggcctgatca caagactcca gacagtgccc 2100
   agcccctcct acagctcatg ctggatgtag aagaagacag acttgcttcc cagggccgag 2160
   ggcctgtggt tgtccactgc agtgcaggaa taggtagaac agggtgtttt attgctacat 2220
   ccattggctg tcaacagctg aaagaagaag gagttgtgga tgcactaagc attgtctgcc 2280
   agcttcgtat ggatagaggt ggaatggtcc aaaccagtga gcagtatgaa tttgtgcacc 2340
   atgctctgtg cctgtatgag agcagacttt cagcagagac tgtccagtga gtcattgaag 2400
   acttgtcaga ccatcaatct cttggggtga ttaatcaaat tacccaccca aggcttctag 2460
   aaggagcttc ctgcaatgga aggaaggaga agctctgaag cccatgtatg gcatggattg 2520
   tggaagactg ggcaacatat ttaagatttc cagctccttg tgtatatgaa tgcatttgta 2580
   agcatccccc aaattattct gaaggttttt tgatgatgga ggtatgatag gtttatcaca 2640
   cagcctaagg cagattttgt tttgtctgta ctgactctat ctgccacaca gaatgtatgt 2700
   atgtaatatt cagtaataaa tgtcatcagg tgatgactgg atgagctgct gaagacattc 2760
   gtattatgtg ttagatgctt taatgtttgc aaaatctgcc ttgtgaatgg actgtcagct 2820
   gttaaactgt tcctgttttg aagtgctatt acctttctca gttaccagaa tcttgctgct 2880
   aaagttgcaa gtgattgata atggattttt aacagagaag tctttgtttt tgaaaaacaa 2940
   aaatcaaaaa cagtaactat tttatatgga aatgtgtctt gataatatta cctattaaat 3000
   gtgtatttat agtccctcct atcaaacaat tacagagcac aatgattgtc attgggtata 3060
   tatgtattta ctctctatta ttgggcataa aggtggcttc tgctccagaa ctctatccac 3120
   tgtatttcca catcgtgagt cattttactt taaaagggaa aaacaaattt gtagcaactc 3180
   tgaagtatca agagttttaa ctacttgact ctcttttgct aagaagggat ttttgaatat 3240
   gctatctacc tggaatctct ctctcaacaa aaggtatatg ccttcaggaa tgatataatc 3300
   tgtcccattt tcgaggctcc ttataaggac atttccatgt atgtccttac atttctgaaa 3360
   gctttcaatc ttcaagagcc aaaaaaaatt aaaataacta ccctcagcaa acactagctg 3420
   ttctgctcat atatgaattt ttaatgcagc aatgttgact ttgtttcata ctgccaataa 3480
   actcttaata ct 3492
<210> 18
   <211> 657
   <212> PRT
   <213> Homo sapiens
<220>
   <223> Description of Sequence: N/A
<400> 18
   Met Arg Arg Ala Val Cys Phe Pro Ala Leu Cys Leu Leu Leu Asn Leu
   1               5                   10                  15
   His Ala Ala Gly Cys Phe Ser Gly Asn Asn Asp His Phe Leu Ala Ile
               20                  25                  30
   Asn Gln Lys Lys Ser Gly Lys Pro Val Phe Ile Tyr Lys His Ser Gln
           35                  40                  45
   Asp Ile Glu Lys Ser Leu Asp Ile Ala Pro Gln Lys Ile Tyr Arg His
       50                  55                  60
   Ser Tyr His Ser Ser Ser Glu Ala Gln Val Ser Lys Arg His Gln Ile
   65                  70                  75                  80
   Val Asn Ser Ala Phe Pro Arg Pro Ala Tyr Asp Pro Ser Leu Asn Leu
                   85                  90                  95
   Leu Ala Met Asp Gly Gln Asp Leu Glu Val Glu Asn Leu Pro Ile Pro
               100                 105                 110
   Ala Ala Asn Val Ile Val Val Thr Leu Gln Met Asp Val Asn Lys Leu
           115                 120                 125
   Asn Ile Thr Leu Leu Arg Ile Phe Arg Gln Gly Val Ala Ala Ala Leu
       130                 135                 140
   Gly Leu Leu Pro Gln Gln Val His Ile Asn Arg Leu Ile Gly Lys Lys
   145                 150                 155                 160
   Asn Ser Ile Glu Leu Phe Val Ser Pro Ile Asn Arg Lys Thr Gly Ile
                   165                 170                 175
   Ser Asp Ala Leu Pro Ser Glu Glu Val Leu Arg Ser Leu Asn Ile Asn
               180                 185                 190
   Val Leu His Gln Ser Leu Ser Gln Phe Gly Ile Thr Glu Val Ser Pro
           195                 200                 205
   Glu Lys Asn Val Leu Gln Gly Gln His Glu Ala Asp Lys Ile Trp Ser
       210                 215                 220
   Lys Glu Gly Phe Tyr Ala Val Val Ile Phe Leu Ser Ile Phe Val Ile
   225                 230                 235                 240
   Ile Val Thr Cys Leu Met Ile Leu Tyr Arg Leu Lys Glu Arg Phe Gln
                   245                 250                 255
   Leu Ser Leu Arg Gln Asp Lys Glu Lys Asn Gln Glu Ile His Leu Ser
   260 265 270
   Pro Ile Thr Leu Gln Pro Ala Leu Ser Glu Ala Lys Thr Val His Ser
   275 280 285
   Met Val Gln Pro Glu Gln Ala Pro Lys Val Leu Asn Val Val Val Asp
   290 295 300
   Pro Gln Gly Arg Gly Ala Pro Glu Ile Arg Ala Thr Thr Ala Thr Ser
   305 310 315 320
   Val Cys Pro Ser Pro Phe Lys Met Lys Pro Ile Gly Leu Gln Glu Arg
   325 330 335
   Arg Gly Ser Asn Val Ser Leu Thr Leu Asp Met Ser Ser Leu Gly Asn
   340 345 350
   Ile Glu Pro Phe Val Ser Ile Pro Thr Pro Arg Glu Lys Val Ala Met
   355 360 365
   Glu Tyr Leu Gln Ser Ala Ser Arg Ile Leu Thr Arg Ser Gln Leu Arg
   370 375 380
   Asp Val Val Ala Ser Ser His Leu Leu Gln Ser Glu Phe Met Glu Ile
   385 390 395 400
   Pro Met Asn Phe Val Asp Pro Lys Glu Ile Asp Ile Pro Arg His Gly
   405 410 415
   Thr Lys Asn Arg Tyr Lys Thr Ile Leu Pro Asn Pro Leu Ser Arg Val
   420 425 430
   Cys Leu Arg Pro Lys Asn Val Thr Asp Ser Leu Ser Thr Tyr Ile Asn
   435 440 445
   Ala Asn Tyr Ile Arg Gly Tyr Ser Gly Lys Glu Lys Ala Phe Ile Ala
   450 455 460
   Thr Gln Gly Pro Met Ile Asn Thr Val Asp Asp Phe Trp Gln Met Val
   465 470 475 480
   Trp Gln Glu Asp Ser Pro Val Ile Val Met Ile Thr Lys Leu Lys Glu
   485 490 495
   Lys Asn Glu Lys Cys Val Leu Tyr Trp Pro Glu Lys Arg Gly Ile Tyr
   500 505 510
   Gly Lys Val Glu Val Leu Val Ile Ser Val Asn Glu Cys Asp Asn Tyr
   515 520 525
   Thr Ile Arg Asn Leu Val Leu Lys Gln Gly Ser His Thr Gln His Val
   530 535 540
   Lys His Tyr Trp Tyr Thr Ser Trp Pro Asp His Lys Thr Pro Asp Ser
   545 550 555 560
   Ala Gln Pro Leu Leu Gln Leu Met Leu Asp Val Glu Glu Asp Arg Leu
   565 570 575
   Ala Ser Gln Gly Arg Gly Pro Val Val Val His Cys Ser Ala Gly Ile
   580 585 590
   Gly Arg Thr Gly Cys Phe Ile Ala Thr Ser Ile Gly Cys Gln Gln Leu
   595 600 605
   Lys Glu Glu Gly Val Val Asp Ala Leu Ser Ile Val Cys Gln Leu Arg
   610 615 620
   Met Asp Arg Gly Gly Met Val Gln Thr Ser Glu Gln Tyr Glu Phe Val
   625 630 635 640
   His His Ala Leu Cys Leu Tyr Glu Ser Arg Leu Ser Ala Glu Thr Val
   645 650 655
   Gln
<210> 19
   <211> 985
   <212> DNA
   <213> Homo sapiens
<220> HSP86
   <223> Description of Sequence: N/A
<400> 19
   ccggcccggt gtggctgtgc cgttggtcct gtgcggtcac ttagccaaga tgcctgagga 60
   aacccagacc caagaccaac cgatggagga ggaggaggtt gagacgttcg cctttcaggc 120
   agaaattgcc cagttgatgt cattgatcat caatactttc tactcgaaca aagagatctt 180
   tctgagagag ctcatttcaa attcatcaga tgcattggac aaaatccggt atgaaagctt 240
   gacagatccc agtaaattag actctgggaa agagctgcat attaacctta taccgaacaa 300
   acaagatcga actctcacta ttgtggatac tggaattgga atgaccaagg ctgacttgat 360
   caataacctt ggtactatcg ccaagtctgg gaccaaagcg ttcatggaag ctttgcaggc 420
   tggtgcagat atctctatga ttggccagtt cggtgttggt ttttattctg cttatttggt 480
   tgctgagaaa gtaactgtga tcaccaaaca taacgatgat gagcagtacg cttgggagtc 540
   ctcagcaggg ggatcattca cagtgaggac agacacaggt gaacctatgg gtcgtggaac 600
   aaaagttatc ctacacctga aagaagacca aactgagtac ttggaggaac gaagaataaa 660
   ggagattgtg aagaaacatt ctcagtttat tggatatccc attactcttt ttgtggagaa 720
   ggaacgtgat aaagaagtaa gcgatgatga ggctgaagaa aaggaagaca aagaagaaga 780
   aaaagaaaaa gaagagaaag agtcggaaga caaacctgaa attgaagatg ttggttctga 840
   tgaggaagaa gaaaagaagg atggtgacaa gaagaagaag aagaagatta aggaaaagta 900
   catcgatcaa gaagagctca acaaaacaaa gcccatctgg accagaaatc ccgacgatat 960
   tactaatgag gagtacggag aattc 985
<210> 20
   <211> 312
   <212> PRT
   <213> Homo sapiens
<220>
   <223> Description of Sequence: N/A
<400> 20
   Met Pro Glu Glu Thr Gln Thr Gln Asp Gln Pro Met Glu Glu Glu Glu
   1 5 10 15
   Val Glu Thr Phe Ala Phe Gln Ala Glu Ile Ala Gln Leu Met Ser Leu
   20 25 30
   Ile Ile Asn Thr Phe Tyr Ser Asn Lys Glu Ile Phe Leu Arg Glu Leu
   35 40 45
   Ile Ser Asn Ser Ser Asp Ala Leu Asp Lys Ile Arg Tyr Glu Ser Leu
   50 55 60
   Thr Asp Pro Ser Lys Leu Asp Ser Gly Lys Glu Leu His Ile Asn Leu
   65 70 75 80
   Ile Pro Asn Lys Gln Asp Arg Thr Leu Thr Ile Val Asp Thr Gly Ile
   85 90 95
   Gly Met Thr Lys Ala Asp Leu Ile Asn Asn Leu Gly Thr Ile Ala Lys
   100 105 110
   Ser Gly Thr Lys Ala Phe Met Glu Ala Leu Gln Ala Gly Ala Asp Ile
   115 120 125
   Ser Met Ile Gly Gln Phe Gly Val Gly Phe Tyr Ser Ala Tyr Leu Val
   130 135 140
   Ala Glu Lys Val Thr Val Ile Thr Lys His Asn Asp Asp Glu Gln Tyr
   145 150 155 160
   Ala Trp Glu Ser Ser Ala Gly Gly Ser Phe Thr Val Arg Thr Asp Thr
   165 170 175
   Gly Glu Pro Met Gly Arg Gly Thr Lys Val Ile Leu His Leu Lys Glu
   180 185 190
   Asp Gln Thr Glu Tyr Leu Glu Glu Arg Arg Ile Lys Glu Ile Val Lys
   195 200 205
   Lys His Ser Gln Phe Ile Gly Tyr Pro Ile Thr Leu Phe Val Glu Lys
   210 215 220
   Glu Arg Asp Lys Glu Val Ser Asp Asp Glu Ala Glu Glu Lys Glu Asp
   225 230 235 240
   Lys Glu Glu Glu Lys Glu Lys Glu Glu Lys Glu Ser Glu Asp Lys Pro
   245 250 255
   Glu Ile Glu Asp Val Gly Ser Asp Glu Glu Glu Glu Lys Lys Asp Gly
   260 265 270
   Asp Lys Lys Lys Lys Lys Lys Ile Lys Glu Lys Tyr Ile Asp Gln Glu
   275 280 285
   Glu Leu Asn Lys Thr Lys Pro Ile Trp Thr Arg Asn Pro Asp Asp Ile
   290 295 300
   Thr Asn Glu Glu Tyr Gly Glu Phe
   305 310

## Description

The present invention relates to the use of the pyridylpyrimidine derivatives as pharmaceutically active agents for the prophylaxis and/or treatment of prion infections and prion diseases.

### Background of the invention

Pynridylpyrimidine derivatives are known from WO 9509851 as effective compounds for chemotherapy of tumors, from WO 9509853. EP-A-0 588 762, WO 9509847, WO 9903854, and EP-B-0 564 409 as effective compounds for treatment of tumors. Furthermore, EP-B-0 564 409 disdoses the use of said compounds in the treatment of artherosclerosis and Exp. Opin. Ther. Patents, 1998, 8(12), 1599-1625 describes the use of pyridylpyrimidine derivatives, especially of Gleevec^{™}, the Novartis compound CGP 57148, as tyrosine kinase inhibitors in can treatment.

WO 00/64 894 discloses the use of a MAP kinase inhibitor for the treatment of Alzheimer's disease and Creutzfeld-Jakob disease.

Prions are infectious agents which do not have a nucleic acid genome. It seems that a protein alone is the infectious agent. A prion has been defined as "small proteinaceous infectious particle which resists inactivation by procedures that modify nudeic acids". The discovery that proteins alone can transmit an infectious disease has come as a considerable surprise to the scientific community. Prion diseases are often called "transmissible spongiform encephalopathies", because of the post mortem appearance of the brain with large vacuoies in the cortex and cerebellum. Probably most mammalian species develop these diseases. Prion diseases are a group of neurodegenerative disorders of humans and animals and the prion diseases can manifest as sporadic, genetic or infectious disorders. Examples for prion diseases acquired by exogenous infection are the Bovine spongiform encephalitis (BSE) of cattle and the new variant of Creutzfeld-Jakob disease (vCJD) caused by BSE. Further examples include kuru, Gerstmann-Sträussler-Scheinker disease of humans as well as scrapie of animals. For many years, the prion diseases were thought to be caused by viruses despite intriguing evidence to the contrary. The unique characteristic common to all of these disorders, whether sporadic, dominantly inherited, or acquired by infection, is that they involve the aberrant metabolism of the prion protein (PrP). In many cases, the cellular prion protein (PrP^{c}) ["c" refers to cellular] is converted into the scrapie isoform (PrP^{Sc}) ["Sc" refers to Scrapie] by a posttranslational process that involves a conformational change. Often, the human prion diseases are transmissible to experimental animals and all of the inherited prion diseases segregate with PrP gene mutations.

These prion diseases in animals and humans have a long incubation period and a long clinical course, and are always fatal leading via decerebration to death within an average period of 7 months (CJD). Neuropathological features consist of neuronal vacuolization, neuronal death and gliosis with hyperastrocytosis. The precise diagnosis of transmissible neurodegenerative diseases can be established only by the examination of the central nervous system after biopsy or autopsy.

Clinical symptoms of the disease are progressive dementia, myoclonus and prominent ataxia with the additional clinical features of dysautonomia and delirious psychomotor excitement and with relatively preserved verbal responses.

Between 1980 and, roughly, 1996, about 750,000 cattle infected with BSE were slaughtered for human consumption in Great Britain (Anderson, R. M. *et al. Nature* 382, 779-788,1996; Ferguson, N. M., Donnelly, C. A., Woolhouse, M. E. J. & Anderson, R. M. *Phil. Trans. R. Soc. Lond. B* 352, 803-838, 1997). The annual incidence of vCJD (3, 10, 10, 18, 14 and 33 deaths in 1995-2000, respectively) can be interpreted as a first sign of a steady or exponential increase over the next years. The suggestion by the European Union Scientific Steering Committee that up to 500,000 people could have been exposed to BSE from a single infected bovine has fuelled speculation that millions of consumers are at risk.

Recent findings demonstrate that the pathogenic PrP^{Sc} of vCJD can be found in the lymph system (e.g. tonsils, lymph nodes) in humans suggesting a high risk of horizontal spread via lymph and/or blood transmission, dramatically increasing the number of people at risk.

The medical need in prion diseases today can be clearly defined as the establishment of a diagnostic system, that can detect the disease as early as possible in living humans and/or animals, to estimate the medical need for the treatment in the future and to identify the infected animals to remove them from the food chain. The medical need for prion diseases in the future (approximately starting in 5-10 years) will be medical treatment that inhibits the disease symptoms, the manifestation and/or progression of the disease.

It is object of the present invention to provide methods wherein novel and also know compounds which can be used as pharmaceutically active agents are used in order to treat prion infections and prion diseases.

The object of the present invention is solved by the teaching of the independent claims. Further advantageous features, aspects and details of the invention are evident from the dependent claims, the description, the examples, and the figures of the present application.

### Description of the invention

One aspect of the present invention is related to the use of compounds of the general formula (I): 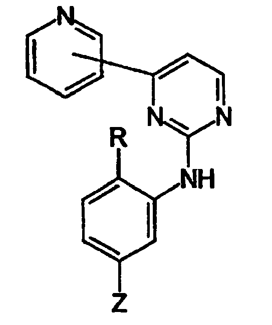 wherein:
R represents hydrogen or methyl;
Y, Y', Y" are independently of each other -H, -F, -Cl, -Br, -I, -CH₂F, -CH₂Cl, -CH₂Br, -CH₂I, -OH, -OCH₃, -CH₃, -CN, -OCF₃, 4-methylpiperazin-1-yl-methyl, -C(CH₃)=N-NH-C(NH)-NH₂;
Z represents -NO₂, -NH₂, -NH-CO-X, -NH-CS-X, -NH-CO-NH-X, -NH-SO₂-X;
X represents thiophenyl, cyclohexyl, isoquinolinyl, naphthyl, quinolinyl, cyclopentyl, pyridinyl, naphthyridinyl, or 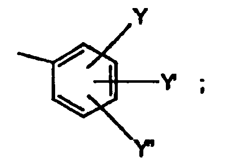
and pharmaceutically acceptable salts thereof for the manufacture of a pharmaceutically composition for the prophylaxis and/or treatment of prion infections and/or diseases induced by prion infection.

Thus, one embodiment of the present invention disclosed herein is the use of the compounds defined above in connection with preventing and/or treating infections and/or diseases associated with said infections in an individual. This comprises administering to the individual an amount of at least one compound according to general formula (I) and/or pharmaceutically acceptable salts thereof effective to prevent and/or treat said infections and/or diseases. Most preferred is the administration of a compound 53.

As revealed for the first time herein, the present invention discloses the use of compounds of the general formula (I) for the prophylaxis and/or treatment of prion infections and prion diseases. As described above, said pyridylpyrimidine derivatives have first of all been used in tumor therapy. The Novartis compound Gleevec^{™} also known as Glivec^{™}, CGP-57148B, imatinib mesylate, STI-571, STI-571A, CAS 152459-95-5, or 4-((Methyl-1-pipetazinyl)methyl)-N-[4-methyl-3-[[4-(3-pyridinyl)-2-pyrimidinyl]amino]-phenyl]benzamide methanesulfonate, has been registered in many countries as anticancer drug. This Gleevec^{™} compound (compound 53) is also the most active one in the indication prion diseases.

The name "prion" is used to describe the causative agents which underlie the transmissible spongiform encephalopathies. A prion is proposed to be a novel infectious particle that differs from viruses and viroids. It is composed solely of one unique protein that resists most inactivation procedures such as heat, radiation, and proteases. The latter characteristic has led to the term protease-resistant isoform of the prion protein. The protease-resistant isoform has been proposed to slowly catalyze the conversion of the normal prion protein into the abnormal form.

The term "isoform" in the context of prions means two proteins with exactly the same amino acid sequence that are folded into molecules with dramatically different tertiary structures. The normal cellular isoform of the prion protein (PrP^{C}) has a high α-helix content, a low β-sheet content, and is sensitive to protease digestion. The abnormal, disease-causing isoform (PrP^{Sc}) has a lower α-helix content, a much higher β-sheet content, and is much more resistant to protease digestion.

Preferred are the compounds wherein R represents hydrogen. Also preferred are compounds wherein Z represents -NH-CO-X or -NH-SO₂-X and/or wherein Y, Y', Y" are independently of each other -H, -F, -Cl, -CH₂F, -CH₂Cl, -OH, -OCH₃, -CN, -OCF₃, or a 4-methylpiperazin-1-yl-methyl residue.

Also preferred are the following pyridytpyrimidine derivatives selected from the group comprising:
- Compound 1:: (3-Nitrophenyl-(4-pyridin-3yl-pyrimidin-2-yl)-amine;
- Compound 2:: (3-Aminophenyl)-(4-pyridin-3-yl-pyrimidin-2-yl)-amine;
- Compound 3:: (5-Amino-2-methylphenyl)-(4-pyridin-3-yl-pyrimidin-2-yl)-amine;
- Compound 4:: 4-Chloromethyl-*N*-[4-methyl-3-(4-pyridin-3-yl-pyrimidin-2-ylamino)-phenyl]-benzamide;
- Compound 5:: 4-Chloromethyl-*N*-[3-(4-pyridin-3-yl-pyrimidin-2-ylamino)-phenyl]-benzamide;
- Compound 6:: 4-(4-Methylpiperazin-1-ylmethyl)-*N*-[3-(4-pyridin-3-yl-pyrimidin-2-ylamino)-phenyl]-benzamide;
- Compound 7:: Thiophene-3-carboxylic acid [4-methyl-3-(4-pyridin-4-yl-pyrimidin-2-ylamino)-phenyl]-amide;
- Compound 8:: 4-Chloro-*N*-[4-methyl-3-(4-pyridin-3-yl-pyrimidin-2-ylamino)-phenyl]-benzamide;
- Compound 9:: 4-Chloro-*N*-[3-(4-pyridin-3-yl-pyrimidin-2-ylamino)-phenyl]-benzamide;
- Compound 10:: 3,4,5-Trimethoxy-*N*-[3-(4-pyridin-3-yl-pyrimidin-2-ylamino)-phenyl]-benzamide;
- Compound 11:: 4-Cyano-*N*-[3-(4-pyridin-3-yl-pyrimidin-2-ylamino)-phenyl]-benzamide;
- Compound 12:: 4-Methoxy-*N*-[3-(4-pyridin-3-yl-pyrimidin-2-ylamino)-phenyl]-benzamide;
- Compound 13:: 4-Chloro-*N*-[3-(4-pyridin-3-yl-pyrimidin-2-ylamino)-phenyl]-benzenesulfonamide;
- Compound 14:: Thiophene-3-carboxylic acid [3-(4-pyridin-3-yl-pyrimidin-2-ylamino)-phenyl]-amide;
- Compound 15:: 3,5-Dimethoxy-*N*-[3-(4-pyridin-3-yl-pyrimidin-2-ylamino)-phenyl]-benzamide;
- Compound 16:: 3,4,5-Trimethoxy-*N*-[4-methyl-3-(4-pyridin-3-yl-pyrimidin-2-ylamino)-phenyl]-benzamide;
- Compound 17:: 4-Cyano-*N*-[4-methyl-3-(4-pyridin-3-yl-pyrimidin-2-ylamino)-phenyl]-benzamide;
- Compound 18:: 4-Methoxy-*N*-[4-methyl-3-(4-pyridin-3-yl-pyrimidin-2-ylamino)-phenyl]-benzamide;
- Compound 19:: 4-Chloro-*N*-[4-methyl-3-(4-pyridin-3-yl-pyrimidin-2-ylamino)-phenyl]-benzenesulfonamide;
- Compound 20:: Thiophene-3-carboxylic acid [4-methyl-3-(4-pyridin-3-yl-pyrimidin-2-ylamino)-phenyl]-amide;
- Compound 21:: 3,5-Dimethoxy-*N*-[4-methyl-3-(4-pyridin-3-yl-pyrimidin-2-ylamino)-phenyl]-benzamide;
- Compound 22:: 4-Trifluoromethoxy-*N*-[4-methyl-3-(4-pyridin-3-yl-pyrimidin-2-ylamino)-phenyl]-benzamide;
- Compound 23:: Cyclohexanecarboxylic acid [4-methyl-3-(4-pyridin-3-yl-pyrimidin-2-ylamino)-phenyl]-amide;
- Compound 24:: Cyclohexanecarboxylic acid [3-(4-pyridin-3-yl-pyrimidin-2-ylamino)-phenyl]-amide;
- Compound 25:: Isoquinoline-5-sulfonic acid [4-methyl-3-(4-pyridin-3-yl-pyrimidin-2-ylamino)-phenyl]-amide;
- Compound 26:: Isoquinoline-5-sulfonic acid [3-(4-pyridin-3-yl-pyrimidin-2-ylamino)-phenyl]-amide;
- Compound 27:: (5-Nitro-2-methylphenyl)-(4-pyridin-2-yl-pyrimidin-2-yl)-amine;
- Compound 28:: (5-Amino-2-methylphenyl)-(4-pyridin-2-yl-pyrimidin-2-yl)-amine;
- Compound 29:: 3,4,5-Trimethoxy-*N*-[4-methyl-3-(4-pyridin-2-yl-pyrimidin-2-ylamino)-phenyl]-benzamide;
- Compound 30:: 4-Cyano-*N*-[4-methyl-3-(4-pyridin-2-yl-pyrimidin-2-ylamino)-phenyl]-benzamide;
- Compound 31:: (3-Aminophenyl)-(4-pyridin-2-yl-pyrimidin-2-yl)-amine;
- Compound 32:: 4-Chloro-*N*-[4-methyl-3-(4-pyridin-2-yl-pyrimidin-2-ylamino)-phenyl]-benzamide;
- Compound 33:: Cyclohexanecarboxylic acid [4-methyl-3-(4-pyridin-2-yl-pyrimidin-2-ylamino)-phenyl]-amide;
- Compound 34:: 4-Cyano-*N*-[4-methyl-3-(4-pyridin-4-yl-pyrimidin-2-ylamino)-phenyl]-benzamide;
- Compound 35:: 4-Chloro-*N*-[4-methyl-3-(4-pyridin-4-yl-pyrimidin-2-ylamino)-phenyl]-benzenesulfonamide;
- Compound 36:: 4-Methoxy-*N*-[4-methyl-3-(4-pyridin-4-yl-pyrimidin-2-ylamino)-phenyl]-benzamide;
- Compound 37:: 4-Chloro-*N*-[4-methyl-3-(4-pyridin-4-yl-pyrimidin-2-ylamino)-phenyl]-benzamide;
- Compound 38:: Cyclohexanecarboxylic acid [3-(4-pyridin-4-yl-pyrimidin-2-ylamino)-phenyl]-amide;
- Compound 39:: 3,5-Dimethoxy-*N*-[3-(4-pyridin-4-yl-pyrimidin-2-ylamino)-phenyl]-benzamide;
- Compound 40:: (5-Amino-2-methylphenyl)-(4-pyridin-4-yl-pyrimidin-2-yl)-amine;
- Compound 41:: Thiophene-3-cartioxylic acid [3-(4-pyridin-4-yl-pyrimidin-2-ylamino)-phenyl]-amide;
- Compound 42:: 4-Chloro-*N*-[3-(4-pyridin-4-yl-pyrimidin-2-ylamino)-phenyl]-benzenesulfonamide;
- Compound 43:: 4-Chloro-*N*-[3-(4-pyridin-4-yl-pyrimidin-2-ylamino)-phenyl]-benzamide;
- Compound 44:: (3-Aminophenyl)-(4-pyridin-4-yl-pyrimidin-2-yl)-amine;
- Compound 45:: (3-Nitrophenyl)-(4-pyridin-4-yl-pyrimidin-2-yl)-amine;
- Compound 46:: 4-Trifluoromethoxy-*N*-[4-methyl-3-(4-pyridin-4-yl-pyrimidin-2-ylamino)-phenyl]-benzamide;
- Compound 47:: Isoquinoline-5-sulfonic acid [3-(4-pyridin-4-yl-pyrimidin-2-ylamino)-phenyl]-amide;
- Compound 48:: 4-Methoxy-*N*-[3-(4-pyridin-4-yl-pyrimidin-2-ylamino)-phenyl]-benzamide;
- Compound 49:: 4-Cyano-*N*-[3-(4-pyridin-4-yl-pyrimidin-2-ylamino)-phenyl]-benzamide;
- Compound 50:: 3,4,5-Trimethoxy-*N*-[3-(4-pyridin-4-yl-pyrimidin-2-ylamino)-phenyl]-benzamide;
- Compound 51:: 3,5-Dimethoxy-*N*-[4-methyl-3-(4-pyridin-4-yl-pyrimidin-2-ylamino)-phenyl]-benzamide;
- Compound 52:: 3,4,5-Trimethoxy-*N*-[4-methyl-3-(4-pyridin-4-yl-pyrimidin-2-ylamino)-phenyl]-benzamide;
- Compound 53:: 4-(4-Methylpiperazin-1-ylmethyl)-*N*-[4-methyl-3-(4-pyridin-3-yl-pyrimidin-2-ylamino)-phenyl]-benzamide (Gleevec^{™});
- Compound 54:: 4-Methyl-*N*-[4-methyl-3-(4-pyridin-2-yl-pyrimidin-2-ylamino)-phenyl]-benzenesulfonamide
- Compound 55:: 4-Methoxy-*N*-[4-methyl-3-(4-pyridin-2-yl-pyrimidin-2-ylamino)-phenyl]-benzamide;
- Compound 56:: 3,5-Dimethoxy-*N*-[4-methyl-3-(4-pyridin-2-yl-pyrimidin-2-ylamino)-phenyl]-benzamide;
- Compound 57:: Naphthalene-2-carboxylic acid [4-methyl-3-(4-pyridin-3-yl-pyrimidin-2-ylamino)-phenyl]-amide;
- Compound 58:: *N*-[3-(4-Pyridin-4-yl-pyrimidin-2-ylamino)-phenyl]-benzamide;
- Compound 59:: 4-Chloro-*N*-[3-(4-pyridin-2-yl-pyrimidin-2-ylamino)-phenyl]-benzamide;
- Compound 60:: 4-Methoxy-*N*-[3-(4-pyridin-2-yl-pyrimidin-2-ylamino)-phenyl]-benzamide;
- Compound 61:: 4-Chloro-*N*-[3-(4-pyridin-2-yl-pyrimidin-2-ylamino)-phenyl]-benzenesulfonamide;
- Compound 62:: Thiophene-2-carboxylic acid 3-(4-pyridin-2-yl-pyrimidin-2-yl-amino)-phenyl]-amide;
- Compound 63:: Naphthalene-2-sulfonic-acid [3-(4-pyridin-2-yl-pyrimidin-2-yl-amino)-phenyl]-amide;
- Compound 64:: Isoquinoline-5-sulfonic-acid [3-(4-pyridin-2-yl-pyrimidin-2-yl-amino)-phenyl]-amide;
- Compound 65:: Cylopentanecarboxylic acid 3-(4-pyridin-2-yl-pyrimidin-2-yl-amino)-phenyl]-amide;
- Compound 66:: Naphthalene-2-carboxylic acid [3-(4-pyridin-2-yl-pyrimidin-2-ylamino)-phenyl]-amide;
- Compound 67:: 4-Cyano-*N*-[3-(4-pyridin-2-yl-pyrimidin-2-ylamino)-phenyl]-benzamide;
- Compound 68:: 3,5-Dimethoxy-*N*-[3-(4-pyridin-2-yl-pyrimidin-2-ylamino)-phenyl]-benzamide;
- Compound 69:: 4-Bromo-*N*-[3-(4-pyridin-2-yl-pyrimidin-2-ylamino)-phenyl]-benzamide;
- Compound 70:: 4-Methyl-*N*-[3-(4-pyridin-2-yl-pyrimidin-2-ylamino)-phenyl]-benzamide;
- Compound 71:: 4-Fluoro-*N*-3-(4-pyridin-2-yl-pyrimidin-2-ylamino)-phenyl]-benzenesulfonamide;
- Compound 72:: 3,5-Dichloro-*N*-[3-(4-pyridin-2-yl-pyrimidin-2-ylamino)-phenyl]-benzamide;
- Compound 73:: *N*-[3-(4-Pyridin-2-yl-pyrimidin-2-ylamino)-phenyl]-benzamide;
- Compound 74:: 4-Chloromethyl-*N*-[3-(4-pyridin-2-yl-pyrimidin-2-ylamino)-phenyl]-benzamide;
- Compound 75:: 4-Methyl-*N*-3-(4-pyridin-2-yl-pyrimidin-2-ylamino)-phenyl]-benzenesulfonamide
- Compound 76:: 4-(4-Methylpiperazin-1-ylmethyl)-*N*-[3-(4-pyridin-2-yl-pyrimidin-2-ylamino)-phenyl]-benzamide;
- Compound 77:: Naphthalene-2-carboxylic acid [3-(4-pyridin-4-yl-pyrimidin-2-ylamino)-phenyl]-amide;
- Compound 78:: 2-Methoxy-*N*-[4-methyl-3-(4-pyridin-3-yl-pyrimidin-2-ylamino)-phenyl]-benzamide;
- Compound 79:: 2-Methoxy-*N*-[3-(4-pyridin-3-yl-pyrimidin-2-ylamino)-phenyl]-benzamide;
- Compound 80:: 4-Methyl-*N*-[3-(4-pyridin-3-yl-pyrimidin-2-ylamino)-phenyl]-benzamide;
- Compound 81:: 4-Methyl-*N*-[4-methyl-3-(4-pyridin-3-yl-pyrimidin-2-ylamino)-phenyl]-benzamide;
- Compound 82:: *N*-[4-Methyl-3-(4-pyridin-3-yl-pyrimidin-2-ylamino)-phenyl]-benzamide;
- Compound 83:: 1-(3,5-Diacetyl-phenyl)-3-[4-methyl-3-(4-pyridin-3-yl-pyrimidin-2-ylamino)-phenyl]-urea;
- Compound 84:: 1-{3,5-Bis-(amidinohydrazone)-phenyl}-3-[4-methyl-3-(4-pyridin-3-yl-pyrimidin-2-ylamino)-phenyl]-urea;
- Compound 85:: *N*-[4-Methyl-3-(4-pyridin-3-yl-pyrimidin-2-ylamino)-phenyl]-nicotinamide;
- Compound 86:: *N*-[3-(4-Pyridin-3-yl-pyrimidin-2-ylamino)-phenyl]-nicotinamide;
- Compound 87:: [1,8]Naphthyridine-2-carboxylic acid [3-(4-pyridin-3-yl-pyrimidin -2-ylamino)-phenyl]-amide;
- Compound 88:: [1,8]Naphthyridine-2-carbothioic acid [3-(4-pyridin-3-yl-pyrimidin-2-ylamino)-phenyl]-amide;
- Compound 89:: 2-Methoxy-*N*-[3-(4-pyridin-4-yl-pyrimidin-2-ylamino)-phenyl]-benzamide;
- Compound 90:: 4-Trifluoromethoxy-*N*-[3-(4-pyridin-4-yl-pyrimidin-2-ylamino)-phenyl]-benzamide;
- Compound 91:: 4-Methyl-*N*-[3-(4-pyridin-4-yl-pyrimidin-2-ylamino)-phenyl]-benzamide;
and pharmaceutically active salts of these compounds.

Recent research has revealed how cells communicate with each other to coordinate the growth and maintenance of the multitude of tissues within the human body. A key element of this communication network is the transmission of a signal from the exterior of a cell to its nucleus, which results in the activation or suppression of specific genes. This process is called signal transduction.

An integral part of signal transduction is the interaction of ligands, their receptors and intracellular signal transduction molecules. Ligands are messengers that bind to specific receptors on the surface of target cells. As a result of the binding, the receptors trigger the activation of a cascade of downstream signaling molecules, thereby transmitting the message from the exterior of the cell to its nucleus. When the message reaches the nucleus, it initiates the modulation of specific genes, resulting in the production of RNA and finally proteins that carry out a specific biological function. Disturbed activity of signal transduction molecules may lead to the malfunctioning of cells and disease processes. Specifically, interference of the pathogenic PrP^{Sc} from prion diseases with neuronal cells is necessary for the prion protein to induce its neuropathological features such as neuronal vacuolization, neuronal death and gliosis with hyperastrocytosis.

A key element of this communication network is the transmission of a signal from the exterior of a cell to its nucleus, which results in the activation or suppression of specific genes. The human cellular protein kinases Abl and clk1 are two of the enzymes involved in said signal transduction process. As revealed herein said kinases Abl and clk1 serve as targets and are inhibited by the pyridylpyrimidine compounds of the general formula (I). It could be proved that prion infections and/or prion diseases can be treated and also be prevented by the inhibition of said kinase Abl using the inventive pyridylpyrimidine derivatives. Inhibition of the kinase clk1 by said pyridylpyrimidine compounds can be used for the treatment of infections and diseases.

A microarray platform technology consisting of more than 1100 signal transduction cDNAs has been established. The technology is used for the identification of changes in RNA expression patterns as a result of the manipulation of the host cell by PrP^{Sc}. In addition, differential display techniques were used in order to pinpoint these changes to those enzymes which could be potential targets for drug intervention.

Employing this predefined set of signal transduction relevant cDNAs on the filters, the expression pattern of signal transduction mRNAs in neuronal mouse cells transfected with the pathogenic form of the prion protein (PrP^{Sc}) were compared with the same cells transfected with the non-pathogenic wild-type form (PrP^{c}) as a control. Interference of the PrP^{Sc} with the cellular signaling events is reflected in different gene expression when compared to the control cellular situation (PrP^{c}).

Using this technology, the human cellular protein kinases FGF-R1 (also known as fig, FI-1, Flt-2, or b-FGFR), Tkt (also known as CCK-2, DDR-2, or EDDR, EC Number 2.7.1.112), Abl (also known as c-abl), clk1, MKK7 (also known as SKK4, SAPKK4, SAPKK5, or JNKK2), LIMK-2, CaM-KI, JNK2 (also known as SAPK1a, SAPKalpha), CDC2 (also known as CDK1), PRK, the human cellular protein phosphatases PTP-SL (also known as MCP83), PTP-zeta, the cellular signal transduction molecules HSP86, and GPIR-1 were identified as potential anti-prion disease targets. Said cellular protein kinases, phosphatases and signal transduction molecules are found to be specifically up- or downregulated by PrP^{Sc} in relevant mouse neuronal cells.

Surprisingly, it was found that the following human cellular targets are significantly up- or downregulated in prion infected cells:

| target | regulation |
|---|---|
| FGF-R1 | 3.6 fold stronger |
| Abl | 5.6 fold stronger |
| MKK7 | 4.1 fold stronger |
| CDC2 | 2.0 fold weaker |
| Tkt | 2.1 fold stronger |
| LIMK-2 | 2.1 fold stronger |
| CaM-KI | 2.1 fold stronger |
| JNK2 | 2.0 fold weaker |
| PRK | 2.0 fold weaker |
| PTPzeta | 4.6 fold weaker |
| PTP-SL | 5.0 fold weaker |
| HSP86 | 4.1 fold weaker |
| GPIR-1 | 2.3 fold weaker |

Thus, one aspect of the present invention relates to a method for preventing and/or treating prion infections and/or diseases associated with said prion infections in an individual which comprises administering to the individual an amount of at least one compound of the general formula (I) and/or pharmaceutically acceptable salts thereof effective to prevent and/or treat said prion infections and/or prion diseases. Most preferred is the administration of a compound according to claim 7.

It could be proven that inhibition of one target selected from FGF-R1, Tkt, Abl, clk1, MKK7, LIMK-2, CaM-KI, JNK2, CDC2, PRK, PTP-SL, PTP-zeta, HSP86, and GPIR-1 was effective to treat prion diseases. Therefore, another aspect of the invention relates to a method for preventing and/or treating prion infections and/or prion diseases in an individual comprising the step of administering a pharmaceutically effective amount of at least one compound according of the general formula (I) and/or pharmaceutically acceptable salts thereof which inhibits at least partially the activity of one target selected from FGF-R1, Tkt, Abl, clk1, MKK7, LIMK-2, CaM-KI, JNK2, CDC2, PRK, PTP-SL, PTP-zeta, HSP86, and GPIR-1.

The nucleoside sequences of the genes coding for the human cellular protein kinase Abl and the protein kinase clk1 and their amino acid sequences are disclosed in form of a sequence listing shown below. The nucleoside and amino acid sequences for the kinase Abl (Accession Number: M14752) and for the kinase clk1 (Accession Numbers: XM002520, NM004071, L29222, L29219) were obtained from NCBI (National Library of Medicine: PubMed).

The compounds of general formula (I) were identified as inhibitors of at least one target selected from the group comprising FGF-R1. Tkt, Abl, clk1, MKK7, LIMK-2, CaM-Kl, JNK2, CDC2, PRK, PTP-SL, PTP-zeta, HSP86, GPIR-1 by the use of a method for detecting compounds useful for the prophylaxis and/or treatment of prion infections and/or diseases. Said method comprises
a) contacting a test compound with at least one human cellular protein kinase, phosphatase or cellular signal transduction molecule selected from the group comprising FGF-R1, Tkt, Abl, clk1, MKK7, LIMK-2, CaM-KI, JNK2, CDC2, PRK, PTP-SL, PTP-zeta, HSP86, GPIR-1; and
b) detecting the activity of said human cellular protein kinase, phosphatase or cellular signal transduction molecule.

The activity of a human cellular protein kinase, phosphatase or cellular signal transduction molecule was preferably measured by means of an enzymatic assay.

As used herein, the term "inhibitor" refers to any compound capable of downregulating, decreasing, suppressing or otherwise regulating the amount and/or activity of at least one human cellular protein kinase, phosphatase or cellular signal transduction molecule selected from the group comprising FGF-R1, Tkt, Abl, clkl, MKK7, LIMK-2, CaM-KI, JNK2, CDC2. PRK, PTP-SL, PTP-zeta, HSP86, and GPIR-1. Generally, said inhibitors, including suicide Inhibitors, may be proteins, oligo- and potypeptides, nucleic acids, genes, small chemical molecules, or other chemical moieties.

The present disclosure teaches for the first time the up- or downregulation of the above-mentioned human cellular protein kinases, phosphatases, or cellular signal transduction molecules specifically involved in prion infections and/or diseases. Thus, described herein is also a method for detecting prion infections and/or diseases in an individual comprising:
a) providing a sample from said individual; and
b) adding to said sample a pharmaceutically effective amount of at least one pharmaceutically active agent; and
c) detecting activity in said sample of at least one human cellular protein kinase, phosphatase or cellular signal transduction molecule selected from the group comprising FGF-R1, Tkt, Abl, clk1, MKK7, LIMK-2, CaM-KI, JNK2, CDC2, PRK, PTP-SL, PTP-zeta, HSP86, GPIR-1.

As used herein the term "sample" refers to any sample that can be taken from a living animal or human for diagnostic purposes, especially said sample comprises blood, milk, saliva, sputum, excrement, urine, spinal cord liquid, liquor, lachrymal gland liquid, biopsies and all other samples that can be taken from a living animal or human for diagnostic purposes.

The term "individual" preferably refers to mammals, especially humans or ruminants. Ruminants are, for instance, muledeer, elk, cow, cattle, sheep, goat, deer, or buffalo. Minks are an example for mammals which do not belong to the species of ruminants.

As used herein the term "ruminants" refers to an animal, for instance, cattle, sheep, goat, deer, elk, or buffalo that has four separate stomach chambers, and is therefore able to digest a wide range of organic and plant foods. The term "ruminants" refers also to exotic ruminants, like captive nyala, gemsbok, Arabian oryx, eland, kudu, scimitar-homed oryx, ankole, or bison which are also accessible to develop spongiform encephalopathy.

Also described herein is a method for detecting prion infections and/or prion diseases in cells, cell cultures and/or cell lysates comprising:
a) providing said cells, cell cultures and/or cell lysates; and
b) adding to said cells, cell cultures and/or cell lysates a pharmaceutically effective amount of at least one pharmaceutically active agent; and
c) detecting activity in said sample of at least one human cellular protein kinase, phosphatase or cellular signal transduction molecule selected from the group comprising FGF-R1, Tkt, Abl, dk1, MKK7, LIMK-2, CaM-KI, JNK2, CDC2, PRK, PTP-SL, PTP-zeta, HSP86, GPIR-1.

Furthermore, it has been shown that the inhibition of at least one target selected from the group comprising FGF-R1, Tkt, Abl, clk1, MKK7, LiMK-2, CaM-KI, JNK2, CDC2, PRK, PTP-SL, PTP-zeta, HSP86, GPIR-1 has an effect on the production of prions. Therefore, another method described herein is a method for regulating the production of prions in an individual or in cells comprising the step of administering a pharmaceutically effective amount of at least one pharmaceutically active agent which inhibits at least partially the activity of at least one human cellular protein kinase, phosphatase or cellular signal transduction molecule selected from the group comprising FGF-R1, Tkt, Abl, clk1, MKK7, LIMK-2, CaM-KI, JNK2, CDC2, PRK, PTP-SL, PTP-zeta, HSP86, GPIR-1, or which inhibits at least partially the production of at least one human cellular protein kinase, phosphatase or cellular signal transduction molecule selected from the group comprising FGF-R1, Tkt, Abl, clk1, MKK7, LIMK-2, CaM-KI, JNK2, CDC2, PRK, PTP-SL, PTP-zeta, HSP86, GPIR-1.

The compounds according to general formula (I) are examples for the above-mentioned pharmaceutically active agent. Preferably the targets FGF-R1, Tkt, Abl, clk1, MKK7, LIMK-2, CaM-KI, and CDC 2 are used with said methods.

Another embodiment of the present invention utilizes the scientific findings that some targets such as JNK2, PRK, PTP-SL, PTP-zeta, HSP86, and GPIR-1 are downregulated during prion infection and that upregulation of the effected target by means of an activator leads to an alternative way of treating prion infections and diseases associated with prion infection.

Thus, a method was developed for regulating the production of prions either in an individual or in cells. Said methods comprise the step of administering an individual or the cells a pharmaceutically effective amount of at least one pharmaceutically active agent wherein said agent activates at least partially the activity of at least one human cellular protein kinase, phosphatase or cellular signal transduction molecule selected from the group comprising FGF-R1, Tkt, Abl, clk1, MKK7, LIMK-2, CaM-KI, JNK2, CDC2, PRK, PTP-5L, PTP-zeta, HSP86, and GPIR-1, or wherein said agent at least partially activates or stimulates the production of at least one human cellular protein kinase, phosphatase or cellular signal transduction molecule selected from the group comprising FGF-R1, Tkt, Abl, Clk1, MKK7, LIMK-2, CaM-KI, JNK2, CDC2, PRK, PTP-SL, PTP-zeta, HSP86, and GPIR-1.

Preferably the targets JNK2, PRK, PTP-SL, PTP-zeta, HSP86, and GPIR-1 are used within the above-described methods.

Because of the fact that the organism may upregulate a given target such as FGF-R1. Tkt, Abl, clk1, MKK7, LIMK-2, CaM-KI, and CDC 2 In order to compete with the prion infection, it is also a reasonable approach to further support said upregulation by means of an activator. Therefore, the above-mentioned methods apply either to targets which are downregulated but also to targets which are upregulated.

The novel and partially known pyridylpyrimidine compounds of the general formula (I) represent a new class of pharmaceutical highly useful for the prophylaxis and treatment of prion infections and prion diseases.

As used herein the Term "prion diseases" refers to transmissible spongiform encephalopathies. This group of neurologic diseases affects humans and many species of animals causing a "sponge-like" degeneration of brain tissue. Among other unique features, all of these diseases are associated with the accumulation of an abnormal form of the prion protein in nerve cells that eventually leads to the death of the host. While prion diseases can all be transmitted from one host to another, it remains contentious as to whether a virus-like infectious agent or the abnormal prion protein itself, the prion, causes the conversion of normal to abnormal protein.

Probably most mammalian species develop prion diseases. Specific examples for animals include:
- **Scrapie** sheep, goat
- **TME** (transmissible mink encephalopathy): mink
- **CWD** (chronic wasting disease): muledeer, deer, elk
- **BSE** (bovine spongiform encephalopathy): cows, cattles

Humans are also susceptible to several prion diseases. Examples are:
- **CJD** Creutzfeld-Jacob Disease
- **GSS** Gerstmann-Sträussler-Scheinker syndrome
- **FFI** Fatal familial Insomnia
- **Kuru**
- **Alpers Syndrome**

The human prion diseases include kuru, sporadic Creutzfeldt-Jakob disease (sCJD), familial CJD (fCJD), iatrogenic CJD (iCJD), Gerstmann-Sträussler-Scheinker (GSS) disease, fatal familial insomnia (FFI), and, more recently, new variant CJD (nvCJD or vCJD). In addition to these human diseases, prion-related diseases, have been recognized in several animal hosts. Scrapie is a naturally occurring disease of sheep and goats that causes ataxia, behavioral changes, and a severe pruritus that leads to scraping behavior, from which the disease was named. Additional prion diseases in animals include transmissible mink encephalopathy (TME), chronic wasting disease (CWD) of deer and elk, feline spongiform encephalopathy (FSE), and bovine spongiform encephalopathy (BSE), among others.

The transmissible nature of prion disease was first demonstrated experimentally in 1936 when Cuillé and Chelle transmitted scrapie to a healthy goat by the intraocular administration of scrapie-infected spinal cord. Thirty years later, sCJD was transmitted to chimpanzees. The pathologic feature common to all these diseases is a prominent vacuolation of the gray matter of the brain that produces a "sponge-like" appearance on light microscopy. This histopathologic appearance, coupled with the transmissible nature of these diseases, led to their collective designation as "transmissible spongiform encephalopathies" or TSEs.

The etiologic agent of the TSEs was proposed to be a "slow virus" to explain its transmissible nature and the prolonged incubation period observed during experimental transmission studies. Early experiments suggested that protein may be a critical component of the infectious agent. These studies established the basis for a new form of a transmissible pathogen, one that is composed ostensibly of only protein and lacks any replicative elements such as nucleic acid.

The term "prion" was coined to indicate an *in*fectious agent with proteinlike properties. The unusual properties of the pathogen were demonstrated in early experiments in which conditions that degrade nucleic acids, such as exposure to ionizing and ultraviolet radiation, did not reduce the infectivity of scrapie fractions. On the other hand, treatments that degrade protein, such as prolonged exposure to proteases, correlated with a reduction in infectivity. A protein with relative resistance to protease digestion was found to be consistently present in the brains of animals and humans with TSE. Surprisingly, this protein was found to be one that is normally encoded by a chromosomal gene of the host.

Thus, the question raised, how a normally expressed protein could also be a transmissible pathogen? It was hypothesized and later demonstrated that PrP exists in two major isoforms: the nonpathogenic or cellular form, designated PrP^{c}, and the pathogenic or scrapie-inducing form, designated PrP^{Sc}. Both PrP^{c} and PrP^{Sc} have the same amino acid sequence, yet they differ in their biochemical properties: PrP^{c} is soluble in nondenaturing detergents and completely degraded by proteases, whereas PrP^{Sc} is insoluble in nondenaturing detergents and shows a relative resistance to proteases. Structural studies of PrP^{c} and PrP^{Sc} indicate a difference in the conformation of the two isoforms: PrP^{c} is predominantly helical, whereas PrP^{Sc} contains at least 40% pleated sheet structure. Conversion to this sheet structure appears to be the fundamental event in prion disease. The ultimate mechanism of how cells die coincident with the generation of prions is still unclear. Simple accumulation of pathogenic protein may not be sufficient to explain disease, however, it may constitute a critical step in cellular dysfunction.

It was shown that the pyridylpyrimidine compounds of the general formula (I) are highly effective for the prophylaxis and/or treatment of prion infections and/or prion diseases selected from the group comprising Scrapie, TME, CWD, BSE, CJD, vCJD, GSS, FFI, Kuru, and Alpers Syndrome. Preferably, the pyridylpyrimidine derivatives are used for preventing and/or treating BSE, vCJD, or CJD.

The above-mentioned prion infections and/or diseases associated with prion infections can be treated using the inventive pyridylpyrimidine derivatives by targeting at least one of the human cellular protein kinases, phosphatases or cellular signal transduction molecules selected from the group comprising FGF-R1, Tkt, Abl, clk1, MKK7, LIMK-2, CaM-KI, JNK2, CDC2, PRK, PTP-SL, PTP-zeta, HSP86, GPIR-1. Thereby, the compounds according to general formula (I) act as inhibitors for at least one of the above-mentioned targets and especially as inhibitors for at least one enzyme selected from the group comprising FGF-R1, Tkt, Abl, clk1, MKK7, LIMK-2, CaM-KI, and CDC 2.

According to these findings a further method described herein is a method for preventing and/or treating prion infections and/or prion diseases in an individual comprising the step of administering a pharmaceutically effective amount of at least one pharmaceutically active agent which inhibits at least partially the activity of at least one human cellular protein kinase, phosphatase or cellular signal transduction molecule selected from the group comprising FGF-R1, Tkt, Abl, clk1, MKK7, LIMK-2, CaM-KI, JNK2, CDC2, PRK, PTP-SL, PTP-zeta, HSP86, GPIR-1, or which inhibits at least partially the production of at least one human cellular protein kinase, phosphatase or cellular signal transduction molecule selected from the group comprising FGF-R1, Tkt, Abl, clk1, MKK7, LIMK-2, CaM-Kl, JNK2, CDC2, PRK, PTP-SL, PTP-zeta, HSP86, GPIR-1.

Another method is described herein a method for preventing and/or treating prion infections and/or prion diseases in cells or cell cultures comprising the step of administering a pharmaceutically effective amount of at least one pharmaceutically active agent which inhibits at least partially the activity of at least one human cellular protein kinase, phosphatase or cellular signal transduction molecule selected from the group comprising FGF-R1, Tkt, Abl, clk1, MKK7, LIMK-2, CaM-KI, JNK2, CDC2, PRK, PTP-SL, PTP-zeta, HSP86, GPIR-1, or which inhibits at least partially the production of at least one human cellular protein kinase, phosphatase or cellular signal transduction molecule selected from the group comprising FGF-R1, Tkt, Abl, clk1, MKK7, LIMK-2, CaM-KI, JNK2, CDC2, PRK, PTP-SL, PTP-zeta, HSP86, GPIR-1.

The pyridylpyrimidine compounds of formula (I) are examples for the above-mentioned inhibitor. Said pyridylpyrimidine compounds and/or pharmaceutically acceptable salts thereof are administered in a dosage corresponding to an effective concentration in the range of 0.01 - 50 µM, preferably in the range of 0.01 -10 µM, more preferably in the range of 0.01 - 1 µM, and most preferably in the range of 0.01 - 0.1 µM.

Because of the fact that the targets JNK2, PRK, PTP-SL, PTP-zeta, HSP86, and GPIR-1 are downregulated in cells infected with prions, an upregulation of said targets represents another strategy in order to treat prion infections and diseases like CJD (nvCJD or vCJD) associated with prion infections. Said upregulation can be performed by activators.

An agent that is able to upregulate, increase, activate, or stimulate the activity of at least one human cellular protein kinase, phosphatase or cellular signal transduction molecule selected from the group comprising FGF-R1, Tkt, Abl, clk1, MKK7, LIMK-2, CaM-KI, JNK2, CDC2, PRK, PTP-SL, PTP-zeta, HSP86, and GPIR-1, but especially of JNK2, PRK, PTP-SL, PTP-zeta, HSP86, and GPIR-1 is named "activator".

Thus, another method described herein is a method for preventing and/or treating prion infections and/or diseases in an individual comprising the step of administering a pharmaceutically effective amount of at least one pharmaceutically active agent which activates at least partially the activity of at least one human cellular protein kinase, phosphatase or cellular signal transduction molecule selected from the group comprising FGF-R1, Tkt, Abl, clk1, MKK7, LIMK-2, CaM-KI, JNK2, CDC2, PRK, PTP-SL, PTP-zeta, HSP86, and GPIR-1, or which activates or stimulates the production of at least one human cellular protein kinase, phosphatase or cellular signal transduction molecule selected from the group comprising FGF-R1, Tkt, Abl, clk1, MKK7, LIMK-2, CaM-KI, JNK2, CDC2, PRK, PTP-SL, PTP-zeta, HSP86, and GPIR-1. Preferably, said method is directed to the targets JNK2, PRK, PTP-SL, PTP-zeta, HSP86, and GPIR-1.

As used herein, the term "agent or "pharmaceutically active agent" refers to any chemical compound capable of down- or upregulating, de- or increasing, suppressing, activation, stimulating or otherwise regulating the amount and/or activity of at least one human cellular protein kinase, phosphatase or cellular signal transduction molecule selected from the group comprising FGF-R1, Tkt, Abl, clk1, MKK7, LIMK-2, CaM-Kl, JNK2, CDC2, PRK, PTP-SL, PTP-zeta, HSP86, and GPIR-1. Generally, said agents may be proteins, oligo- and polypeptides, nucleic acids, genes, aptamers, small chemical molecules, or other chemical moieties. An agent may be either an inhibitor or an activator and especially an inhibitor for the enzymes FGF-R1, Tkt, Abl, clk1, MKK7, LIMK-2, CaM-KI, and CDC 2 and an activator for the targets JNK2, PRK, PTP-SL, PTP-zeta, HSP86, and GPIR-1.

One special kind of said pharmaceutically active agents are aptamers which function as regulators of the activity of a wide range of cellular molecules such as human cellular protein kinase and phosphatase. Aptamers are nucleic acid molecules selected in vitro to bind small molecules, peptides, or proteins with high affinity and specificity. Aptamers not only exhibit highly specific molecular recognition properties but are also able to modulate the function of their cognate targets in a highly specific manner by agonistic or antagonistic mechanisms. Most famous examples for aptamers are DNA aptamers or RNA aptamers.

Further examples for pharmaceutically active agents are the pyridylpyrimidine compounds of the present invention and/or pharmaceutically acceptable salts thereof. Said compounds are administered in a dosage corresponding to an effective concentration in the range of 0.01 - 50 µM, preferably in the range of 0.01 - 10 µM, more preferably in the range of 0.01 - 1 µM, and most preferably in the range of 0.01 - 0.1 µM.

The compounds of general formula (I) can be administered in a daily dosage in the range of 25 mg to 1000 mg, preferably in a daily dosage of 400 mg to 600 mg, more preferably in a daily dosage of 500 mg, and most preferably in continuously increased daily dosages starting at a initial daily dosage of 400 mg and ending up in a daily dosage of 600 mg at the end of the treatment.

A question is how PrP^{c} does convert to PrP^{Sc}? Potential mechanisms that initiate conversion of PrP^{c} to PrP^{Sc} include a germ line mutation of the human prion protein gene (PRNP), a somatic mutation within a particular neuron, and spontaneous conversion of PrP^{c} to an aberrant conformation that is not refolded appropriately to its native structure. The prion protein gene (PRNP) is the single gene on the short arm of chromosome 20 in humans which encodes the normal cellular isoform of the prion protein. Regardless of the initiating event, once an "infectious unit" has been generated, PrP^{Sc} appears to act as a conformational template by which PrP^{c} is converted to a new molecule of PrP^{Sc} through protein-protein interaction of PrP^{Sc} and PrP^{c}. This concept is supported by several studies which show that mice with the normal PrP gene deleted (PrP knockout mice) do not develop prion disease after inoculation with scrapie. Furthermore, transgenic (Tg) mice that express a chimeric PrP gene made of human and mouse segments develop protease-resistant chimeric mouse-human PrP^{Sc} in their brains when inoculated with brain extracts from humans with prion disease. These findings clearly illustrate that prions do not self-replicate but instead convert nonpathogenic PrP^{c} to pathogenic PrP^{Sc}.

In its sporadic or nonfamilial form, **CJD** is the most common of the human prion diseases. Confusion and forgetfulness which progress rapidly to severe cortical dementia in combination with ataxia, myoclonus, and an abnormal electroencephalogram (EEG) represents the "classic tetrad" of CJD. However, a host of other neurologic signs and symptoms, including diffuse or focal weakness, painful neuropathy, chore-iform movements, hallucinations, cortical blindness, primary language disturbance, supranuclear ophthalmoplegia, and alien hand syndrome, among others, have been observed. As the disease progresses from the early stage, ataxia commonly limits the patient's mobility.

**Familial CJD** (fCJD) includes those cases with a dominantly inherited mutation of the *PRNP* gene, in which the pathologic features of spongiform change occur in the absence of GSS-type plaques. Although, familial cases of CJD tend to have a clinical and pathologic phenotype similar to that of sCJD.

The original description of a patient with the onset of ataxia and dysarthria followed by variable degrees of pyramidal and extrapyramidal symptoms and late developing dementia defines the classic presentation of **GSS**. The duration of said disease ranges from 2 to 10 years. Death usually results from secondary infection, often from aspiration pneumonia because of impaired swallowing. The presence of plaque deposits regionally or diffusely throughout the cortex that are immunoreactive to anti-human PrP antibodies is the hallmark of this form of prion disease.

**FFI** is a genetic disorder which manifests itself by many symptoms due to the degeneration of a certain part of the brain, the thalamus. The affected area of the brain is the area responsible for sleep, the thalamus. The thalamus is the center which communications from the brain to the body and the body to the brain pass through for proper directions to where a signal should be received. When sleep takes place, it is thought that the thalamus becomes less efficient at this signal transfer function allowing for the vegetative state of sleep to come over an individual. Consequently, the symptoms of fatal familial insomnia are directly related to the malfunction of the responsibilities of the thalamus, namely sleep.

There are four stages of the disease before an individual's life ends. The first stage is progressive insomnia, the characteristic feature of fatal familial insomnia. By now, there is no cure for this illness.

The term "familial" means: affecting several members of the same family, usually as a result of an underlying genetic mutation.

The occurrence of **vCJD** is sobering because it appears to represent a situation in which the prion has "jumped" species, in this case from cow to human. Because the pathologic features and clinical presentation of vCJD differ significantly from those of sCJD, it is considered a new "strain" of human prion disease. The same "protein signature" was observed following experimental transmission of BSE to several animal hosts, supporting the idea that vCJD results from the infection of humans with BSE. vCJD occurs primarily in younger individuals (average age 27) with a somewhat protracted course of approximately 16 months. The brain shows diffuse vacuolation and the presence of distinctive dense core PrP-containing plaques surrounded by a halo of spongiform change.

**Kuru** is the condition which first brought prion diseases to prominence in the 1950s. The disease was found in geographically Isolated tribes in New Guinea. It was established that ingesting brain tissue of dead relatives for religious reasons was likely to be the route of transmission.

**Alpers Syndrome** is the name given to prion diseases in infants.

**Scrapie** is the accepted, albeit somewhat colloquial, name for the naturally occurring transmissible spongiform encephalopathy of sheep and goats found worldwide. Scrapie also infects laboratory mice and hamsters making it one of the most important sources of new scientific information about this group of disorders. Scrapie was the first example of this type of disease to be noticed and has been known about for many hundreds of years. There are two possible methods of transmission in sheep: a) Infection of pasture with placental tissue carrying the agent followed by ingestion, or b) direct sheep-lamb transmission.

**CWD** is a fatal neurodegenerative disease of deer and elk, now known to be a transmissible spongiform encephalopathy. To date, affected animals have been found exclusively in the United States.

### BSE

Bovine spongiform encephalopathy or "mad cow disease" appears to have originated from scrapie that has been recognized in Europe since the mid-18th century. It has since spread to most sheep-breeding countries and is widespread in the United Kingdom, where until 1988 the rendered carcasses of livestock (including sheep) were fed to ruminants and other animals as a protein-rich nutritional supplement.

During rendering, carcasses from which all consumable parts had been removed were milled and then decomposed in large vats by boiling at atmospheric or higher pressures, producing an aqueous slurry of protein under a layer of fat (tallow). After the fat was removed, the slurry was desiccated into a meat and bone meal product that was packaged by the animal food Industry and distributed to owners of livestock and other captive animals (e.g., zoo and laboratory animals, breeding species, pets).

A further method is described herein is a method for regulating the expression of at least one human cellular protein kinase, phosphatase or cellular signal transduction molecule selected from the group comprising FGF-R1, Tkt, Abl, clk1, MKK7, LIMK-2, CaM-KI, JNK2, CDC2, PRK, PTP-SL, PTP-zeta, HSP86, and GPIR-1 in an individual comprising the step of administering the individual a pharmaceutically effective amount of at least one pharmaceutically active agent wherein said agent inhibits at least partially the transcription of DNA or the translation of RNA.

A still further method described herein is a method for regulating the expression of at least one human cellular protein kinase, phosphatase or cellular signal transduction molecule selected from the group comprising FGF-R1, Tkt, Abl, clk1, MKK7, LIMK-2, CaM-KI, JNK2, CDC2, PRK, PTP-SL, PTP-zeta, HSP86, and GPIR-1 in the cells, the method comprising the step of administering the cells a pharmaceutically effective amount of at least one pharmaceutically active agent wherein said agent inhibits at least partially the transcription of DNA or the translation of RNA.

As used herein, the term "regulating expression and/or activity" generally refers to any process that functions to control or modulate the quantity or activity (functionality) of a cellular component. Static regulation maintains expression and/or activity at some given level. Upregulation refers to a relative increase In expression and/or activity. Accordingly downregulation refers to a relative decrease in expression and/or activity. Downregulation is synonymous with inhibition of a given cellular component's activity.

The transcription of DNA and the translation of RNA can be inhibited by oligonucleotides or oligonucleotide derivatives. Thus, disclosed herein are oligonucleotides and derivatives of oligonucleotides which may be used in the above-mentioned methods. The oligonucleotide and/or its derivatives bind to the DNA and/or RNA encoding a human cellular protein kinase, phosphatase or a cellular signal transduction molecule selected from the group comprising FGF-R1, Tkt, Abl, clk1, MKK7, LIMK-2, CaM-Kl, JNK2. CDC2, PRK, PTP-SL, PTP-zeta, HSP86, and GPIR-1 and suppress the transcription of DNA or translation of RNA.

As described above, said prion infection and/or disease associated with said prion infection is selected from the group comprising Scrapie, TME, CWD, BSE, vCJD, CJD, GSS, FFI, Kuru, and Alpers Syndrome. Preferably, the method is used for prophylaxis and/or treatment of BSE, vCJD, or CJD. The above disclosed methods are preferably applied to CJD, vCJD, and BSE, more preferably applied to vCJD and BSE, and most perferably applied to BSE.

Some methods described herein identify compounds useful for prophylaxis and/or treatment of prion infections and/or diseases by screening a test compound, or a library of test compounds, for its ability to inhibit at least one of the above-mentioned human cellular protein kinases, phosphatases, or cellular signal transduction molecules, identified herein as characteristically up- or downregulated during prion production or growth inside a cell or individual. A variety of assay protocols and detection techniques are well known in the art and easily adapted for this purpose by a skilled practitioner. Such methods include, but are not limited to, high throughput assays (e.g., microarray technology, phage display technology), and *in vitro* and *in vivo* cellular and tissue assays.

Thus, a solid support is disclosed herein which is useful for screening compounds useful for the prophylaxis and/or treatment of prion infections and/or diseases in an individual, the solid support comprising at least one immobilized oligonucleotide, wherein said oligonucleotide encodes one human cellular protein kinase, phosphatase or cellular signal transduction molecule selected from the group comprising FGF-R1. Tkt, Abl, clk1, MKK7, LIMK-2, CaM-KI, JNK2, CDC2, PRK. PTP-SL, PTP-zeta, HSP86. and GPIR-1.

Also described herein is a solid support useful for screening compounds useful for the prophylaxis and/or treatment of prion infections and/or diseases in an individual, the solid support comprising at least one immobilized human cellular protein kinase, phosphatase or cellular signal transduction molecule selected from the group comprising FGF-R1, Tkt, Abl, clk1, MKK7, LIMK-2, CaM-KI, JNK2, CDC2, PRK, PTP-SL, PTP-zeta, HSP86, and GPIR-1.

A component of the above-mentioned methods may comprise peptide fragments of one or more of the above-identified human cellular protein kinases, phosphatases or cellular signal transduction molecules immobilized on a solid support Once again the most preferred solid support would contain polymers of sufficient quality and quantity to detect all of the above-mentioned human cellular protein kinases, phosphatase and cellular signal transduction molecules (e.g., a nucleic acid or a peptide microarray). A variety of supports and constructions of the same for the methods disclosed herein are well known In the art and easily adapted for this purpose by a skilled practitioner (cf., for example: Marschall, 1999 "Do-it-yourself gene watching" Science 286, 444-447; Service 2000 "Protein arrays step out of DNA's shadow" Science 289, 1673).

It is preferred that mRNA is measured as an indication of expression. Methods for assaying for mRNA include, but are not limited to, Northern blots, slot blots, dot blots, and hybridization to an ordered array of oligonucleotides. Nucleic acid probes useful for assay of a sample are preferably of sufficient length to specifically hybridize only to appropriate, complementary transcripts. Typically the oligonucleotide probes will be at least 10 to 25 nucleotides in length. In some cases longer probes of at least 30 to 50 nucleotides will be desirable.

The cDNA oligonucleotides immobilized on said membrane filter which are used for detecting the up- or downregulation of the above-mentioned human cellular protein kinases, phosphatases, and cellular signal transduction molecules by hybridization to the radioactively labeled cDNA probes have the nucleotide sequences listed in table 1.

**Table 1: Nucleotide sequences of cDNA-arrays**

| Cellular kinase, phosphatase, or signal transduction molecule | Sequence of immobilized DNA on arrays (in relation to the respective Acc No) |
|---|---|
| FGF-R1 | 41 bp - 2619bp (X52833) |
| Tkt (EC 2.7.1.112) | 1 bp - 3096bp (X74764) |
| Abl | 2153 bp - 3765 bp (M14752) |
| clk1 | 156 bp -1610 bp (L29219) |
| MKK7 | 77 bp - 1323 bp (AF013588) |
| CDC2 | 77 bp - 1050 bp (X05360) |
| CaMKI | 145 bp - 1452 bp (L41816) |
| JNK2 | 507 bp - 1782 bp (L31951) |
| LIMK-2 | 963 bp - 2047 bp (D45906) |
| PRK | n.a bp - 1862 bp (U56998) |
| PTP zeta (EC 3.1.3.48) | 148 bp - 7604 bp (X54135) |
| PTP-SL | 862 bp - 1902 bp (NM 002849) |
| HSP86 | n.a bp - n.a bp (X07270) |
| GPIR-1 | n.a bp - n.a bp (n.a) |

| | |
|---|---|
| Tkt has been assigned to the EC Number. 2.7.1.112 | |
| PTP zeta has been assigned to the EC Number: 3.1.3.48 | |

The nudeoside sequences of the genes coding for the human cellular protein kinases, phosphatases, or cellular signal transduction molecules listed in Table 1 together with the amino add sequences and the enzyme commission numbers (E.C. numbers) of said enzymes can be obtained from NCBI (National Library of Medicine: PubMed; Web address: www.ncbi.nlm.nih.gov/entrez).

The polypeptide product of gene expression may be assayed to determine the amount of expression as well. Methods for assaying for a protein include, but are not limited to, western blot, immuno-precipitation, radioimmuno assay, and peptide Immobilization in an ordered array, It is understood, however, that any method for specifically and quantitatively measuring a specific protein or mRNA product can be used.

A variety of supports upon which nudeic acids or peptides can be Immobilized are known in the art, for example filters, or polyvinyl chloride dishes. Any solid surface to which oligonudeotides or peputies can be bound, either directly or indirectly, either covalently or non-covalently, can be used. A preferred solid support is a microarray membrane filter or a "biochip". These contain particular polymer probes in predetermined locations on the array. Each predetermined location may contain more than one molecule of the probe, but each molecule within the predetermined location has an identical sequence.

The methods described herein may involve the use of techniques well known in the field of molecular biology. These techniques include, but are not limited to, techniques described in the following publications:
Ausubel, F.M. et al. eds., "Short Protocols In Molecular Biology" 4^{th} Ed. 1999, John Wiley & Sons, NY (ISBN 0-471-32938-X);
Old, R.W. & S.B. Primrose "Principles of Gene Manipulation: An Introduction To Genetic Engineering" 3^{rd} Ed. 1985, Blackwell Scientific Publications, Boston. Studies in Microbiology: V.2, 409 pp. (ISBN 0-632-01318-4);
Mayer, R.J. & J.H. Walker eds. "Immunochemical Methods In Cell and Molecular Biology" 1987, Academic Press, London. 325 pp. (ISBN 0-12480-855-7);
Winnacker, E.L. "From Genes To Clones: Introduction To Gene Technology" 1987 VCH Publishers, NY. (translated by Horst Ibelgaufts) 634 pp. (ISBN 0-89573-614-4).

As described above, a microarray platform technology was developed consisting of more than 1100 signal transduction cDNAs immobilized on a solid support. Thus, also described herein is a solid support useful for detecting prion infections and/or diseases in an individual, the solid support comprising an immobilized oligonucleotide, wherein said oligonudeotide is capable of detecting activity of at least one human cellular protein kinase, phosphatase or cellular signal transduction molecule selected from the group comprising FGF-R1, Tkt, Abl, clk1, MKK7, LIMK-2, CaM-KI, JNK2, CDC2, PRK. PTP-SL, PTP-zeta, HSP86, and GPIR-1.

Disclosed herein is also for the first time a solid support useful for detecting prion infections and/or diseases in cells, the solid support comprising an immobilized oligonucleotide, wherein said oligonucleotide is capable of detecting activity of at least one human cellular protein kinase, phosphatase or cellular signal transduction molecule selected from the group comprising FGF-R1, Tkt, Abl, clk1, MKK7. LIMK-2, CaM-KI, JNK2. CDC2, PRK, PTP-SL. PTP-zeta. HSP86, and GPIR-1.

The subject matter described herein may also involve the use of techniques well known in the field of microarray construction and analysis. These techniques include, but are not limited to, techniques described in the following patents and patent applications describing array of biopolymeric compounds and methods for their fabricadon:
U.S. Pat. Nos. 5,807,522; 6,087,102; WO 93/17126: WO 95/11995; WO 95/35505; EP 742 287; and EP 799 897.

Techniques also include, but are not limited to, techniques described in the following patents and patent application describing methods of using arrays in various applications:
U.S. Pat. Nos. 5,994,076; 6,033,860; 6,040,138; 6,040,140; WO 95/21265; WO 96/31622; WO 97/10365; WO 97/27317; EP 373 203; and EP 785 280

Still a further aspect of the present invention is directed to pharmaceutical compositions comprising at least one pharmaceutically active agent together with a pharmaceutically acceptable carrier, excipient or diluents. Examples for pharmaceutically active agents are the above-mentioned inventive compounds according to formula (I). Said prion infections and diseases are preferably Scrapie, TME, CWD, BSE, vCJD, CJD, GSS, FFI, Kuru, and Alpers Syndrome.

Thus, the pharmaceutical compositions according to the present invention may comprise an inhibitor, such as the inventive pyridylpyrimidine compounds for at least one target selected from FGF-R1, Tkt, Abl, clk1, MKK7, LIMK-2, CaM-KI, JNK2, CDC2, PRK, PTP-SL, PTP-zeta, HSP86, and GPIR-1. It is also possible to have a combination of inhibitors or activators as active ingredients in one single pharmaceutical composition. Furthermore, suitable are also combinations of at least one inhibitor and at least one activator for different targets within a single pharmaceutical composition. For example, a pharmaceutical composition could comprise compound 12 as an inhibitor for, for instance, the target Abl, and an activator such as an aptamer for, for instance, the human cellular protein kinase JNK2.

Said pharmaceutical compositions are useful for the prophylaxis and/or treatment of an individual afflicted with prions comprising at least one agent capable of inhibiting and/or activating at least partially the activity, the expression, and/or the production of at least one human cellular protein kinase, phosphatase or cellular signal transduction molecule selected from the group comprising FGF-R1, Tkt, Abl, clk1, MKK7, LIMK-2, CaM-KI, JNK2, CDC2, PRK, PTP-SL, PTP-zeta, HSP86, and GPIR-1.

The pyridylpyrimidine compounds of the present invention are basic and form pharmaceutically acceptable salts with organic and inorganic acids. Examples of suitable acids for such acid addition salt formation are hydrochloric acid, hydrobromic acid, sulfuric acid, phosphoric acid, acetic acid, citric acid, oxalic acid, malonic acid, salicylic acid, p-aminosalicylic acid, malic acid, fumaric acid, succinic acid, ascorbic acid, maleic acid, sulfonic acid, phosphonic acid, perchloric acid, nitric acid, formic acid, propionic add, gluconic acid, lactic acid, tartaric acid, hydroxymaleic acid, pyruvic acid, phenylacetic acid, benzoic acid, p-aminobenzoic acid, p-hydroxybenzoic acid, methanesulfonic acid, ethanesulfonic acid, nitrous acid, hydroxyethanesulfonic acid, ethylenesulfonic acid, p-toluenesulfonic acid, naphthylsulfonic acid, sulfanilic acid, camphorsulfonic acid, china acid, mandelic acid, o-methylmandelic acid, hydrogen-benzenesulfonic acid, picric acid, adipic acid, d-o-tolyltartaric acid, tartronic acid, α-toluic acid, (o, m, p)-toluic acid, naphthylamine sulfonic acid, and other mineral or carboxylic acids well known to those skilled in the art. The salts are prepared by contacting the free base form with a sufficient amount of the desired acid to produce a salt in the conventional manner.

It is also possible to obtain acid addition salts with amino acids like methionine, tryptophane, lysine or arginine, especially with pyridylpyrimidine compounds of the general formula (I) carrying a carboxylic acid residue.

Depending upon the substituents on the inventive pyridylpyrimidine compounds, one may be able to form salts with bases, too. Thus, for example, if there are carboxylic acid substituents in the molecule, salts may be formed with inorganic as well as organic bases such as, for example, NaOH, KOH, NH₄OH, tetraalkylammonium hydroxide, and the like.

The compounds of the general formula (I) can also be administered in form of their pharmaceutically active salts optionally using substantially nontoxic pharmaceutically acceptable carriers, excipients or diluents. The medications of the present invention are prepared in a conventional solid or liquid carrier or diluents and a conventional pharmaceutically-made adjuvant at suitable dosage level in a known way. The preferred preparations are in administratable form which is suitable for oral application. These administratable forms, for example, include pills, tablets, film tablets, coated tablets, capsules, powders and deposits.

The preferred administratable forms are tablets, film tablets, coated tablets, gelatin capsules, and opaque capsules. Each pharmaceutical composition contains at least one compound of the general formula (I), preferably compound 53 and/or pharmaceutically acceptable salts thereof in an amount of 50 mg to 150 mg, preferably 80 mg to 120 mg, and most preferably in an amount of 100 mg per formulation.

Furthermore, the subject of the present invention also includes pharmaceutical preparations for parenteral, including dermal, intradermal, intragastrical, intracutaneous, intravasal, intravenous, intramuscular, intraperitoneal, intranasal, intravaginal, intrabuccal, percutaneous, rectal, subcutaneous, sublingual, topical or transdermal application, which in addition to typical vehicles and diluents contain a pyridylpyrimidine compound of the general formula (I) and/or a pharmaceutically acceptable salt thereof as active ingredient.

Within the disclosed methods the pharmaceutical compositions of the present invention, containing pyridylpyrimidine derivatives of the general formula (I) as active ingredients, will typically be administered in admixture with suitable carrier materials selected with respect to the intended form of administration, i.e. oral tablets, capsules (either solid-filled, semi-solid filled or liquid filled), powders for constitution, oral gels, elixirs, dispersible granules, syrups, suspensions, and the like, and consistent with conventional pharmaceutical practices. For example, for oral administration in the form of tablets or capsules, the active drug component may be combined with any oral nontoxic pharmaceutically acceptable inert carrier, such as lactose, starch, sucrose, cellulose, magnesium stearate, dicalcium phosphate, calcium sulfate, talc, mannitol, ethyl alcohol (liquid forms) and the like. Moreover, when desired or needed, suitable binders, lubricants, disintegrating agents and coloring agents may also be incorporated in the mixture. Powders and tablets may be comprised of from about 5 to about 95 percent inventive composition.

Suitable binders include starch, gelatin, natural sugars, corn sweeteners, natural and synthetic gums such as acacia, sodium alginate, carboxymethylcellulose, polyethylene glycol and waxes. Among the lubricants, there may be mentioned for use in these dosage forms, boric acid, sodium benzoate, sodium acetate, sodium chloride, and the like. Disintegrants include starch, methylcellulose, guar gum and the like. Sweetening and flavoring agents and preservatives may also be included where appropriate. Some of the terms noted above, namely disintegrants, diluents, lubricants, binders and the like, are discussed in more detail below.

Additionally, the compositions of the present invention may be formulated in sustained release form to provide the rate controlled release of any one or more of the components or active ingredients to optimize the therapeutic effects, i.e. antihistaminic activity and the like. Suitable dosage forms for sustained release include layered tablets containing layers of varying disintegration rates or controlled release polymeric matrices impregnated with the active components and shaped in tablet form or capsules containing such impregnated or encapsulated porous polymeric matrices.

Liquid form preparations include solutions, suspensions and emulsions. As an example may be mentioned water or water-propylene glycol solutions for parenteral injections or addition of sweeteners and opacifiers for oral solutions, suspensions and emulsions. Liquid form preparations may also include solutions for lntranasal administration.

Aerosol preparations suitable for inhalation may include solutions and solids in powder form, which may be in combination with a pharmaceutically acceptable carrier such as inert compressed gas, e.g. nitrogen.

For preparing suppositories, a low melting wax such as a mixture of fatty acid glycerides such as cocoa butter is first melted, and the active ingredient is dispersed homogeneously therein by stirring or similar mixing. The molten homogeneous mixture is then poured into convenient sized molds, allowed to cool and thereby solidifies.

Also included are solid form preparations which are intended to be converted, shortly before use, to liquid form preparations for either oral or parenteral administration. Such liquid forms Include solutions, suspensions and emulsions.

The pyridylpyrimidine compounds of the present invention may also be deliverable transdermally. The transdermal compositions may take the form of creams, lotions, aerosols and/or emulsions and can be Included in a transdermal patch of the matrix or reservoir type as are conventional in the art for this purpose.

The term capsule refers to a special container or enclosure made of methyl cellulose, polyvinyl alcohols, or denatured gelatins or starch for holding or containing compositions comprising the active ingredients. Hard shell capsules are typically made of blends of relatively high gel strength bone and pork skin gelatins. The capsule itself may contain small amounts of dyes, opaquing agents, plasticizers and preservatives.

Tablet means compressed or molded solid dosage form containing the active ingredients with suitable diluents. The tablet can be prepared by compression of mixtures or granulations obtained by wet granulation, dry granulation or by compaction well known to a person skilled in the art.

Oral gels refers to the active ingredients dispersed or solubilized in a hydrophillic semi-solid matrix.

Powders for constitution refers to powder blends containing the active ingredients and suitable diluents which can be suspended in water or juices.

Suitable diluents are substances that usually make up the major portion of the composition or dosage form. Suitable diluents include sugars such as lactose, sucrose, mannitol and sorbitol, starches derived from wheat, corn rice and potato, and celluloses such as microcrystalline cellulose. The amount of diluents in the composition can range from about 5 to about 95% by weight of the total composition, preferably from about 25 to about 75%, more preferably from about 30 to about 60% by weight.

The term disintegrants refers to materials added to the composition to help it break apart (disintegrate) and release the medicaments. Suitable disintegrants include starches, "cold water soluble" modified starches such as sodium carboxymethyl starch, natural and synthetic gums such as locust bean, karaya, guar, tragacanth and agar, cellulose derivatives such as methylcellulose and sodium carboxymethylcellulose, microcrystalline celluloses and cross-linked microcrystalline celluloses such as sodium croscarmellose, alginates such as alginic acid and sodium alginate, clays such as bentonites, and effervescent mixtures. The amount of disintegrant in the composition can range from about 2 to about 20% by weight of the composition, more preferably from about 5 to about 10% by weight.

Binders characterize substances that bind or "glue" powders together and make them cohesive by forming granules, thus serving as the "adhesive" in the formulation. Binders add cohesive strength already available in the diluents or bulking agent. Suitable binders include sugars such as sucrose, starches derived from wheat, corn rice and potato; natural gums such as acacia, gelatin and tragacanth; derivatives of seaweed such as alginic acid, sodium alginate and ammonium calcium alginate; cellulosic materials such as methylcellulose and sodium carboxymethylcellulose and hydroxypropylmethylcellulose; polyvinylpyrrolidone; and inorganics such as magnesium aluminum silicate. The amount of binder in the composition can range from about 2 to about 20% by weight of the composition, more preferably from about 3 to about 10% by weight, even more preferably from about 3 to about 6% by weight.

Lubricant refers to a substance added to the dosage form to enable the tablet, granules, etc. after it has been compressed, to release from the mold or die by reducing friction or wear. Suitable lubricants include metallic stearates such as magnesium stearate, calcium stearate or potassium stearate; stearic acid; high melting point waxes; and water soluble lubricants such as sodium chloride, sodium benzoate, sodium acetate, sodium oleate, polyethylene glycols and D,L-leucine. Lubricants are usually added at the very last step before compression, since they must be present on the surfaces of the granules and in between them and the parts of the tablet press. The amount of lubricant in the composition can range from about 0.2 to about 5% by weight of the composition, preferably from about 0.5 to about 2%, more preferably from about 0.3 to about 1.5% by weight.

Glidents are materials that prevent caking and improve the flow characteristics of granulations, so that flow is smooth and uniform. Suitable glidents include silicon dioxide and talc. The amount of glident in the composition can range from about 0.1% to about 5% by weight of the total composition, preferably from about 0.5 to about 2% by weight.

Coloring agents are excipients that provide coloration to the composition or the dosage form. Such excipients can include food grade dyes and food grade dyes adsorbed onto a suitable adsorbent such as clay or aluminum oxide. The amount of the coloring agent can vary from about 0.1 to about 5% by weight of the composition, preferably from about 0.1 to about 1%.

As used herein, a "pharmaceutically effective amount" of an inhibitor and/or an activator is an amount effective to achieve the desired physiological result, either in cells treated *in vitro* or in a subject treated *in vivo*. Specifically, a pharmaceutically effective amount is an amount sufficient to inhibit and or activate, for some period of time, one or more of the clinically defined pathological processes associated with the prion infection. The effective amount may vary depending on the specific inhibitor and/or activator selected, and is also dependent on a variety of factors and conditions related to the subject to be treated and the severity of the infection. For example, if an inhibitor and/or activator is to be administered *in vivo,* factors such as the age, weight and health of the patient as well as dose response curves and toxicity data obtained in pre-clinical animal work would be among those considered. If the inhibitor and/or activator is to be contacted with the cells *in vitro*, one would also design a variety of pre-clinical *in vitro* studies to assess such parameters as uptake, half-life, dose, toxicity, etc. The determination of a pharmaceutically effective amount for a given pharmaceutically active agent is well within the ability of those skilled in the art.

It is also apparent to a person skilled in the art that detection includes any method known in the art useful to indicate the presence, absence, or amount of a detection target. Such methods may include, but are not limited to, any molecular or cellular techniques, used singularly or in combination, including, but not limited to: hybridization and/or binding techniques, including blotting techniques and immunoassays; labeling techniques (chemiluminescent, colorimetric, fluorescent, radioisotopic); spectroscopic techniques; separations technology, including precipitations, electrophoresis, chromatography, centrifugation, ultrafiltration, cell sorting; and enzymatic manipulations (e.g., digestion).

It should be stressed that all above-mentioned features, aspects, and details of the present invention discussed and described in connection with infections and infectious diseases, equally apply to neurodegenerative diseases, like Alzheimer.

It is readily apparent to those skilled in the art that other suitable modifications and adaptations of the compositions and methods of the invention described herein are evident and may be made without departing from the scope of the invention or the embodiments disclosed herein. Having now described the present invention in detail, the same will be more clearly understood by reference to the following examples, which are included for purposes of illustration only and are not intended to be limiting of the invention.

### Description of figures

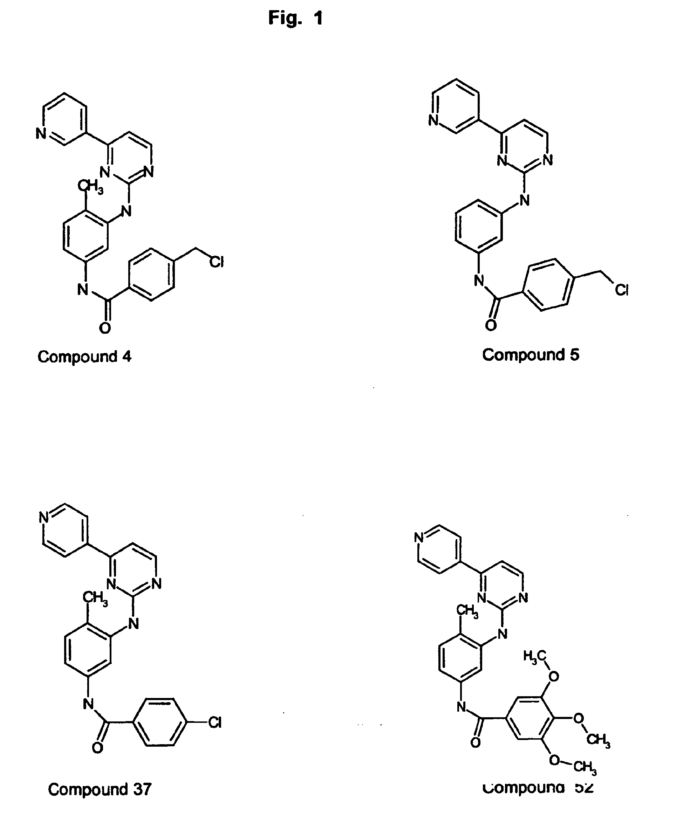

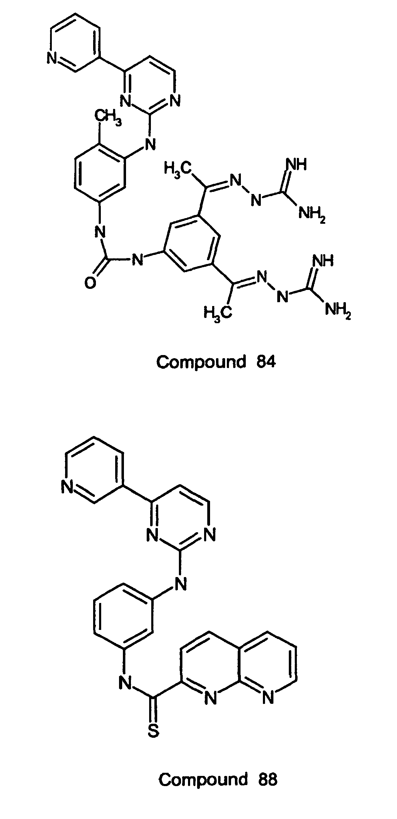

- Fig. 1: shows 6 selected pyridylpyrimidine derivatives which are suitable inhibitors for prion diseases, namely compounds 4, 5, 37, 52, 84, and 88;
- Fig. 2: shows the compound 4-(4-Methylpiperazin-1-ylmethyl)-*N*-[4-methyl-3-(4-pyridin-3-yl-pyrimidin-2-ylamino)-phenyl]-benzamide, also known as Gleevec^{™};
- Fig. 3: shows selected compounds that have been identified as potent inhibitors in a prion propagation assay at a concentration of 5 µm.

### Examples

### Materials and methods

### 1. Generation of cDNA-arrays on membranes

In order to manufacture cDNAs-arrays on membranes, the following strategy was pursued: cDNAs encoding parts of or full length proteins of interest - in the following referred to as "target cDNAs" - were cloned into the plasmid Bluescript II KS⁺ (Stratagene, USA). Large scale purifications of these plasmids were performed according to standard techniques and 200 µl aliquots (1 µg/µl plasmid concentration) were transferred into appropriate 96well plates. Plates were closed with sealing tape and chilled on ice for 5 minutes after incubation for 10 minutes at 95°C. 10 µl of 0.6 N NaOH were added and the mix was stored for 20 minutes at room temperature before addition of 10 µl 2.5 M Tris-HCI pH 7.1 and 20 µl 40x SSC (3 M NaCl, 300 mM Sodium Citrate, pH 7.0). Target cDNAs were spotted onto Nylon or Nitrocellulose membranes using a BioGrid (BioRobotics, UK) equipped with a 0.7 mm pintool. In this way, between 200 ng and 350 ng of plasmids encoding target cDNAs were transferred onto the membranes and crosslinked to the membranes by ultraviolet light (1.2x10⁵ µJ/cm²). The arrays were stored for use in subsequent experiments at room temperature.

### 2. Generation of cells

PrP^{Sc}- and PrP^{c}-transfected mouse neuronal cells (N2A) were cultured in MEM (Minimum Essential Medium, Life Technologies) supplemented with 10% fetal calf serum at 37°C and 5% CO₂ to obtain ~6x10⁶ cells per tissue culture flask.

### 3. Lysis of cells, isolation of total RNA and purification of polyA⁺ RNA

After incubation of the cells with the virus for the respective time-points, cells were washed twice with phosphate buffered saline (PBS) and then trypsinized. Subsequently, cells were removed from the culture dish by resuspension with PBS. Afterwards, cells were sedimented and directly lysed in Tri reagent by repetitive pipetting using in 1ml of Tri reagent (Molecular Research Centre, Inc., USA) per 1x10⁶ cells.

The lysates were stored at room temperature for 5 minutes and then centrifuged at 12000xg for 15 minutes at 4°C. The supernatant was mixed with 0,1 ml of 1-bromo-3-chloropropane per 1 ml of Tri reagent and vigorously shaken. The suspension was stored for 5 minutes at room temperature and then centrifuged at 12000xg for 15 minutes at 4°C.

The colourless upper phase was transferred into new tubes, mixed with 5 µl of poly-acryl-carrier (Molecular Research Centre, Inc., USA) and with 0.5 ml of isopropanol per 1 ml of Tri reagent and vigorously shaken. The samples were stored at room temperature for 5 minutes and then centrifuged at 12000xg for 8 minutes at 4°C. The supernatant was removed and the RNA pellet washed twice with 1 ml of 75% ethanol. The pellet was dried and resuspended for 10 minutes at 55°C in 50 µl of RNase-free buffer (5 mM Tris-HCl pH 7.5). The integrity of the isolated RNA was determined by agarose/formaldehyde gel electrophoresis and the RNA was finally stored at -70°C for use in subsequent experiments.

### 4. Preparation of radioactively labelled cDNA probes from RNA

In order to obtain radioactively labelled cDNA probes total RNA was transcribed into a cDNA-probe in the presence of radioactively labelled dATP. 12 µl bidestilled DEPC (Diethylpyrocarbonate) treated H₂O containing 0.5 µg of primer TXN (5'-TTT TTT TTT TTT TTT TXN-3' with T → dTTP; N → dATP, dCTP, dGTP or dTTP; X → dATP, dCTP or dGTP) and total RNA (1 to 10 µg) were shaken between 5 and 15' at 60°C and then incubated on ice for 2 minutes. After centrifugation (30 seconds, 10000xg) 7 µl of a mix consisting of 100 µCi dATP-P³³ (Amersham, UK) which were dried under vacuum previously and resuspended in 4 µl first strand buffer (Life Technologies, USA), 2 µl 0.1 M DTT (Dithiothreitol) and 1 µl labelling solution (4 mM dCTP, dGTP, dTTP each and 80 µM dATP final concentration) were added. Following the addition of 1 µl Superscript II reverse transcriptase (Life Technologies, USA) the reaction was incubated for 10 minutes at room temperature and then for 60 minutes at 38°C. Subsequently, the reaction was vigorously shaken for 30 minutes at 68°C after adding 5 µl 0.5 M EDTA and 25 µl 0.6M NaOH.

Unincorporated nucleotides were removed from the labelling reaction using ProbeQuant G-50 columns (Amersham, UK). The column was vigorously shaken and centrifuged for 1 minute at 735xg in an appropriate reaction tube after bottom closure and lid were removed. The column was placed into a new reaction tube, the probe was applied onto the centre of the column material and the column was centrifuged for 2 minutes at 735xg. The flow-trough was transferred into new reaction tubes and filled up to a volume of 100 µl with bidestilled H₂O. The probe was precipitated by centrifugation for 15 minutes at 12000xg after 4 µl 5M NaCl, 1 µl poly-acryl-carrier (Molecular Research Centre, Inc., USA) and 250 µl ethanol were added. The supernatant was discarded and the pellet was dried at 50°C for 5 minutes before starting with the hybridisation.

### 5. Hybridisation of radioactively labelled cDNA-probes to cDNA-arrays

The pellet was resuspended in 10 µl C₀T DNA (1 µg/µl, Roche Diagnostics, Germany), 10 µl yeast tRNA (1 µg/µl Sigma, USA) and 10 µl polyA (1 µg/µl, Roche Diagnostics, Germany) and incubated at 55°C for 5 minutes. Herring sperm DNA was added to a final concentration of 100 µg/ml and the volume was filled up to 100 µl with 5 µl 10% SDS (Sodiumdodecylsulfat), 25 µl 20x SSPE (3M Sodium chloride, 0,2 M Sodium dihydrogen phosphate monohydrate, 0,02 M Ethylenedinitrilo tetraacetic acid, disodium salt dihydrate; pH 7,4) and bidestilled H₂O. The mix was put on 95°C for 5 minutes, centrifuged for 30 seconds at 10000xg and vigorously shaken for 60 minutes at 65°C. A 1 µl aliquot of the probe was used to measure the incorporation of radioactive dATP with a scintillation counter. Probes with at least a total of 20x10⁶ cpm were used.

The arrays were prehybridised for at least 3 hours at 42°C in hybridisation solution in a roller bottle oven. After prehybridization the radioactively labelled probe was added into the hybridisation solution and hybridisation was continued for 20 to 40 hours.

The probe was discarded and replaced with wash solution A (2xSSC). The arrays were washed twice in wash solution A at room temperature in the roller oven. Afterwards, wash solution A was replaced by wash solution B (2x SSC, 0.5% SDS) preheated to 65°C and arrays were washed twice for 30 minutes at 65°C. Then, wash solution B was replaced by wash solution C (0.5x SSC, 0.5% SDS) preheated to 65°C and arrays were washed twice for 30 minutes at 65°C. The moist arrays were wrapped in airtight bags and exposed for 8 to 72 hours on erased phosphoimager screens (Fujifilm, Japan).

### 6. Analysis of cDNA-arrays

The exposed phosphoimager screens were scanned with a resolution of 100µ and 16bits per pixel using a BAS-1800 (Fujifilm, Japan). Files were imported into the computer program ArrayVision (Imaging Research, Canada). Using the program's features, the hybridization signals of each target cDNA were converted into numbers. The strength of the hybridization signals reflected the quantity of RNA molecules present in the probe. Differentially expressed genes were selected according to the ratio of their signal strength after normalization to the overall intensity of the arrays.

### 7. Cell culture and expression of 3F4-taaaed PrP (3F4-ScN2a)

The mouse neuroblastoma cell line 3F4-ScN2a represents a stably transfected clone of ScN2a cells (PrP^{Sc} infected N2a cells) which overexpress 3F4-epitope-tagged murine PrP. Residues 109 and 112 of murine PrP were replaced by methionine to introduce the epitope for reactivity with the monoclonal anti-PrP antibody 3F4. Cells were maintained in Dulbecco's modified Eagle's (DMEM) or Opti-MEM medium containing 10 % fetal calf serum, antibiotics and glutamin. For generation of stable transfectants we used the vector pcDNA3.1/Zeo (Invitrogen; Leek, The Netherlands). Lipofection of cells with recombinant plasmids was done using standard procedures and recombinant clones were selected by addition of 300 µg Zeocin/ml medium.

### 8. Treatment of cells with inhibitors

All tested compounds were solubilized in DMSO (dimethylsulfoxide), and prepared as 10 mM stock solutions. The drugs were applied to the cells described above for three days in final concentrations between 5 and 20 µM.

### 9. Immunoblot and proteinase K (PK) analysis

Confluent cell cultures were lysed in cold lysis buffer (10 mM Tris-HCl, pH 7.5; 100 mM NaCl; 10 mM EDTA; 0.5 % Triton X-100; 0.5 % DOC) (EDTA: ethylene diamine tetraacetate; Triton X-100: t-octylphenoxypolyethoxyethanol; DOC: deoxycholic acid). Postnuclear lysates were split between those with and without proteinase K digestion. Samples without proteinase K digestion were supplemented with proteinase inhibitors (5 mM PMSF, 0.5 mM Pefabloc, and aprotinin) (PMSF: phenylmethylsulfonyl fluoride) and directly precipitated with ethanol. Samples for proteinase K digestion were incubated with 20 µg/ml proteinase K for 30 min at 37°C; digestion was stopped with proteinase inhibitors, and samples were ethanol precipitated. After centrifuging for 30 min at 3,500 rpm the pellets were redissolved in TNE buffer (10 mM Tris-HCl pH7.5, 100 mM NaCl, 1mM EDTA) and gel loading buffer was then added. After boiling for 5 min an aliquot was analyzed on 12.5 % PAGE. For Western blot analysis, the proteins were electrotransferred to PVDF membranes (polyvinylidendifluorid). The membrane was blocked with 5 % non-fat dry milk in TBST (0.05 % Tween 20, 100 mM NaCl, 10 mM Tris-HCl, pH 7.8) (Tween 20: polyoxyethylenesorbitan monolaurate; Tris-HCl: Tris-(hydroxymethyl)-aminomethane-hydrochloride), incubated overnight with the primary antibody at 4°C and stained using the enhanced chemiluminescence blotting kit from Amersham Corporation. Specific immuno-staining of the PrP^{c} and PrP^{Sc} forms were obtained with the prion protein specific antibody 3F4 (Signet Pathologies, U.S.A.).

### 10. Results

Determination of the amount of the pathogenic form of the prion protein PrP^{Sc} upon treatment of prion infected cells with different types of small molecule protein kinase inhibitors resulted in the identification of a compound class of pyridylpyrimidine derivatives examplified by the compound 4-(4-Methylpiperazin-1-ylmethyl)-*N*-[4-methyl-3-(4-pyridin-3-yl-pyrimidin-2-ylamino)-phenyl]-benzamide (compound 53) and compounds 4, 5, and 37.

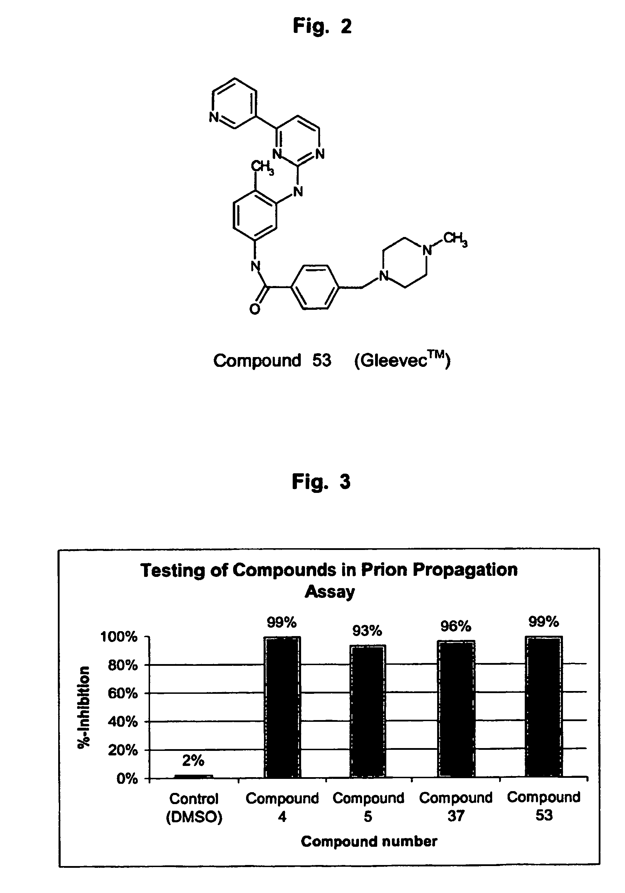

These compounds significantly reduced the amount of PrP^{Sc} in prion infected cells in a concentration range between 5 and 20 µM (final concentration). As shown in Fig. 3 the selected compounds 4, 5, 37, and 53 inhibit almost completely the activity of prion propagation within said concentration range.

The compounds did not show any toxic effects on the cells in these concentrations. Therefore these molecules described herein serve as potential inhibitors for the medical intervention of prion diseases such as transmissible spongiform encephalitis (TSE) infections which include Bovine spongiform encephalitis (BSE) or the new variant of Creutzfeld Jakob disease (vCJK).

## Claims

1. Use of a compound having the general formula (I): 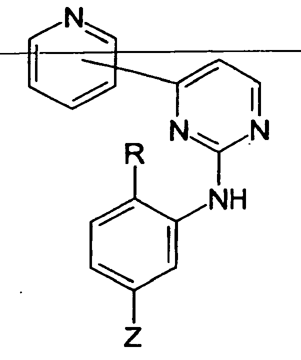 wherein:
R represents hydrogen or methyl;
Y, Y', Y" are independently of each other -H, -F, -Cl, -Br, -I, -CH₂F, -CH₂Cl, -CH₂Br, -CH₂I, -OH, -OCH₃, -CH₃, -CN, -OCF₃, 4-methylpiperazin-1-yl-methyl, -C(CH₃)=N-NH-C(NH)-NH₂;
Z represents -NO₂, -NH₂, -NH-CO-X, -NH-CS-X, -NH-CO-NH-X, -NH-SO₂-X;
X represents thiophenyl, cyclohexyl, isoquinolinyl, naphthyl, quinolinyl, cyclopentyl, pyridinyl, naphthyridinyl, or 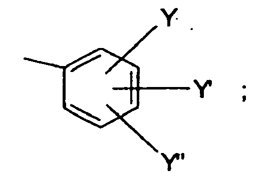
or a pharmaceutical acceptable salt thereof for the manufacture of a pharmaceutical composition for the prophylaxis and/or treatment of prion infections and/or diseases induced by prion infection.

2. Use according to claim 1 wherein said prion infection and/or disease is selected from the group comprising Scrapie, TME, CWD, BSE, CJD, vCJD, GSS, FFI, Kuru, and Alpers Syndrome.

3. Use according to claim 2 wherein said prion infection is BSE, vCJD, or CJD.

4. Use of a compound according to any one of claims 1 - 3 wherein R represents hydrogen.

5. Use of a compound according to any one of claims 1 - 4 wherein Z represents -NH-CO-X or -NH-SO₂-X.

6. Use of a compound according to any one of claims 1 - 5 wherein Y, Y', Y" are independently of each other -H, -F, -Cl, -CH₂F, -CH₂Cl, -OH, -OCH₃, -CH₃, -CN, -OCF₃, 4-methylpiperazin-1-yl-methyl.

7. Use of a compound according to any one of claims 1-6 wherein the compound is selected from the group comprising:
(3-Nitrophenyl)-(4-pyridin-3-yl-pyrimidin-2-yl)-amine;
(3-Aminophenyl)-(4-pyridin-3-yl-pyrimidin-2-yl)-amine;
(5-Amino-2-methylphenyl)-(4-pyridin-3-yl-pyrimidin-2-yl)-amine;
4-Chloromethyl-*N*-[4-methyl-3-(4-pyridin-3-yl-pyrimidin-2-ylamino)-phenyl]-benzamide;
4-Chloromethyl-*N*-[3-(4-pyridin-3-yl-pyrimidin-2-ylamino)-phenyl]-benzamide;
4-(4-Methylpiperazin-1-ylmethyl)-*N*-[3-(4-pyridin-3-yl-pyrimidin-2-ylamino)-phenyl]-benzamide;
Thiophene-3-carboxylic acid [4-methyl-3-(4-pyridin-4-yl-pyrimidin-2-ylamino)-phenyl]-amide;
4-Chloro-*N*-[4-methyl-3-(4-pyridin-3-yl-pyrimidin-2-ylamino)-phenyl]-benzamide;
4-Chloro-*N*-[3-(4-pyridin-3-yl-pyrimidin-2-ylamino)-phenyl]-benzamide;
3,4,5-Trimethoxy-*N*-[3-(4-pyridin-3-yl-pyrimidin-2-ylamino)-phenyl]-benzamide;
4-Cyano-*N*-[3-(4-pyridin-3-yl-pyrimidin-2-ylamino)-phenyl]-benzamide;
4-Methoxy-*N*-[3-(4-pyridin-3-yl-pyrimidin-2-ylamino)-phenyl]-benzamide;
4-Chloro-*N*-[3-(4-pyridin-3-yl-pyrimidin-2-ylamino)-phenyl]-benzenesulfonamide;
Thiophene-3-carboxylic acid [3-(4-pyridin-3-yl-pyrimidin-2-ylamino)-phenyl]-amide;
3,5-Dimethoxy-*N*-[3-(4-pyridin-3-yl-pyrimidin-2-ylamino)-phenyl]-benzamide;
3,4,5-Trimethoxy-*N*-[4-methyl-3-(4-pyridin-3-yl-pyrimidin-2-ylamino)-phenyl]-benzamide;
4-Cyano-*N*-[4-methyl-3-(4-pyridin-3-yl-pyrimidin-2-ylamino)-phenyl]-benzamide;
4-Methoxy-*N*-[4-methyl-3-(4-pyridin-3-yl-pyrimidin-2-ylamino)-phenyl]-benzamide;
4-Chloro-*N*-[4-methyl-3-(4-pyridin-3-yl-pyrimidin-2-ylamino)-phenyl]-benzenesulfonamide;
Thiophene-3-carboxylic acid [4-methyl-3-(4-pyridin-3-yl-pyrimidin-2-ylamino)-phenyl]-amide;
3,5-Dimethoxy-*N*-[4-methyl-3-(4-pyridin-3-yl-pyrimidin-2-ylamino)-phenyl]-benzamide;
4-Trifluoromethoxy-*N*-[4-methyl-3-(4-pyridin-3-yl-pyrimidin-2-ylamino)-phenyl]-benzamide;
Cyclohexanecarboxylic acid [4-methyl-3-(4-pyridin-3-yl-pyrimidin-2-ylamino)-phenyl]-amide;
Cyclohexanecarboxylic acid [3-(4-pyridin-3-yl-pyrimidin-2-ylamino)-phenyl]-amide;
Isoquinoline-5-sulfonic acid [4-methyl-3-(4-pyidin-3-yl-pyrimidin-2-ylamino)-phenyl]-amide;
Isoquinoline-5-sulfonic acid [3-(4-pyridin-3-yl-pyrimidin-2-ylamino)-phenyl]-amide;
(5-Nitro-2-methylphenyl)-(4-pyridin-2-yl-pyrimidin-2-yl)-amine;
(5-Amino-2-methylphenyl)-(4-pyridin-2-yl-pyrimidin-2-yl)-amine;
3,4,5-Trimethoxy-*N*-[4-methyl-3-(4-pyridin-2-yl-pyrimidin-2-ylamino)-phenyl]-benzamide;
4-Cyano-*N*-[4-methyl-3-(4-pyridin-2-yl-pyrimidin-2-ylamino)-phenyl]-benzamide;
(3-Aminophenyl)-(4-pyridin-2-yl-pyrimidin-2-yl)-amine;
4-Chlom-*N*-[4-methyl-3-(4-pyridin-2-yl-pyrimidin-2-ylamino)-phenyl]-benzamide;
Cyclohexanecarboxylic acid [4-methyl-3-(4-pyridin-2-yl-pyrimidin-2-ylamino)-phenyl]-amide;
4-Cyano-*N*-[4-methyl-3-(4-pyridin-4-yl-pyrimidin-2-ylamino)-phenyl]-benzamide;
4-Chloro-*N*-[4-methyl-3-(4-pyridin-4-yl-pydmidin-2-ylamino)-phenyl]-benzenesutfonamide;
4-Methoxy-*N*-[4-methyl-3-(4-pyridin-4-yl-pyrimidin-2-ylamino)-phenyl]-benzamide;
4-Chloro-*N*-[4-methyl-3-(4-pyridin-4-yl-pyrimidin-2-ylamino)-phenyl]-benzamide;
Cyclohexanecarboxylic acid [3-(4-pyridin-4-yl-pyrimidin-2-ylamino)-phenyl]-amide;
3,5-Dimethoxy-*N*-[3-(4-pyridin-4-yl-pyrimidin-2-ylamino)-phenyl]-benzamide;
(5-Amino-2-methylphenyl)-(4-pyridin-4-yl-pyrimidin-2-yl)-amine;
Thiophene-3-carboxylic acid [3-(4-pyridin-4-yl-pyrimidin-2-ylamino)-phenyl]-amide;
4-Chloro-*N*-[3-(4-pyridin-4-yl-pyrimidin-2-ylamino)-phenyl]-benzenesulfonamide;
4-Chloro-*N*-[3-(4-pyridin-4-yl-pyrimidin-2-ylamino)-phenyl]-benzamide;
(3-Aminophenyl)-(4-pyridin-4-yl-pyrimidin-2-yl)-amine;
(3-Nitrophenyl)-(4-pyridin-4-yl-pyrimidin-2-yl)-amine;
4-Trifluoromethoxy-*N*-[4-methyl-3-(4-pyridin-4-yl-pyrimidin-2-ylamino)-phenyl]-benzamide;
Isoquinoline-5-sulfonic acid [3-(4-pyridin-4-yl-pyrimidin-2-ylamino)-phenyl]-amide;
4-Methoxy-*N*-[3-(4-pyridin-4-yl-pyimidin-2-ylamino)-phenyl]-benzamide;
4-Cyano-*N*-[3-(4-pyridin-4-yl-pyrimidin-2-ylamino)-phenyl]-benzamide;
3,4,5-Trimethoxy-*N*-[3-(4-pyridin-4-yl-pyrimidin-2-ylamino)-phenyl]-benzamide;
3,5-Dimethoxy-*N*-[4-methyl-3-(4-pyridin-4-yl-pyrimidin-2-ylamino)-phenyl]-benzamide;
3,4,5-Trimethoxy-*N*-[4-methyl-3-(4-pyridin-4-yl-pyrimidin-2-ylamino)-phenyl]-benzamide;
4-(4-Methylpiperazin-1-ylmethyl)-N-[4-methyl-3-(4-pyridin-3-yl-pyrimidin-2-ylamino)-phenyl]-benzamide;
4-Methyl-*N*-[4-methyl-3-(4-pyridin-2-yl-pyrimidin-2-ylamino)-phenyl]-benzenesulfonamide;
4-Methoxy-*N*-[4-methyl-3-(4-pyridin-2-yl-pyrimidin-2-ylamino)-phenyl]-benzamide;
3,5-Dimethoxy-*N*-[4-methyl-3-(4-pyridin-2-yl-pyrimidin-2-ylamino)-phenyl]-benzamide;
Naphthalene-2-carboxylic acid [4-methyl-3-(4-pyridin-3-yl-pyrimidin-2-ylamino)-phenyl]-amide;
*N*-[3-(4-Pyridin-4-yl-pyrimidin-2-ylamino)-phenyl]-benzamide;
4-Chloro-*N*-[3-(4-pyridin-2-yl-pyrimidin-2-ylamino)-phenyl]-benzamide;
4-Methoxy-*N*-[3-(4-pyridin-2-yl-pyrimidin-2-ylamino)-phenyl]-benzamide;
4-Chloro-*N*-[3-(4-pyridin-2-yl-pyrimidin-2-ylamino)-phenyl]-benzenesulfonamide;
Thiophene-2-carboxylic acid 3-(4-pyridin-2-yl-pyrimidin-2-yl-amino)-phenyl]-amide;
Naphthalene-2-sulfonic-acid [3-(4-pyridin-2-yl-pyrimidin-2-yl-amino)-phenyl]-amide;
Isoquinoline-5-sulfonic-acid [3-(4-pyridin-2-yl-pyrimidin-2-yl-amino)-phenyl]-amide;
Cylopentanecarboxylic acid 3-(4-pyridin-2-yl-pyimidin-2-yl-amino)-phenyl]-amide;
Naphthalene-2-carboxylic acid [3-(4-pyridin-2-yl-pyrimidin-2-ylamino)-phenyl]-amide;
4-Cyano-*N*-[3-(4-pyridin-2-yl-pyrimidin-2-ylamino)-phenyl]-benzamide;
3,5-Dimethoxy-*N*-[3-(4-pyridin-2-yl-pyrimidin-2-ylamino)-phenyl]-benzamide;
4-Bromo-*N*-[3-(4-pyridin-2-yl-pyrimidin-2-ylamino)-phenyl]-benzamide;
4-Methyl-*N*-[3-(4-pyridin-2-yl-pyrimidin-2-ylamino)-phenyl]-benzamide;
4-Fluoro-*N*-3-(4-pyridin-2-yl-pyrimidin-2-ylamino)-phenyl]-benzenesulfonamide;
3,5-Dichloro-*N*-[3-(4-pyridin-2-yl-pyrimidin-2-ylamino)-phenyl]-benzamide;
*N*-[3-(4-Pyridin-2-yl-pyrimidin-2-ylamino)-phenyl]-benzamide;
4-Chloromethyl-*N*-[3-(4-pyridin-2-yl-pyrimidin-2-ylamino)-phenyl]-benzamide;
4-Methyl-*N*-3-(4-pyridin-2-yl-pyrimidin-2-ylamino)-phenyl]-benzenesulfonamide;
4-(4-Methylpiperazin-1-ylmethyl)-*N*-[3-(4-pyridin-2-yl-pyrimidin-2-ylamino)-phenyl]-benzamide;
Naphthalene-2-carboxylic acid [3-(4-pyridin-4-yl-pyrimidin-2-ylamino)-phenyl]-amide;
2-Methoxy-*N*-[4-methyl-3-(4-pyridin-3-yl-pyrimidin-2-ylamino)-phenyl]-benzamide;
2-Methoxy-*N*-[3-(4-pyridin-3-yl-pyrimidin-2-ylamino)-phenyl]-benzamide;
4-Methyl-*N*-[3-(4-pyridin-3-yl-pyrimidin-2-ylamino)-phenyl]-benzamide;
4-Methyl-*N*-[4-methyl-3-(4-pyridin-3-yl-pyrimidin-2-ylamino)-phenyl]-benzamide;
*N*-[4-Methyl-3-(4-pyridin-3-yl-pyrimidin-2-ylamino)-phenyl]-benzamide;
1-(3,5-Diacetyl-phenyl)-3-[4-methyl-3-(4-pyridin-3-yl-pyrimidin-2-ylamino)-phenyl]-urea;
1-{3,5-Bis-(amidinohydrazone)-phenyl}-3-[4-methyl-3-(4-pyridin-3-yl-pyrimidin-2-ylamino)-phenyl]-urea;
*N*-[4-Methyl-3-(4-pyridin-3-yl-pyrimidin-2-ylamino)-phenyl]-nicotinamide;
*N*-[3-(4-Pyridin-3-yl-pyrimidin-2-ylamino)-phenyl]-nicotinamide;
[1,8]Naphthyridine-2-carboxylic acid [3-(4-pyridin-3-yl-pyrimidin-2-ylamino)-phenyl]-amide;
[1,8]Naphthyridine-2-carbothioic acid [3-(4-pyridin-3-yl-pyrimidin-2-ylamino)-phenyl]-amide;
2-Methoxy-*N*-[3-(4-pyridin-4-yl-pyimidin-2-ylamino)-phenyl]-benzamide;
4-Trifluoromethoxy-*N*-[3-(4-pyridin-4-yl-pyrimidin-2-ylamino)-phenyl]-benzamide;
4-Methyl-*N*-[3-(4-pyridin-4-yl-pyrimidin-2-ylamino)-phenyl]-benzamide;
and/or a pharmaceutically acceptable salt of these compounds.

8. Use according to claim 7 wherein the compound is 4-(4-Methylpiperazin-1-ylmethyl)-*N*-[4-methyl-3-(4-pyridin-3-yl-pyrimidin-2-ylamino)-phenyl]-benzamide.

## Patentansprüche

1. Verwendung einer Verbindung der allgemeinen Formel (I): 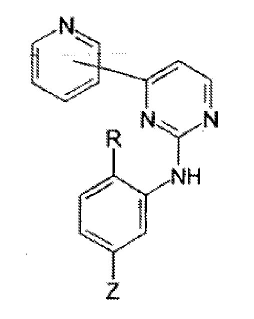 in der
R ein Wasserstoffatom oder ein Methylrest ist;
Y, Y' und Y" unabhängig voneinander -H, -F, -Cl, -Br, -I, -CH₂F, -CH₂Cl, -CH₂Br,-CH₂I, -OH, -OCH₃, -CH₃, -CN, -OCF₃,
4-Methylpiperazin-1-yl-methyl, -C(CH₃)=N-NH-C(NH)-NH₂ sind;
Z -NO₂, -NH₂, -NH-CO-X, -NH-CS-X, -NH-CO-NH-X, -NH-SO₂-X ist; X Thiophenyl, Cyclohexyl, Isochinolinyl, Naphtyl, Chinolinyl, Cyclopentyl, Pyridinyl, Naphtyridinyl, oder 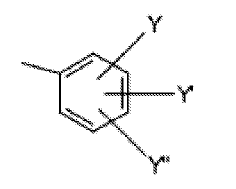 ist, oder eines pharmazeutisch verträglichen Salzes davon, zur Herstellung einer pharmazeutischen Zusammensetzung für die Prophylaxe und/oder Behandlung von Prion-Infektionen und/oder Krankheiten, die durch Prion-Infektion ausgelöst werden.

2. Die Verwendung gemäß Anspruch 1, wobei die Prion-Infektion und/oder Krankheit ausgewählt ist aus der Gruppe bestehend aus Scrapie, TME, CWD, BSE, CJD, vCJD, GSS, FFI, Kuru und Alpers Syndrom.

3. Die Verwendung gemäß Anspruch 2, wobei die Prion-Infektion BSE, vCJD oder CJD ist.

4. Die Verwendung einer Verbindung gemäß einem der Ansprüche 1 bis 3, wobei R ein Wasserstoffatom darstellt.

5. Die Verwendung einer Verbindung gemäß einem der Ansprüche 1 bis 4, wobei Z einen Rest -NH-CO-X oder -NH-SO₂-X darstellt.

6. Die Verwendung einer Verbindung gemäß einem der Ansprüche 1 bis 5, wobei Y, Y' und Y" unabhängig voneinander -H, -F, -Cl, -CH₂F, -CH₂Cl, -OH, -OCH₃, -CH₃, -CN, -OCF₃, oder 4-Methylpiperazin-1-yl-methyl sind.

7. Die Verwendung einer Verbindung gemäß einem der Ansprüche 1 bis 6, in der die Verbindung ausgewählt ist aus der Gruppe bestehend aus:
(3-Nitrophenyl)-(4-pyridin-3-yl-pyrimidin-2-yl)-amin;
(3-Aminophenyl)-(4-pyridin-3-yl-pyrimidin-2-yl)-amin;
(5-Amino-2-methylphenyl)-(4-pyridin-3-yl-pyrimidin-2-yl)-amin;
4-Chlon-nethyl-*N*-[4-methyl-3-(4-pyridin-3-yl-pyrimidin-2-ylamino)-phenyl]-benzamid;
4-Chlormethyl-*N*-[3-(4-pyädin-3-yl-pyämidin-2-ylamino)-phenyl]-benzamid;
4-(4-Methylpiperazin-1-ylmethyl)-N-[3-(4-pyridin-3-yl-pyrimidin-2-ylamino)-phenyl]-benzamid;
Thiophen-3-carbonsäure-[4-methyl-3-(4-pyridin-4-yl-pyrimidin-2-ylamino)-phenyl]-amid;
4-Chlor-*N*-[4-methyl-3-(4-pyridin-3-yl-pyrimidin-2-ylamino)-phenyl]-benzamid;
4-Chlor-*N*-[3-(4-pyridin-3-yl-pyrimidin-2-ylamino)-phenyl]-benzamid;
3,4,5-Trimethoxy-*N*-[3-(4-pyridin-3-yl-pyrimidin-2-ylamino)-phenyl]-benzamid;
4-Cyan-*N*-[3-(4-pyridin-3-yl-pyrimidin-2-ylamino)-phenyl]-benzamid;
4-Methoxy-*N*-[3-(4-pyridin-3-yl-pyrimidin-2-ylamino)-phenyl]-benzamid;
4-Chlor-*N*-[3-(4-pyridin-3-yl-pyrimidin-2-ylwnino)-phenyl]-benzylsulfonamid;
Thiophen-3-carbonsäure-[3-(4-pyridin-3-yl-pyrimidin-2-ylamino)-phenyl]-amid;
3,5-Dimethoxy-*N*-[3-(4-pyridin-3-yl-pyrimidin-2-ylamino)-phenyl]-benzamid;
3,4,5-Trimethoxy-*N*-[4-methyl-3-(4-pyridin-3-yl-pyrimidin-2-ylamino)-phenyl]-benzamid;
4-Cyan-*N*-[4-methyl-3-(4-pyridin-3-yl-pyrimidin-2-ylamino)-phenyl]-benzamid;
4-Methoxy-*N*-[4-methyl-3-(4-pyridin-3-yl-pyrimidin-2-ylamino)-phenyl]-benzamid;
4-Chlor-*N*-[4-methyl-3-(4-pyridin-3-yl-pyrimidin-2-ylamino)-phenyl]-benzylsulfonamid;
Thiophen-3-carbonsäure-[4-methyl-3-(4-pyridin-3-yl-pyrimidin-2-ylamino)-phenyl]-amid;
3,5-Dimethoxy-*N*-[4-methyl-3-(4-pyridin-3-yl-pyrimidin-2-ylamino)-phenyl]-benzamid;
4-Trifluormethoxy-*N*-[4-methyl-3-(4-pyridin-3-yl-pyrimidin-2-ylamino)-phenyl]-benzamid;
Cyclohexancarbonsäure-[4-methyl-3-(4-pyridin-3-yl-pyrimidin-2-ylamino)-phenyl]-amid;
Cyclohexancarbonsäure-[3-(4-pyridin-3-yl-pyrimidin-2-ylamino)-phenyl]-amid;
Isochinolin-5-sulfonsäure-[4-methyl-3-(4-pyridin-3-yl-pyrimidin-2-ylamino)-phenyl]-amid;
Isochinolin-5-sulfonsäure-[3-(4-pyridin-3-yl-pyrimidin-2-ylamino)-phenyl]-amid;
(5-Nitro-2-methylphenyl)-(4-pyridin-2-yl-pyrimidin-2-yl)-amin;
(5-Amino-2-methylphenyl)-(4-pyridin-2-yl-pyrimidin-2-yl)-amin;
3,4,5-Trimethoxy-*N*-[4-methyl-3-(4-pyridin-2-yl-pyrimidin-2-ylamino)-phenyl]-benzamid;
4-Cyan-*N*-[4-methyl-3-(4-pyridin-2-yl-pyrimidin-2-ylamino)-phenyl]-benzamid
(3-Aminophenyl)-(4-pyridin-2-yl-pyrimidin-2-yl)-amin;
4-Chlor-*N*-[4-methyl-3-(4-pyridin-2-yl-pyrimidin-2-ylamino)-phenyl]-benzamid;
Cyclohexancarbonsäure-[4-Methyl-3-(4-pyridin-2-yl-pyrimidin-2-ylamino)-phenyl]-amid;
4-Cyan-*N*-[4-methyl-3-(4-pyridin-4-yl-pyrimidin-2-ylamino)-phenyl]-benzamid;
4-Chlor-*N*-[4-methyl-3-(4-pyridin-4-yl-pyrimidin-2-ylamino)-phenyl]-benzylsulfonamid;
4-Methoxy-*N*-[4-methyl-3-(4-pyridin-4-yl-pyrimidine-2-ylamino)-phenyl]-benzamid;
4-Chlor-*N*-[4-methyl-3-(4-pyridin-4-yl-pyrimidin-2-ylamino)-phenyl]-benzamid;
Cyclohexancarbonsäure-[3-(4-pyridin-4-yl-pyrimidin-2-ylamino)-phenyl]-amid;
3,5-Dimethoxy-*N*-[3-(4-pyridin-4-yl-pyrimidin-2-ylamino)-phenyl]-benzamid;
(5-Amino-2-methylphenyl)-(4-pyridin-4-yl-pyrimidin-2-yl)-amin;
Thiophen-3-carbonsäure-[3-(4-pyridin-4-yl-pyrimidin-2-ylamino)-phenyl]-amid;
4-Chlor-*N*-[3-(4-pyridin-4-yl-pyrimidin-2-ylwnino)-phenyl]-benzylsulfonamid;
4-Chlor-*N*-[3-(4-pyridin-4-yl-pyrimidin-2-ylamino)-phenyl]-benzamid;
(3-Aminophenyl)-(4-pyridin-4-yl-pyrimidin-2-yl)-amin;
(3-Nitrophenyl)-(4-pyridin-4-yl-pyrimidin-2-yl)-amin;
4-Trifluormethoxy-*N*-[4-methyl-3-(4-pyridin-4-yl-pyrimidin-2-ylamino)-phenyl]-benzamid;
Isochinolin-5-sulfonsäure-[3-(4-pyridin-4-yl-pyrimidin-2-ylamino)-phenyl]-amid;
4-Methoxy-*N*-[3-(4-pyridin-4-yl-pyrimidin-2-ylamino)-phenyl]-benzamid;
4-Cyan-*N*-[3-(4-pyridin-4-yl-pyrimidin-2-ylamino)-phenyl]-benzamid;
3,4,5-Trimethoxy-*N*-[3-(4-pyridin-4-yl-pyrimidin-2-ylamino)-phenyl]-benzamid;
3,5-Dimethoxy-*N*-[4-methyl-3-(4-pyridin-4-yl-pyrimidin-2-ylamino)-phenyl]-benzamid;
3,4,5-Trimethoxy-*N*-[4-methyl-3-(4-pyridin-4-yl-pyrimidin-2-ylamino)-phenyl]-benzamid;
4-(4-Methylpiperazin-1-ylmethyl)-*N*-[4-methyl-3-(4-pyridin-3-yl-pyrimidin-2-ylamino)-phenyl]-benzamid;
4-Methyl-*N*-[4-methyl-3-(4-pyridin-2-yl-pyrimidin-2-ylamino)-phenyl]-benzylsulfonamid;
4-Methoxy-*N*-[4-methyl-3-(4-pyridin-2-yl-pyrimidin-2-ylamino)-phenyl]-benzamid;
3,5-Dimethoxy-N-[4-methyl-3-(4-pyridin-2-yl-pyrimidin-2-ylamino)-phenyl]-benzamid;
Naphtalen-2-carbonsäure-[4-methyl-3-(4-pyridin-3-yl-pyrimidin-2-ylamino)-phenyl]-amid;
*N*-[3-(4-Pyridin-4-yl-pyrimidin-2-ylamino)-phenyl]-benzamid;
4-Chlor-*N*-[3-(4-pyridin-2-yl-pyrimidin-2-ylamino)-phenyl]-benzamid;
4-Methoxy-*N*-[3-(4-pyridin-2-yl-pyrimidin-2-ylamino)-phenyl]-benzamid;
4-Chlor-*N*-[3-(4-pyridin-2-yl-pyrimidin-2-ylamino)-phenyl]-benzylsulfonamid;
Thiophen-2-carbonsäure-3-(4-pyridin-2-yl-pyrimidin-2-ylamino)-phenyl]-amid;
Naphtalen-2-sulfonsäure-[3-(4-pyridin-2-yl-pyrimidin-2-ylamino)-phenyl]-amid;
Isochinolin-5-sulfonsäure-[3-(4-pyridin-2-yl-pyrimidin-2-ylamino)-phenyl]-amid;
Cyclopentancarbonsäure-3-(4-pyridin-2-yl-pyrimidin-2-ylamino)-phenyl]-amid;
Naphtalen-2-carbonsäure [3-(4-pyridin-2-yl-pyrimidin-2-ylamino)-phenyl]-amid;
4-Cyan-*N*-[3-(4-pyridin-2-yl-pyrimidin-2-ylamino)-phenyl]-benzamid;
3,5-Dimethoxy-*N*-[3-(4-pyridin-2-yl-pyrimidin-2-ylamino)-phenyl]-benzamid;
4-Brom-*N*-[3-(4-pyridin-2-yl-pyrimidin-2-ylamino)-phenyl]-benzamid;
4-Methyl-*N*-[3-(4-pyridin-2-yl-pyrimidin-2-ylamino)-phenyl]-benzamid;
4-Fluor-*N*-3-(4-pyridin-2-yl-pyrimidin-2-ylamino)-phenyl]-benzylsulfonamid;
3,5-Dichlor-*N*-[3-(4-pyridin-2-yl-pyrimidin-2-ylamino)-phenyl]-benzamid;
*N*-[3-(4-pyridin-2-yl-pyrimidin-2-ylamino)-phenyl]-benzamid;
4-Chlormethyl-*N*-[3-(4-pyridin-2-yl-pyrimidin-2-ylamino)-phenyl]-benzamid;
4-Methyl-*N*-3-(4-pyridin-2-yl-pyrimidin-2-ylamino)-phenyl]-benzylsulfonamid;
4-(4-Methylpiperazin-1-ylmethyl)-*N*-[3-(4-pyridin-2-yl-pyrimidin-2-ylamino)-phenyl]-benzamid;
Naphtalen-2-carbonsäure-[3-(4-pyridin-4-yl-pyrimidin-2-ylamino)-phenyl]-amid;
2-Methoxy-*N*-[4-methyl-3-(4-pyridin-3-yl-pyrimidin-2-ylamino)-phenyl]-benzamid;
2-Methoxy-*N*-[3-(4-pyridin-3-yl-pyrimidin-2-ylamino)-phenyl]-benzamid;
4-Methyl-*N*-[3-(4-pyridin-3-yl-pyrimidin-2-ylamino)-phenyl]-benzamid;
4-Methyl-*N*-[4-methyl-3-(4-pyridin-3-yl-pyrimidin-2-ylamino)-phenyl]-benzamid;
*N*-[4-Methyl-3-(4-pyridin-3-yl-pyrimidin-2-ylamino)-phenyl]-benzamid;
1-(3,5-Diacetyl-phenyl)-3-[4-methyl-3-(4-pyridin-3-yl-pyrimidin-2-ylamino)-phenyl]-harnstoff;
1-{3,5-Bis-(amidinohydrazon)-phenyl}-3-[4-methyl-3-(4-pyridin-3-yl-pyrimidin-2-ylamino)-phenyl]-harnstoff;
*N*-[4-Methyl-3-(4-pyridin-3-yl-pyrimidin-2-ylamino)-phenyl]-nicotinamid;
*N*-[3-(4-Pyridin-3-yl-pyrimidin-2-ylamino)-phenyl]-nicotinamid;
[1,8]Naphtyridin-2-carbonsäure-[3-(4-pyridin-3-yl-pyrimidin-2-ylamino)-phenyl]-amid;
[1,8]Naphtyridin-2-thiocarbonsäure-[3-(4-pyridin-3-yl-pyrimidin-2-ylamino)-phenyl]-amid;
2-Methoxy-*N*-[3-(4-pyridin-4-yl-pyrimidin-2-ylamino)-phenyl]-benzamid;
4-Trifluormethoxy-*N*-[3-(4-pyridin-4-yl-pyrimidin-2-ylamino)-phenyl]-benzamid;
4-Methyl-*N*-[3-(4-pyridin-4-yl-pyrimidin-2-ylamino)-phenyl]-benzamid;
und/oder ein pharmazeutisch verträgliches Salz dieser Verbindungen.

8. Die Verwendung gemäß Anspruch 7, in der die Verbindung 4-(4-Methylpiperazin-1-ylmethyl)-*N*-[4-methyl-3-(4-pyridin-3-yl-pyrimidin-2-ylamino)-phenyl]-benzamid ist.

## Revendications

1. Utilisation d'un composé répondant à la formule générale (I) : 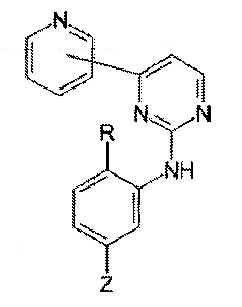 dans laquelle:
R représente un atome d'hydrogène ou un groupe méthyle;
Y, Y' et Y" représentent, indépendamment les uns des autres, un groupe -H, -F, -Cl, -Br, -I, -CH₂F, -CH₂Cl, -CH₂Br, -CH₂I, -OH, -OCH₃, -CH₃, -CN, -OCF₃, 4-méthylpipérazine-1-yl-méthyle, -C(CH₃)=N-NH-C(NH)-NH₂;
Z représente un groupe -NO₂, -NH₂, -NH-CO-X, -NH-CS-X, -NH-CO-NH-X, -NH-SO₂₋X;
X représente un groupe thiophényle, cyclohexyle, isoquinolinyle, naphtyle, quinolinyle, cyclopentyle, pyridinyle, naphtyridinyle, ou 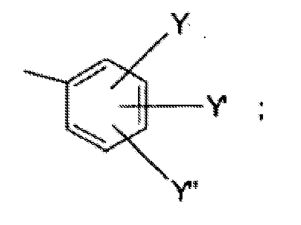
ou d'un de ses sels pharmaceutiquement acceptables pour la production d'une composition pharmaceutique destinée à la prophylaxie et/ou au traitement d'infections à prions et/ou de maladies induites par un infection à prions.

2. Utilisation suivant la revendication 1, dans laquelle ladite infection et/ou maladie à prions est choisie dans le groupe consistant en Scrapie, TME, CWD, BSE, CJD, vCJD, GSS, FFI, Kuru et le syndrome d'Alpers.

3. Utilisation suivant la revendication 2, dans laquelle ladite infection à prions est BSE, vCJD ou CJD.

4. Utilisation d'un composé suivant l'une quelconque des revendications 1 à 3, dans laquelle R représente un atome d'hydrogène.

5. Utilisation d'un composé suivant l'une quelconque des revendications 1 à 4, dans laquelle Z représente un groupe -NH-CO-X ou -NH-SO₂-X.

6. Utilisation d'un composé suivant l'une quelconque des revendications 1 à 5, dans laquelle Y, Y' et Y" représentent, indépendamment les uns des autres, un groupe -H, -F,-Cl, -CH₂F, -CH₂Cl, -OH, -OCH₃, -CH₃, -CN, -OCF₃, 4-méthylpipérazine-1-yl-méthyle.

7. Utilisation d'un composé suivant l'une quelconque des revendications 1 à 6, dans lequel le composé est choisi dans le groupe comprenant:
(3-nitrophényl)-(4-pyridine-3-yl-pyrimidine-2-yl)-amine ;
(3-aminophényl)-(4-pyridine-3-yl-pyrimidine-2-yl)-amine ;
(5-amino-2-méthylphényl)-(4-pyridine-3-yl-pyrimidine-2-yl)-amine ;
4-chlorométhyl*-N*-[4-méthyl-3-(4-pyridine-3-yl-pyrimidine-2-ylamino)-phényl]-benzamide ;
4-chlorométhyl-*N*-[3-(4-pyridine-3-yl-pyrimidine-2-ylamino)-phényl]-benzamide ;
4-(4-méthylpipérazine-1-ylméthyl)-*N*-[3-(4-pyridine-3-yl-pyrimidine-2-ylamino)-phényl]-benzamide ;
[4-méthyl-3-(4-pyridine-4-yl-pyrimidine-2-ylamino)-phényl]-amide d'acide thiophène-3-carboxylique ;
4-chloro-*N*-[4-méthyl-3-(4-pyridine-3-yl-pyrimidine-2-ylamino)-phényl]-benzamide ;
4-chloro-*N*-[3-(4-pyridine-3-yl-pyrimidine-2-ylamino)-phényl]-benzamide ;
3,4,5-triméthoxy-*N*-[3-(4-pyridine-3-yl-pyrimidine-2-ylamino)-phényl]-benzamide ;
4-cyano-*N*-[3-(4-pyridine-3-yl-pyrimidine-2-ylamino)-phényl]-benzamide ;
4-méthoxy-*N*-[3-(4-pyridine-3-yl-pyrimidine-2-ylamino)-phényl]-benzamide ;
4-chloro-*N*-[3-(4-pyridine-3-yl-pyrimidine-2-ylamino)-phényl]-benzènesulfonamide ;
[3-(4-pyridine-3-yl-pyrimidine-2-ylamino)-phényl]-amide d'acide thiophène-3-carboxylique ;
3,5-diméthoxy-*N*-[3-(4-pyridine-3-yl-pyrimidine-2-ylamino)-phényl]-benzamide ;
3,4,5-triméthoxy-*N*-[4-méthyl-3-(4-pyridine-3-yl-pyrimidine-2-ylamino)-phényl]-benzamide ;
4-cyano-*N*-[4-méthyl-3-(4-pyridine-3-yl-pyrimidine-2-ylamino)-phényl]-benzamide ;
4-méthoxy-*N*-[4-méthyl-3-(4-pyridine-3-yl-pyrimidine-2-ylamino)-phényl]-benzamide ;
4-chloro-*N*-[4-méthyl-3-(4-pyridine-3-yl-pyrimidine-2-ylamino)-phényl]-benzènesulfonamide ;
[4-méthyl-3-(4-pyridine-3-yl-pyrimidine-2-ylamino)-phényl]-amide d'acide thiophène-3-carboxylique ;
3,5-diméthoxy-*N*-[4-méthyl-3-(4-pyridine-3-yl-pyrimidine-2-ylamino)-phényl]-benzamide ;
4-trifluorométhoxy-*N*-[4-méthyl-3-(4-pyridine-3-yl-pyrimidine-2-ylamino)-phényl]-benzamide ;
[4-méthyl-3-(4-pyridine-3-yl-pyrimidine-2-ylamino)-phényl]-amide d'acide cyclohexanecarboxylique ;
[3-(4-pyridine-3-yl-pyrimidine-2-ylamino)-phényl]-amide d'acide cyclohexanecarboxylique ;
[4-méthyl-3-(4-pyridine-3-yl-pyrimidine-2-ylamino)-phényl]-amide d'acide isoquinoléine-5-sulfonique ;
[3-(4-pyridine-3-yl-pyrimidine-2-ylamino)-phényl]-amide d'acide isoquinoléine-5-sulfonique ;
(5-nitro-2-méthylphényl)-(4-pyridine-2-yl-pyrimidine-2-yl)-amine ;
(5-amino-2-méthylphényl)-(4-pyridine-2-yl-pyrimidine-2-yl)-amine ;
3,4,5-triméthoxy-*N*-[4-méthyl-3-(4-pyridine-2-yl-pyrimidine-2-ylamino)-phényl]-benzamide ;
4-cyano-*N*-[4-méthyl-3-(4-pyridine-2-yl-pyrimidine-2-ylamino)-phényl]-benzamide ;
(3-aminophényl)-(4-pyridine-2-yl-pyrimidine-2-yl)-amine ;
4-chloro-*N*-[4-méthyl-3-(4-pyridine-2-yl-pyrimidine-2-ylamino)-phényl]-benzamide ;
[4-méthyl-3-(4-pyridine-2-yl-pyrimidine-2-ylamino)-phényl]-amide d'acide cyclohexanecarboxylique ;
4-cyano-*N*-[4-méthyl-3-(4-pyridine-4-yl-pyrimidine-2-ylamino)-phényl]-benzamide ;
4-chloro-*N*-[4-méthyl-3-(4-pyridine-4-yl-pyrimidine-2-ylamino)-phényl]-benzènesulfonamide ;
4-méthoxy-*N*-[4-méthyl-3-(4-pyridine-4-yl-pyrimidine-2-ylamino)-phényl]-benzamide ;
4-chloro-*N*-[4-méthyl-3-(4-pyridine-4-yl-pyrimidine-2-ylamino)-phényl]-benzamide ;
[3-(4-pyridine-4-yl-pyrimidine-2-ylamino)-phényl]-amide d'acide cyclohexanecarboxylique ;
3,5-diméthoxy-*N*-[3-(4-pyridine-4-yl-pyrimidine-2-ylamino)-phényl]-benzamide ;
(5-amino-2-méthylphényl)-(4-pyridine-4-yl-pyrimidine-2-yl)-amine ;
[3-(4-pyridine-4-yl-pyrimidine-2-ylamino)-phényl]-amide d'acide thiophène-3-carboxylique ;
4-chloro-*N*-[3-(4-pyridine-4-yl-pyrimidine-2-ylamino)-phényl]-benzènesulfonamide ;
4-chloro-*N*-[3-(4-pyridine-4-yl-pyrimidine-2-ylamino)-phényl]-benzamide;
(3-aminophényl)-(4-pyridine-4-yl-pyrimidine-2-yl)-amine ;
(3-nitrophényl)-(4-pyridine-4-yl-pyrimidine-2-yl)-amine ;
4-trifluorométhoxy-*N*-[4-méthyl-3-(4-pyridine-4-yl-pyrimidine-2-ylamino)-phényl]-benzamide ;
[3-(4-pyridine-4-yl-pyrimidine-2-ylamino)-phényl]-amide d'acide isoquinoléine-5-sulfonique ;
4-méthoxy-*N*-[3-(4-pyridine-4-yl-pyrimidine-2-ylamino)-phényl]-benzamide ;
4-cyano-*N*-[3-(4-pyridine-4-yl-pyrimidine-2-ylamino)-phényl]-benzamide ;
3,4,5-triméthoxy-*N*-[3-(4-pyridine-4-yl-pyrimidine-2-ylamino)-phényl]-benzamide ;
3,5-diméthoxy-*N*-[4-méthyl-3-(4-pyridine-4-yl-pyrimidine-2-ylamino)-phényl]-benzamide ;
3,4,5-triméthoxy-*N*-[4-méthyl-3-(4-pyridine-4-yl-pyrimidine-2-ylamino)-phényl]-benzamide ;
4-(4-méthylpipérazine-1-ylméthyl)-N-[4-méthyl-3-(4-pyridine-3-yl-pyrimidine-2-ylamino)-phényl]-benzamide ;
4-méthyl-*N*-[4-méthyl-3-(4-pyridine-2-yl-pyrimidine-2-ylamino)-phényl]-benzènesulfonamide ;
4-méthoxy-*N*-[4-méthyl-3-(4-pyridine-2-yl-pyrimidine-2-ylamino)-phényl]-benzamide ;
3,5-diméthoxy-*N*-[4-méthyl-3-(4-pyridine-2-yl-pyrimidine-2-ylamino)-phényl]-benzamide ;
[4-méthyl-3-(4-pyridine-3-yl-pyrimidine-2-ylamino)-phényl]-amide d'acide naphtalène-2-carboxylique ;
*N*-[3-(4-pyridine-4-yl-pyrimidine-2-ylamino)-phényl]-benzamide ;
4-chloro-*N*-[3-(4-pyridine-2-yl-pyrimidine-2-ylamino)-phényl]-benzamide ;
4-méthoxy-*N*-[3-(4-pyridine-2-yl-pyrimidine-2-ylamino)-phényl]-benzamide ;
4-chloro-*N*-[3-(4-pyridine-2-yl-pyrimidine-2-ylamino)-phényl]-benzènesulfonamide ;
3-(4-pyridine-2-yl-pyrimidine-2-ylamino)-phényl]-amide d'acide thiophène-2-carboxylique ;
[3-(4-pyridine-2-yl-pyrimidine-2-ylamino)-phényl]-amide d'acide naphtalène-2-sulfonique ;
[3-(4-pyridine-2-yl-pyrimidine-2-ylamino)-phényl]-amide d'acide isoquinoléine-5-sulfonique ;
3-(4-pyridine-2-yl-pyrimidine-2-ylamino)-phényl]-amide d'acide cyclopentanecarboxylique ;
[3-(4-pyridine-2-yl-pyrimidine-2-ylamino)-phényl]-amide d'acide naphtalène-2-carboxylique ;
4-cyano-*N*-[3-(4-pyridine-2-yl-pyrimidine-2-ylamino)-phényl]-benzamide ;
3,5-diméthoxy-*N*-[3-(4-pyridine-2-yl-pyrimidine-2-ylamino)-phényl]-benzamide ;
4-bromo-*N*-[3-(4-pyridine-2-yl-pyrimidine-2-ylamino)-phényl]-benzamide ;
4-méthyl-*N*-[3-(4-pyridine-2-yl-pyrimidine-2-ylamino)-phényl]-benzamide ;
4-fluoro-*N*-3-(4-pyridine-2-yl-pyrimidine-2-ylamino)-phényl]-benzènesulfonamide ;
3,5-dichloro-*N*-[3-(4-pyridine-2-yl-pyrimidine-2-ylamino)-phényl]-benzamide ;
*N*-[3-(4-pyridine-2-yl-pyrimidine-2-ylamino)-phényl]-benzamide ;
4-chlorométhyl-*N*-[3-(4-pyridine-2-yl-pyrimidine-2-ylamino)-phényl]-benzamide ;
4-méthyl-*N*-3-(4-pyridine-2-yl-pyrimidine-2-ylamino)-phényl]-benzènesulfonamide ;
4-(4-méthylpipérazine-1-ylméthyl)-*N*-[3-(4-pyridine-2-yl-pyrimidine-2-ylamino)-phényl]-benzamide ;
[3-(4-pyridine-4-yl-pyrimidine-2-ylamino)-phényl]-amide d'acide naphtalène-2-carboxylique ;
2-méthoxy-*N*-[4-méthyl-3-(4-pyridine-3-yl-pyrimidine-2-ylamino)-phényl]-benzamide ;
2-méthoxy-*N*-[3-(4-pyridine-3-yl-pyrimidine-2-ylamino)-phényl]-benzamide ;
4-méthyl-*N*-[3-(4-pyridine-3-yl-pyrimidine-2-ylamino)-phényl]-benzamide ;
4-méthyl-*N*-[4-méthyl-3-(4-pyridine-3-yl-pyrimidine-2-ylamino)-phényl]-benzamide ;
*N*-[4-méthyl-3-(4-pyridine-3-yl-pyrimidine-2-ylamino)-phényl]-benzamide ;
1-(3,5-diacétyl-phényl)-3-[4-méthyl-3-(4-pyridine-3-yl-pyrimidine-2-ylamino)-phényl]-urée ;
1-{3,5-bis-(amidinohydrazone)-phényl}-3-[4-méthyl-3-(4-pyridine-3-yl-pyrimidine-2-ylamino)-phényl]-urée ;
*N*-[4-méthyl-3-(4-pyridine-3-yl-pyrimidine-2-ylamino)-phényl]-nicotinamide ;
*N*-[3-(4-pyridine-3-yl-pyrimidine-2-ylamino)-phényl]-nicotinamide ;
[3-(4-pyridine-3-yl-pyrimidine-2-ylamino)-phényl]-amide d'acide [1,8]naphtyridine-2-carboxylique ;
[3-(4-pyridine-3-yl-pyrimidine-2-ylamino)-phényl]-amide d'acide [1,8]naphtyridine-2-carbothioïque ;
2-méthoxy-*N*-[3-(4-pyridine-4-yl-pyrimidine-2-ylamino)-phényl]-benzamide ;
4-trifluorométhoxy-*N*-[3-(4-pyridine-4-yl-pyrimidine-2-ylamino)-phényl]-benzamide ;
4-méthyl-*N*-[3-(4-pyridine-4-yl-pyrimidine-2-ylamino)-phényl]-benzamide ;
et/ou d'un sel pharmaceutiquement acceptable de ces composés.

8. Utilisation suivant la revendication 7, dans laquelle le composé est le 4-(4-méthylpipérazine-1-ylméthyl)-N-[4-méthyl-3-(4-pyridine-3-yl-pyrimidine-2-ylamino)-phényl]-benzamide.
